# FASCICULE DE BREVET EUROPEEN

(11) **EP 0 941 341 B1**
(45) Date de publication et mention de la délivrance du brevet: **24.01.2007**
(21) Numéro de dépôt: 97950244.0
(22) Date de dépôt: 05.12.1997
(51) Int. Cl.: C12N 15/86, C12N 15/11, C12N 9/02, C12N 5/10, C12Q 1/26, C12Q 1/68, C07K 16/40, A61K 38/44, A61K 39/395, A61K 48/00, G01N 33/50, G01N 33/573

(54) **SEQUENCE NUCLEOTIDIQUE CODANT POUR UNE FLAVINE MONO-OXGYNASE, PROTEINE CORRESPONDANTE ET LEURS APPLICATIONS DANS LES DOMAINES DU DIAGNOSTIC ET DE LA THERAPIE**
NUKLEOTIDSEQUENZ, DIE FÜR EINE FLAVINMONOOXYGENASE KODIERT, DAS KORRESPONDIERENDE PROTEIN, SOWIE DEREN VERWENDUNGEN IN DIAGNOSTIK UND THERAPIE
NUCLEOTIDE SEQUENCE CODING FOR A FLAVIN-CONTAINING MONOOXYGENASE, CORRESPONDING PROTEIN AND THEIR APPLICATIONS IN THE FIELDS OF DIAGNOSIS AND THERAPY

(30) Priorité: 06.12.1996 FR 9615032
(43) Date de publication de la demande: 15.09.1999
(73) Titulaire: Serono Genetics Institute S.A., 91030 Evry Cedex (FR); INSTITUT NATIONAL DE LA SANTE ET DE LA RECHERCHE MEDICALE (INSERM), 75013 Paris (FR)
(72) Inventeur: BLUMENFELD, Marta, F-75013 Paris (FR); TCHOUMAKOV, Ilia, F-77000 Vaux le Penil (FR); GARCHON, Henri-Jean, F-75016 Paris (FR); BACH, Jean-François, F-75007 Paris (FR)
(74) Mandataire: Warcoin, Jacques
(86) Numéro de dépôt international: PCT/FR1997/002226
(87) Numéro de publication internationale: WO 1998/024914

(56) Documents cités:
- EP-A- 0 712 932
- MCCOMBIE R. ET AL.: "Localization of human flavin-containing monooxygenase genes FMO2 and FMO5 to chromosome 1q" GENOMICS, vol. 34, no. 3, 15 juin 1996, pages 426-429, XP002039325
- YUEH M. ET AL.: "AC U59453" EMBL DATABASE, 25 juin 1996, HEIDELBERG, XP002039326
- PHILLIPS I. ET AL.: "The molecular biology of the flavin-containing monooxygenases of man" CHEMICO-BIOLOGICAL INTERACTIONS, vol. 96, no. 1, 28 avril 1995, pages 17-32, XP002039327
- LARSEN-SU S. AND WILLIAMS D.: "Dietary indole-3-carbonil inhibits FMO activity and the expression of flavin-containing monooxygenase form 1 in rat liver and intestine" DRUG METABOLISM AND DISPOSITION, vol. 24, no. 9, septembre 1996, pages 927-931, XP002039328
- SUNDEN S. ET AL.: "Fine mapping of the autosomal dominant juvenile open angle glaucoma (GLC1A) region and evaluation of candidate genes" GENOME RESEARCH, vol. 6, no. 9, septembre 1996, pages 862-869, XP002039329 cité dans la demande
- LAWTON M. ET AL.: "A nomenclature for the mammalian flavin-containing monooxygenase gene family based on amino acid sequence identities" ARCHIVES OF BIOCHEMISTRY AND BIOPHYSICS, vol. 308, no. 1, janvier 1994, pages 254-257, XP002039330 cité dans la demande
- GASSER R.: "The flavin-containing monooxygenase system" EXPERIMENTAL AND TOXICOLOGIC PATHOLOGY, vol. 48, no. 5, septembre 1996, pages 467-470, XP002039331
- DOLPHIN C. ET AL.: "AC Y09267" EMBL DATABASE, 6 février 1997, HEIDELBERG, XP002060752

## Description

La présente invention concerne une flavine mono-oxygénase 2 humaine (hFMO2) mutée, ainsi que ses séquences nucléotidiques et polypeptidiques. La présente invention concerne également, des vecteurs de clonage et/ou d'expression contenant lesdites séquences nucléotidiques et des cellules transformées par ces vecteurs ainsi que des méthodes de préparation desdits polypeptides. L'invention comprend aussi des méthodes de sélection de composés et de diagnostic de prédisposition à des pathologies et/ou des déficiences liées à cette FMO2 humaine mutée ainsi que des compositions pharmaceutiques comportant lesdits composés destinés au traitement et/ou à la prévention de ces pathologies.

Les flavines mono-oxygénases (FMOs) (Lawton et al., 1994) forment une famille d'enzymes microsomales catalysant l'oxydation NADPH-dépendante de nombreux composés organiques exogènes (xénobiotiques) possédant un hétéroatome nucléophile comme en particulier l'atome d'azote, de soufre, de phospore ou de sélénium (Ziegler, D.M., 1988 ; Ziegler, D.M., 1993), qu'il s'agisse de médicaments, de pesticides ou autres substances potentiellement toxiques. La cystéamine est le seul substrat endogène actuellement connu des FMOs.

Les FMOs représentent une famille multigénique. L'expression de formes différentes de FMOs est dépendante à la fois du tissu et de l'espèce considérés.

Les FMOs ont été localisées dans différents types de tissus, en particulier le foie, les poumons et les reins.

A ce jour, cinq isoformes des FMOs ont été caractérisées dans l'espèce de référence, le lapin. Leur homologie est de 50-60 %. On peut également citer le document Yueh et al. (AC U59453, EMBL Database, Heidelberg) qui décrit la séquence de la protéine FMO2 de macaque. Quatre de ces isoformes, FMO1, FMO3, FMO4 et FMO5 ont été identifiées chez l'homme (séquences GeneBank M64082, M83772, Z11737 et L37080 respectivement). Parmi les espèces mammifères, l'homologie entre FMO orthologues est supérieure à 80 %. L'existence d'une FMO2, voire d'autres isoformes, chez l'homme, peut être raisonnablement postulée.

Les FMOs sont associées au réticulum endoplasmique et sont impliquées dans la détoxification de composés xénobiotiques, la mono-oxygénatian permettant de transformer le xénobiotique en substance plus polaire, étape préliminaire avant son excrétion. Elles peuvent également être impliquées dans l'activation métabolique de différents composés toxiques et/ou carcinogènes présents dans l'environnement.

Le mécanisme de la réaction FMO a été décrit de manière détaillée (Poulsen, L.L. et al., 1995). Par opposition à toutes les autres oxydases ou mono-oxygénases connues, les FMOs ont la propriété unique de former une enzyme intermédiaire stable 4α-hydropéroxy flavine, NADP(H)- et oxygène-dépendante, en l'absence de substrat oxydable. Parce que l'énergie de catalyse est déjà présente dans l'enzyme FMO avant le contact avec son substrat potentiel, l'adéquation du substrat n'a pas besoin d'être aussi précise que pour d'autres types d'enzymes. Cette caractéristique spécifique de la FMO est responsable de la grande variété de substrats acceptés par les FMOs (incluant par exemple les alkyl- et aryl-amines tertiaires et secondaires, de nombreuses hydrazines, thiocarbamides, thioamides, sulfides, disulfides, thiols).

De nombreuses molécules, composés actifs de médicaments, sont reconnues comme substrats des FMOs, soit pour une N-oxydation, soit pour une S-oxydation (Gasser, 1996), parmi lesquels on trouve notamment des antidépresseurs, des antipsychotiques, des anti-ulcéreux, des vasodilatateurs et des anti-hypertenseurs.

Bien que certains substrats de FMO soient oxydés en dérivés moins actifs, de nombreux composés nucléophiles peuvent être métabolisés en intermédiaires pouvant être plus réactifs et/ou potentiellement toxiques ; plutôt que d'être excrétés, de tels produits peuvent induire des réponses toxiques par fixation covalente à des macromolécules cellulaires, ou par d'autres mécanismes. Par exemple, les mercaptopyrimidines et les thiocarbamides peuvent être activés de manière prédominance par une activité FMO (Hines et al., 1994). De manière plus précise, il a été montré que la néphrotoxicité associée au conjugué glutathion de l'acroléine est liée à son métabolisme médié par la FMO rénale ; la FMO forme un S-oxyde qui est ensuite libéré, par réaction d'élimination catalysée en milieu basique, sous forme d'acroléine cytotoxique (Park, S.B. et al., 1992). Ainsi, les FMOs peuvent jouer un rôle important aussi bien dans les premières étapes de toxicité chimique que dans la détoxification de composés xénobiotiques.

Comme décrit ci-dessus, un grand nombre de médicaments aujourd'hui en phase d'essais cliniques, ou largement prescrits, contiennent des fonctions à caractère nucléophile de type azote, soufre, phosphore ou autres. Le rôle de la FMO dans le métabolisme oxydatif des médicaments et des composés chimiques endogènes chez l'homme est cependant mal connu.

Cashman et al. (1996) ont récemment étudié les contributions des enzymes FMOs dans le métabolisme physiologique de la cimétidine et de la S-nicotine in vivo. La plus grande partie de leurs résultats confirme le fait que l'activité FMO3 du foie d'adulte est responsable de l'oxygénation de la cimétidine et de la S-nicotine, cette oxygénation étant stéréospécifique. Les auteurs montrent en outre que la stéréochimie des métabolites principaux de la cimétidine et de la S-nicotine chez des petits animaux d'expérimentation est distincte de celle observée chez l'homme, et suggèrent que différentes isoformes de FMOs pouvant être prédominantes selon les espèces, ceci peut avoir des conséquences importantes quant au choix des animaux d'expérimentation pour les programmes d'élaboration et de développement de médicaments chez l'homme.

La FMO1 est connue pour être exprimée chez l'homme dans les reins, mais pas dans le foie. La FMO2 est exprimée majoritairement dans les poumons chez toutes les espèces de mammifères testées. La FMO3 a été isolée chez l'homme dans le foie où elle est prédominante chez l'adulte. La FMO3 est l'isoforme majeure impliquée dans la sulfoxydation de la méthionine et dans l'oxygénation stéréospécifique de la cimétidine et de la S-nicotine. La FMO3 présente une spécificité pour son substrat plus grande que celle des FMO1 trouvées dans le foie de la plupart des espèces animales étudiées. La FMO4 est une isoforme mineure dont la fonction et la spécificité de substrat sont peu connues. Elle est présente dans le foie humain et est aussi exprimée dans le cerveau où elle pourrait être impliquée dans l'oxydation de médicaments antidépresseurs comme l'imipramine. La FMO5 est exprimée dans le foie de l'homme de manière moins importante que la FMO3. Son apparent manque d'efficacité en tant qu'enzyme impliquée dans le métabolisme de médicaments, suggère qu'elle pourrait être impliquée dans une fonction physiologique.

Les différents profils d'expression des isoformes de FMO selon les tissus et/ou espèces constituent donc probablement un facteur significatif contribuant aux différences d'activités FMO observées entre les tissus et/ou entre espèces.-Ainsi, la variété des formes de FMOs pourrait avoir un impact significatif sur la différence des réponses de tissus et/ou espèces à l'exposition à un composé xénobiotique. En effet, les différences observées entre les tissus et/ou espèces dans la réponse aux composés xénobiotiques et dans leur toxicité sont liées pour une part importante aux variations d'activité et de spécificité impliquées dans le métabolisme de ces substrats par les FMOs. Facteurs génétiques et spécificité tissulaire dans l'expression des FMOs sont des facteurs importants de ces variations.

Concernant les facteurs génétiques, il a été décrit par exemple que la triméthylaminurie, pathologie présente chez 1 % de sujets blancs britanniques et qui se manifeste par une forte odeur de poisson avarié dans l'air expiré, la sueur ou l'urine, est liée à une déficience d'origine génétique du fonctionnement d'une FMO hépatique.

Pour les raisons évoquées précédemment, il existe donc aujourd'hui un besoin important d'identifier de nouvelles isoformes de FMO ainsi que les polymorphismes génétiques éventuellement associés, présentant des spécificités quant à leurs substrats et/ou leur profil d'expression tissulaire, qui pourraient être impliquées dans le métabolisme de xénobiotiques, tel que le métabolisme de médicaments ou de substances exogènes présentes dans l'environnement comme par exemple les pesticides, ou encore qui pourraient être impliquées dans une fonction physiologique. Ceci est précisément l'objet de la présente invention.

Plusieurs gènes de la famille des FMOs humaines ont été localisés sur la région 1q23-25 du chromosome 1 par hybridation *in situ* du chromosome en métaphase.

Dès lors qu'une telle région candidate a été définie, il est nécessaire d'avoir accès au fragment du génome couvrant l'intervalle où se situe(nt) le(s) gène(s) recherché(s). Cette étape passe par l'établissement d'une carte physique, à savoir le recouvrement de la région par un ensemble de fragments clonés et ordonnés. Aujourd'hui, grâce aux données de la carte intégrée CEPH/Généthon du génome humain, environ 80 % du génome est recouvert par des clones de YACs, sous clonés en BACs dont la localisation sur les chromosomes se fait par l'intermédiaire de marqueurs polymorphes et génétiquement ordonnés (Chumakov et al., 1995). Cette carte physico-génétique permet de gagner un temps considérable, notamment par l'utilisation du séquençage exhaustif des régions d'intérêt.

Ainsi selon la présente invention, il a été établi, après localisation du BAC 123H04M sur le locus génétique 1q24-25 précédemment cité, que l'insertion qu'il porte contient les parties 3' de hFM03, 5' de hFMO1, ainsi que la séquence complète de hFM02, et celle d'un autre nouveau gène membre de la famille FMO, le hFMOx.

En outre, grâce à l'utilisation de banques d'étiquettes 5', on peut vérifier l'expression des gènes candidats identifiés comme précédemment : l'identification d'une étiquette hybridant à l'une des séquences candidates indique, puisque celle-ci est issue d'une banque d'ADNc, la présence d'ARNm et donc d'une expression des séquences en question dans les tissus considérés.

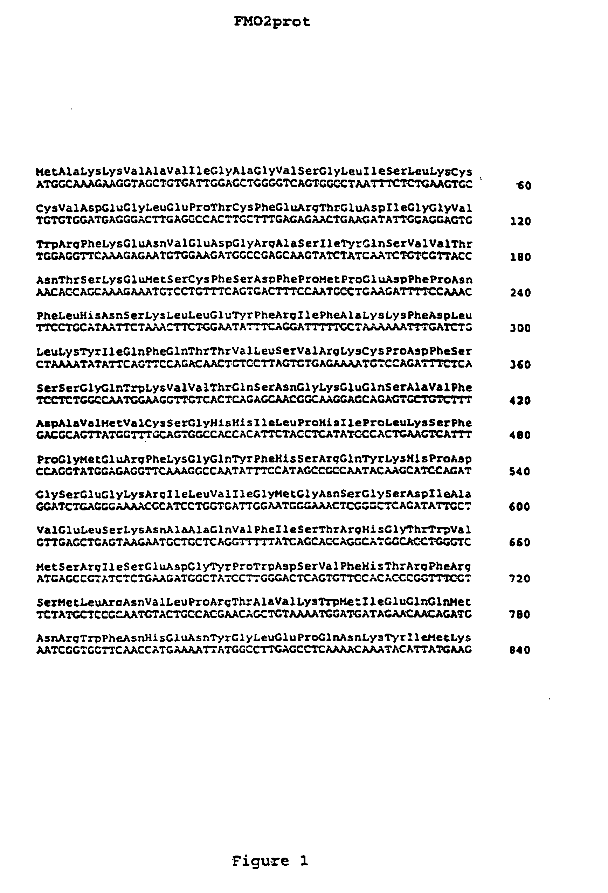

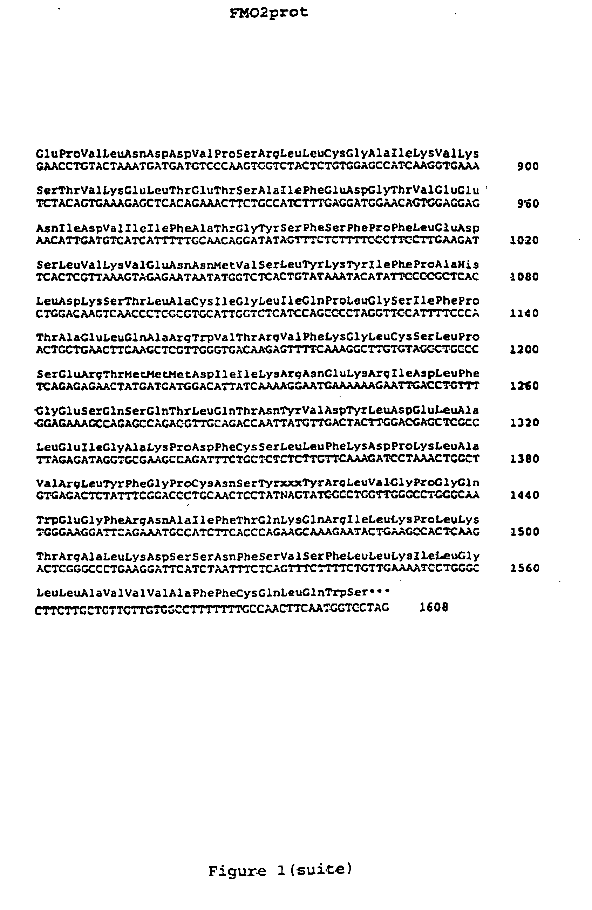

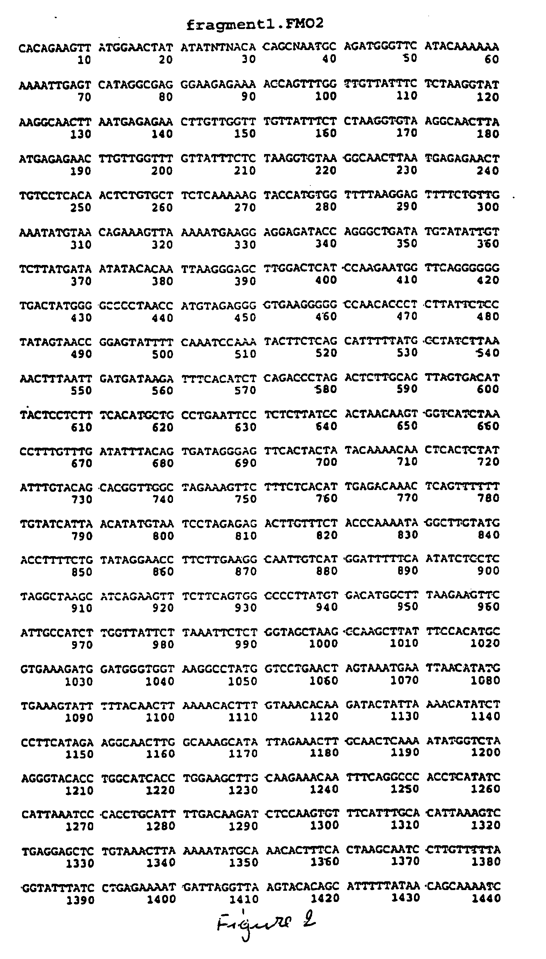

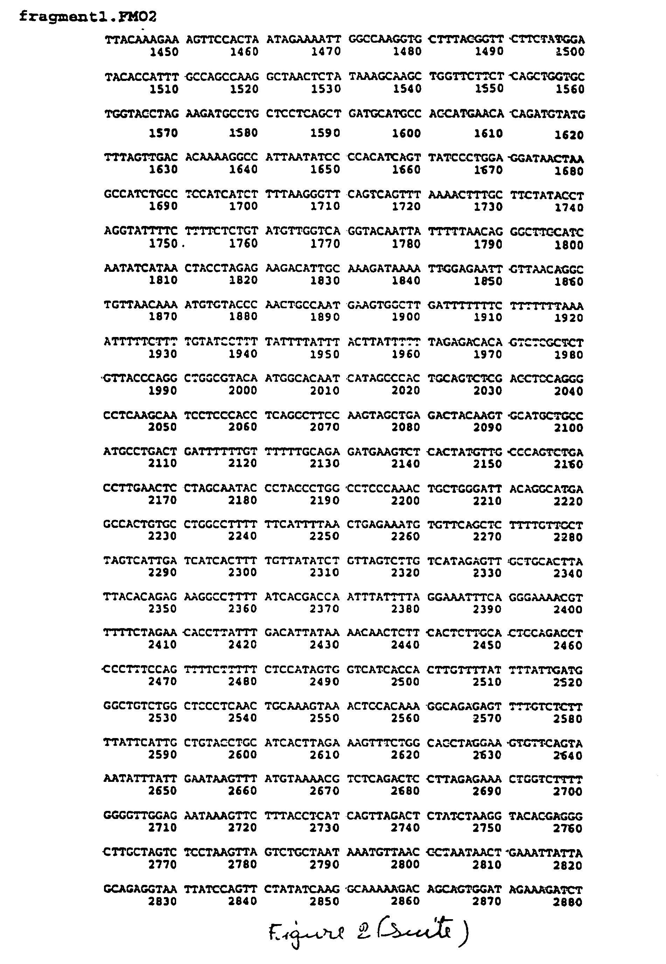

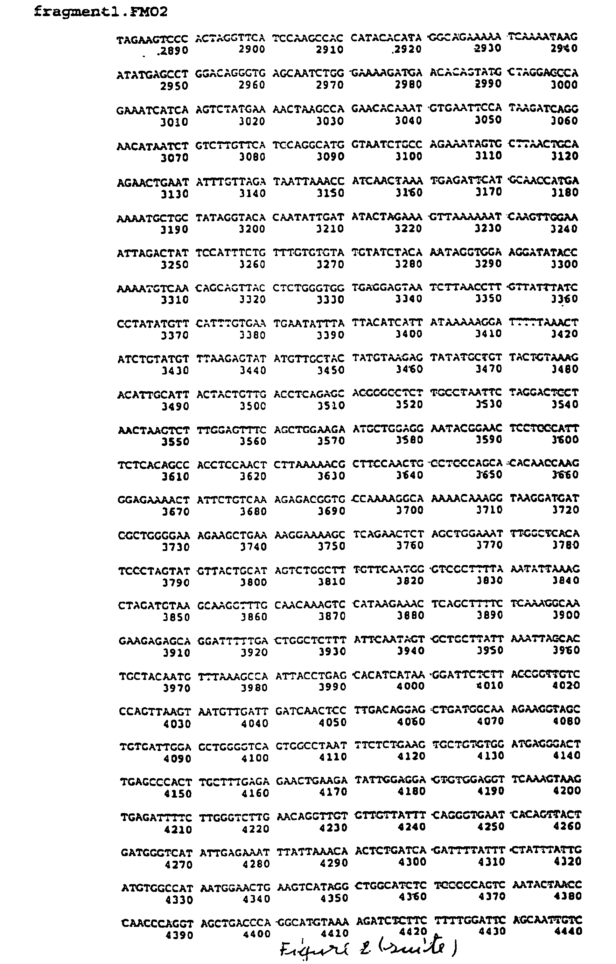

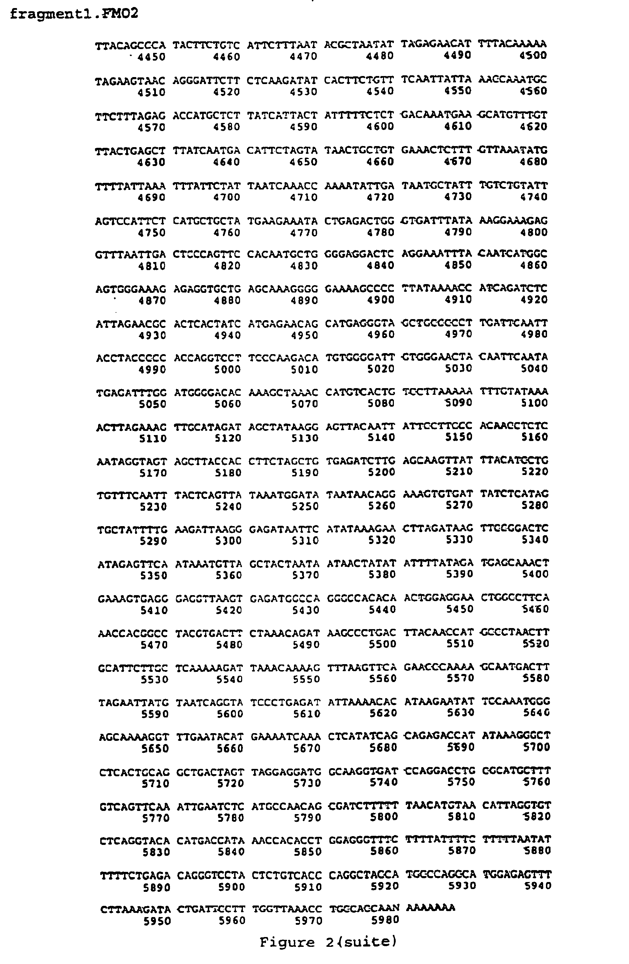

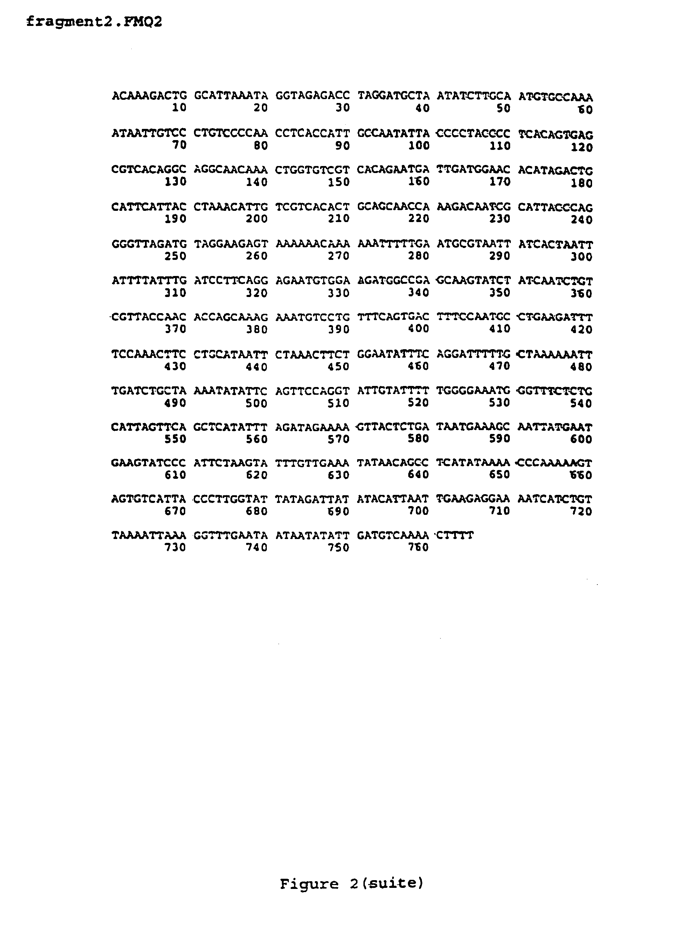

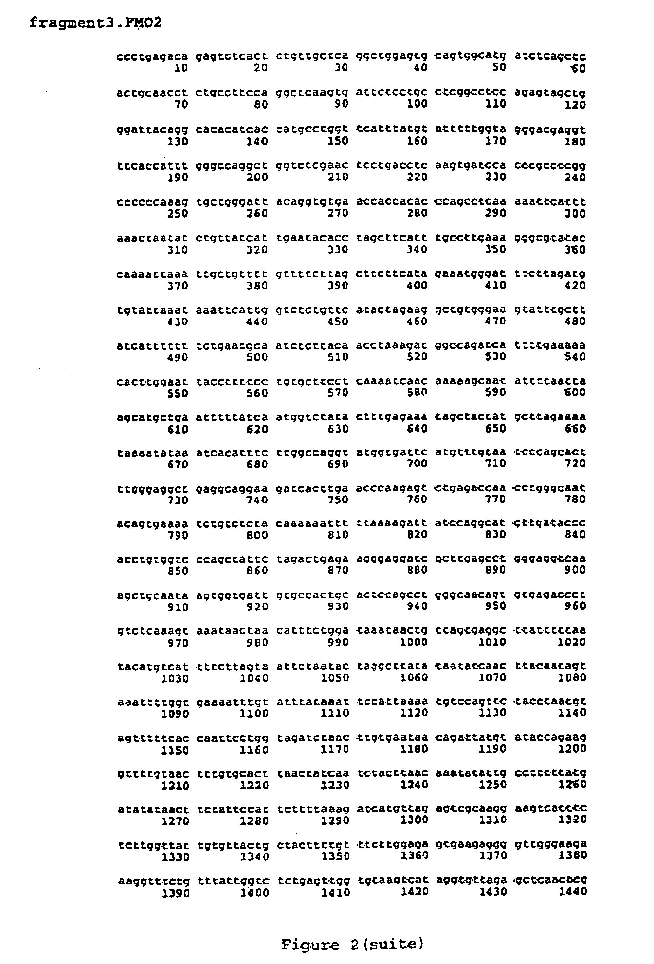

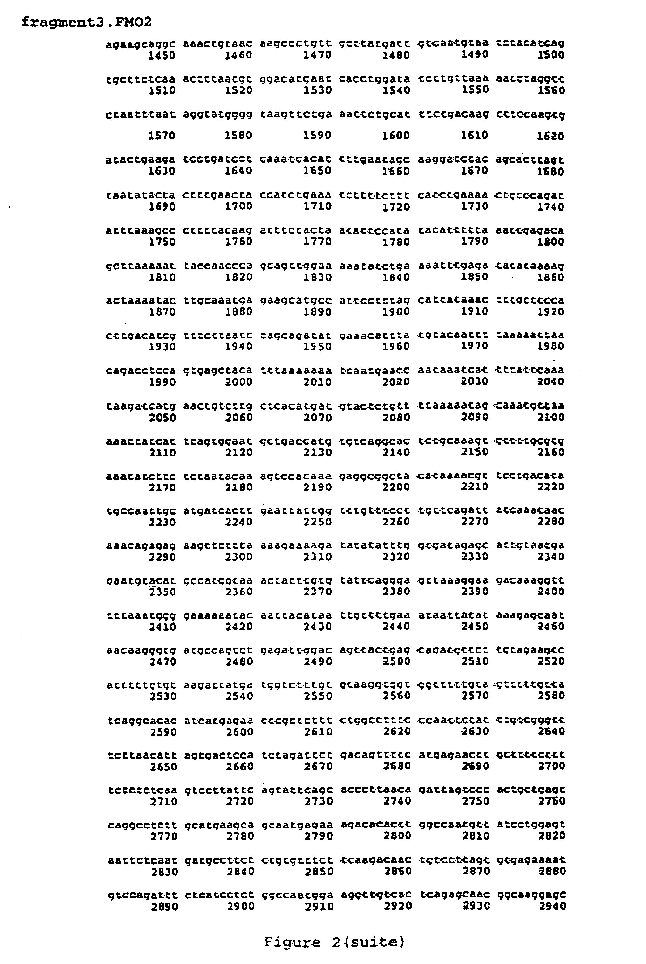

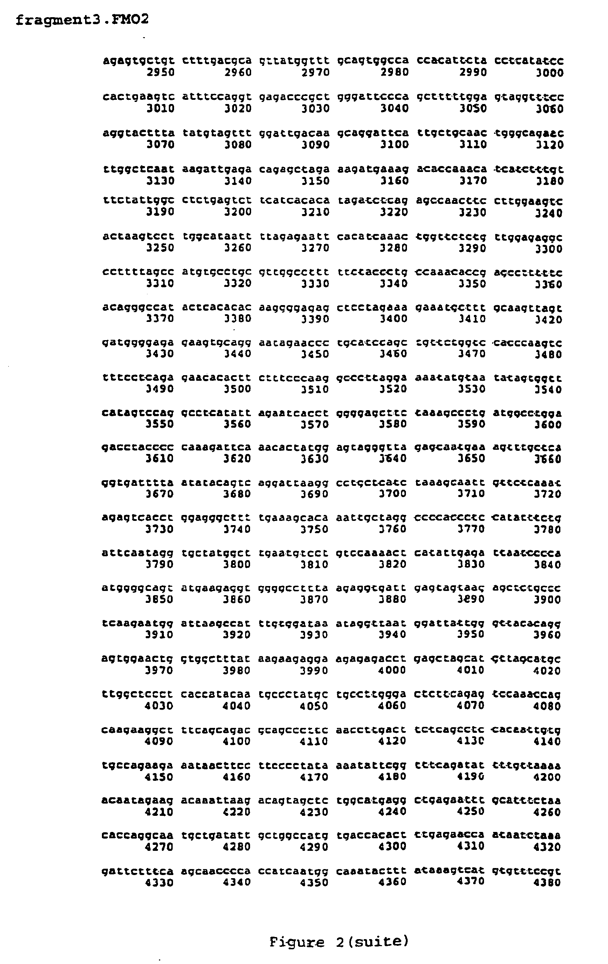

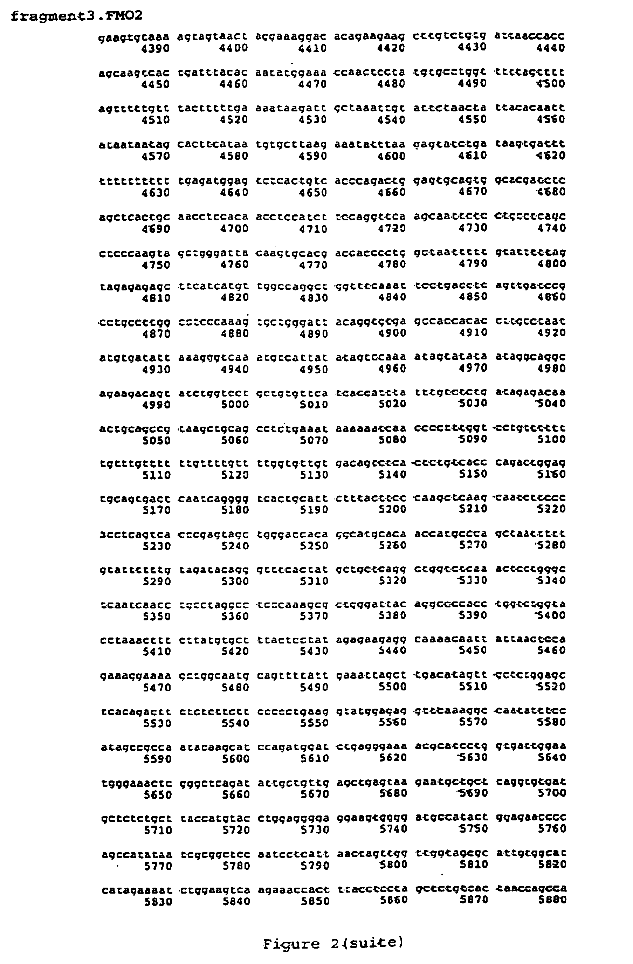

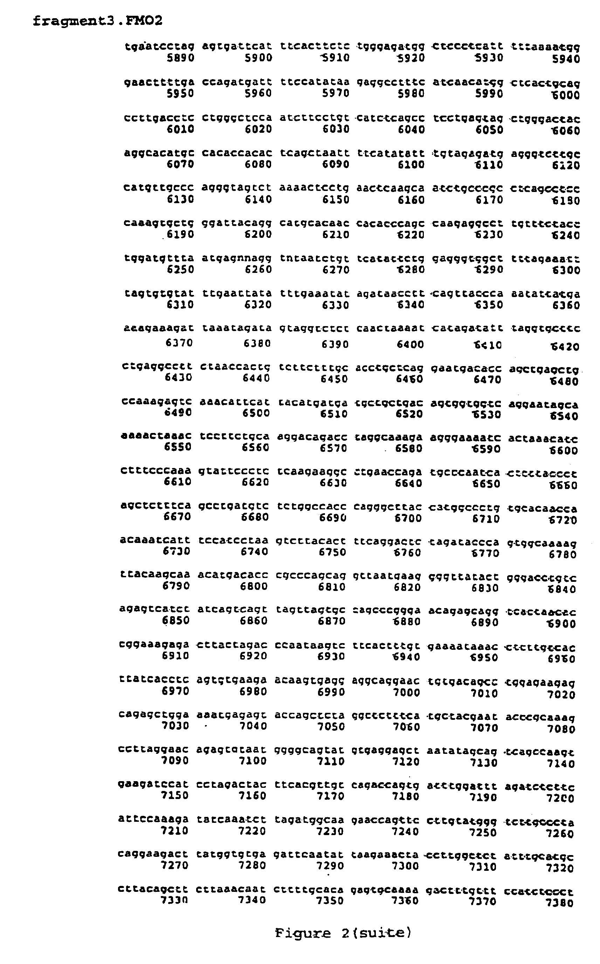

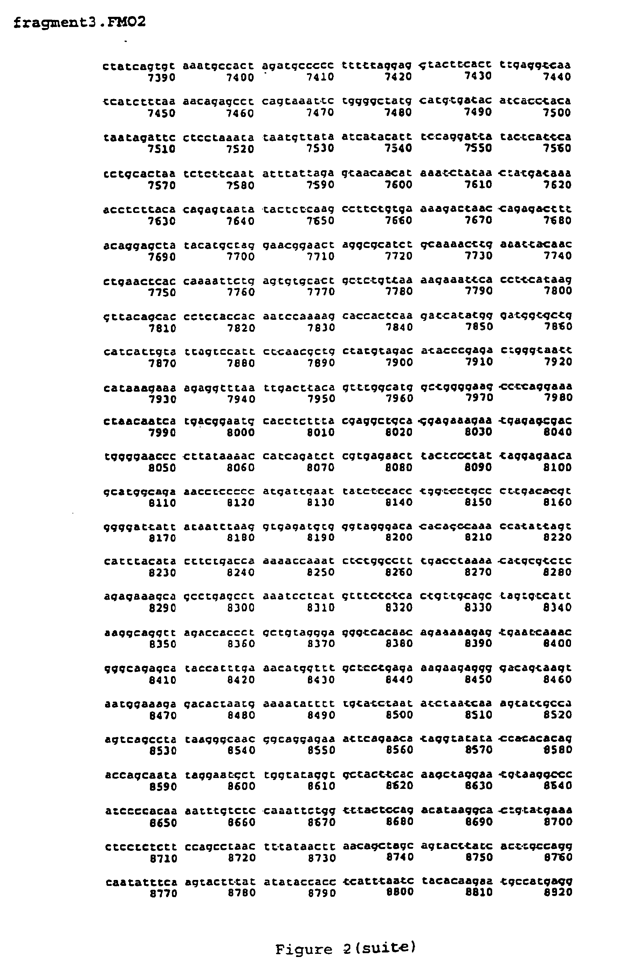

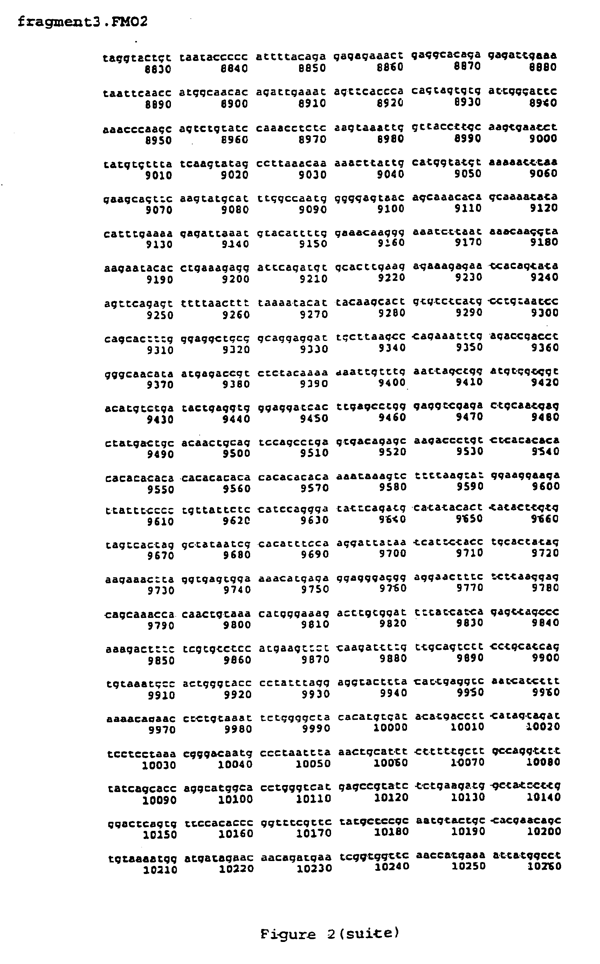

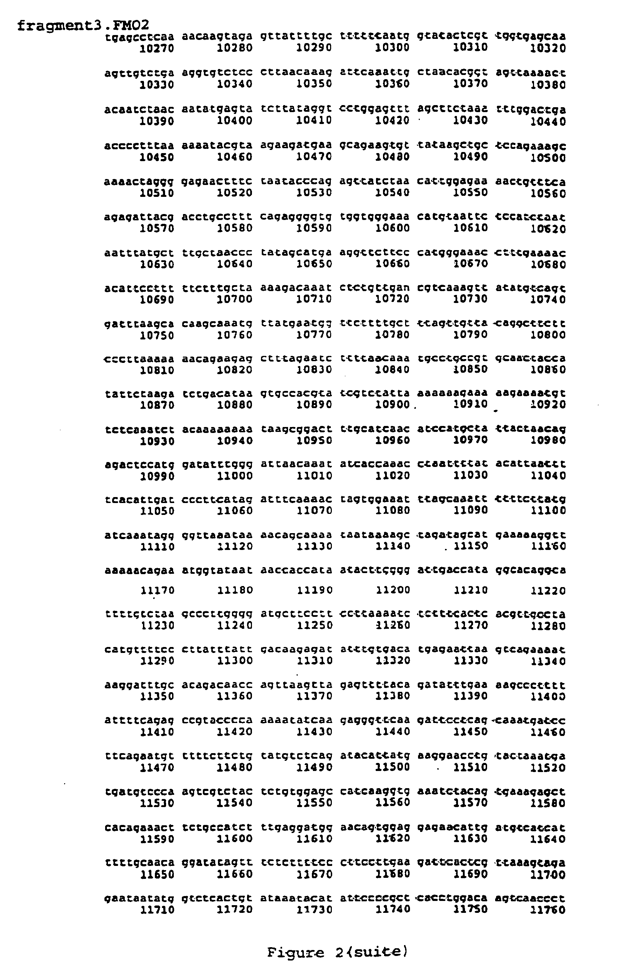

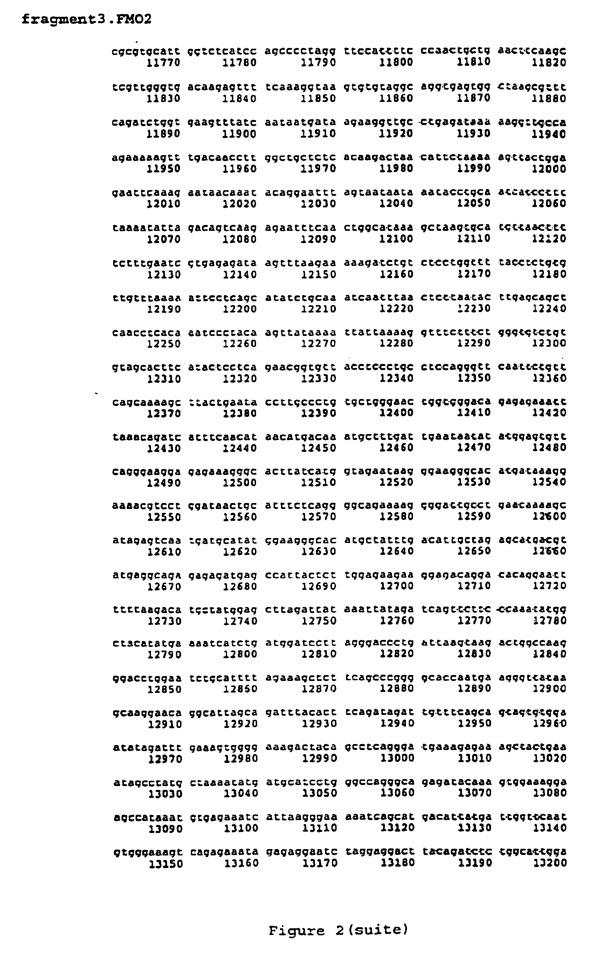

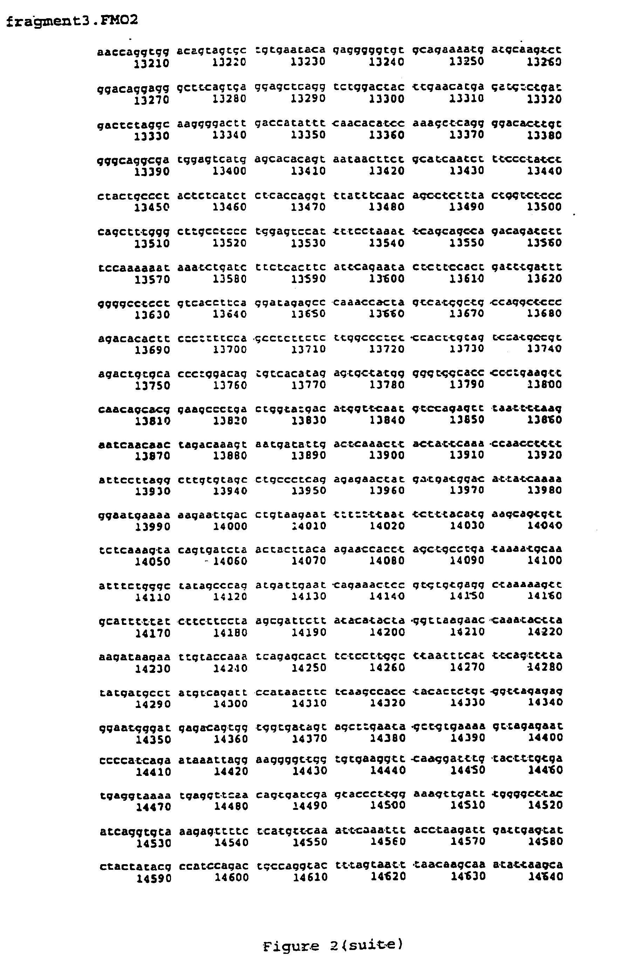

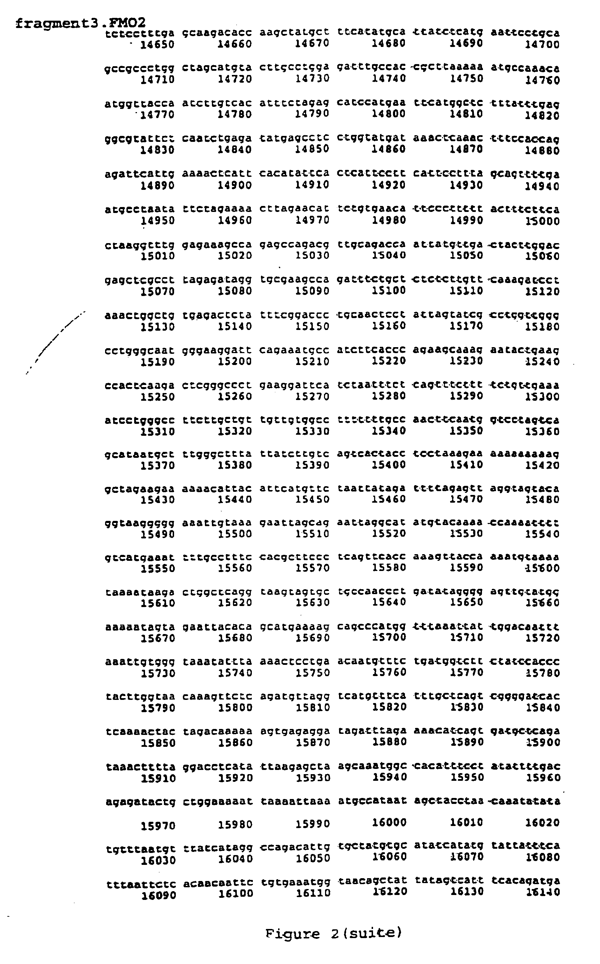

La présente invention décrit notamment un polynucléotide isolé, dont la séquence SEQ ID N° 1 est partiellement représentée sur la Figure 2, et qui code pour un polypeptide de séquence SEQ ID N° 3 représentée sur la Figure 1.

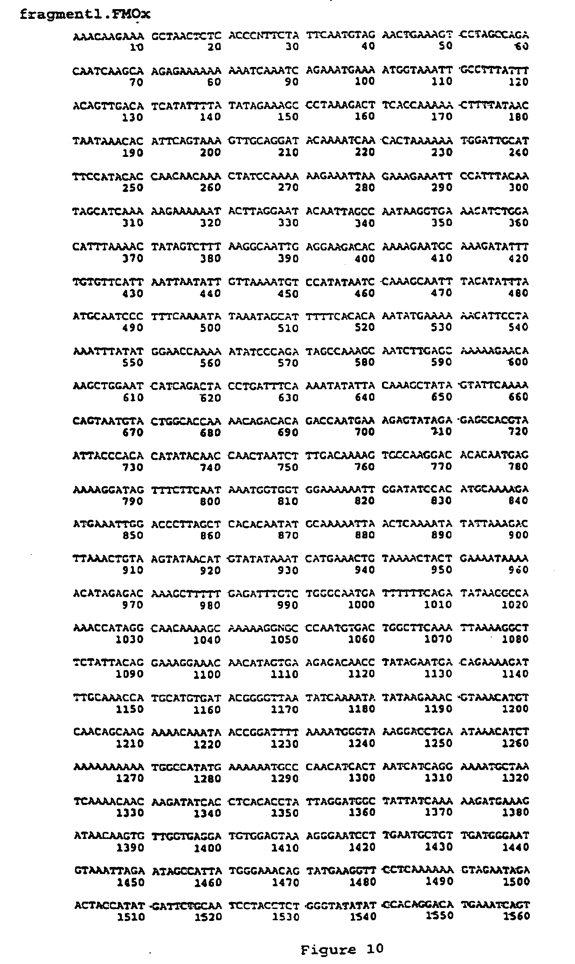

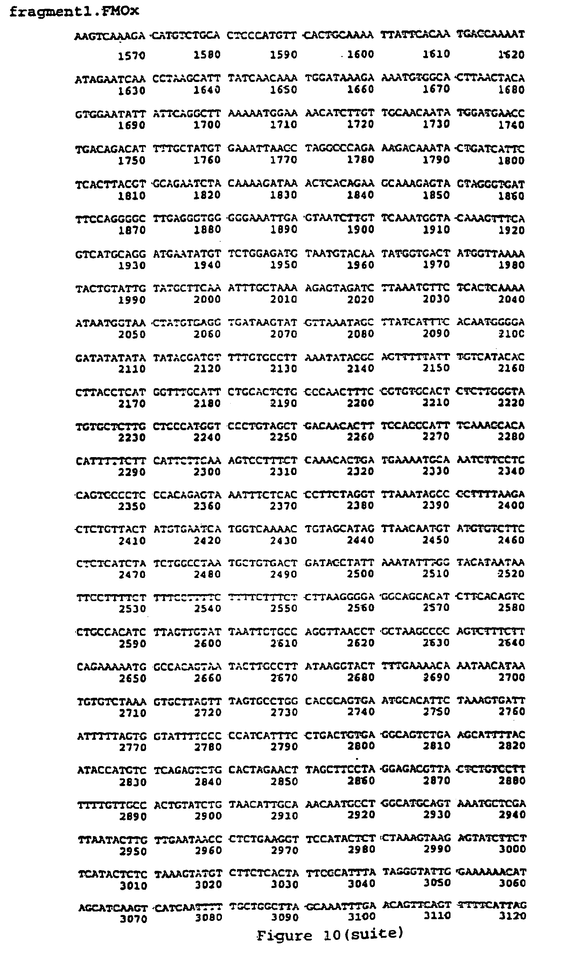

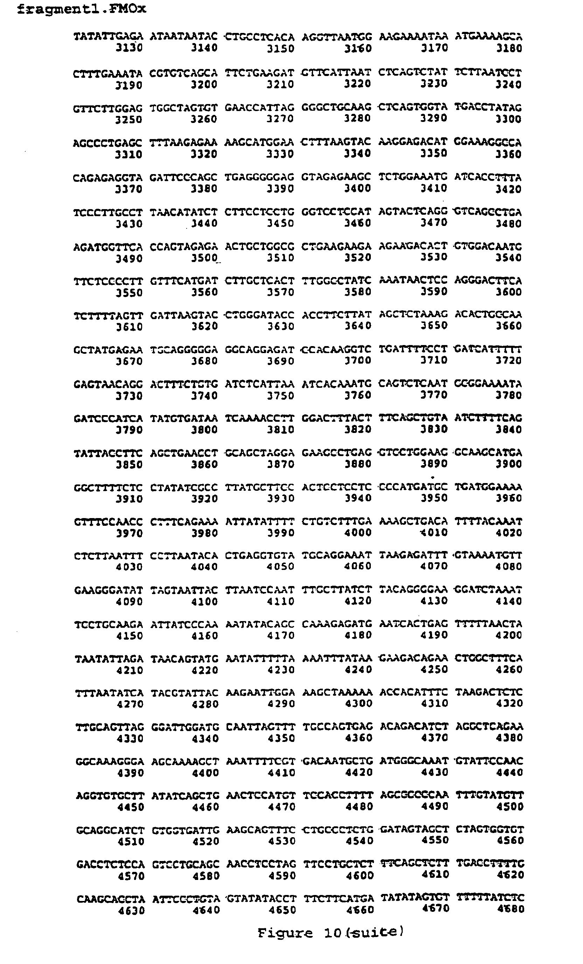

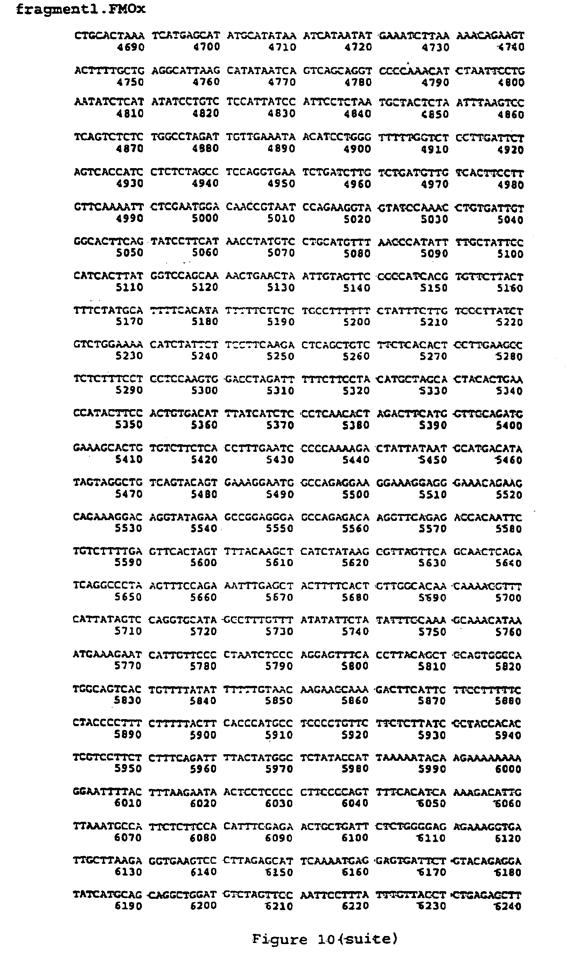

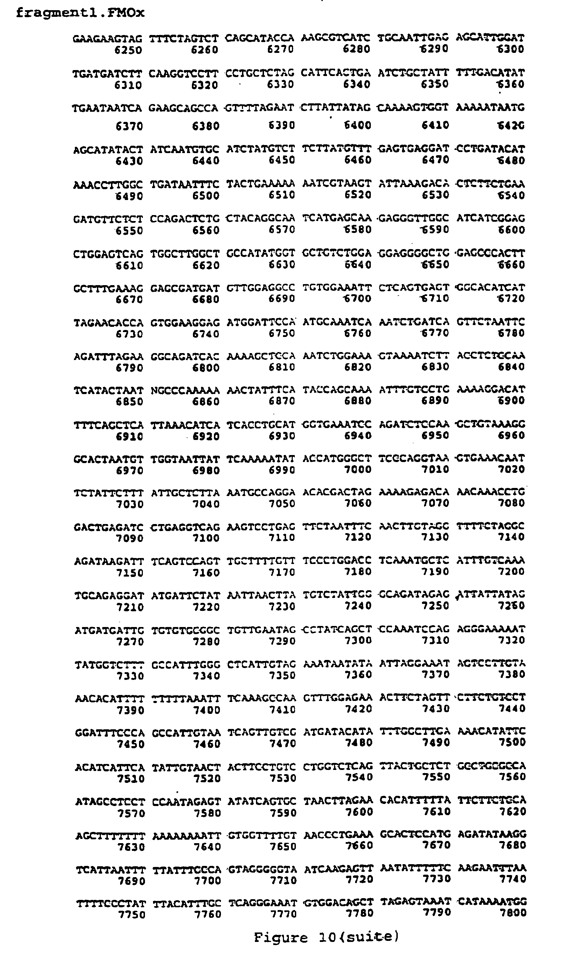

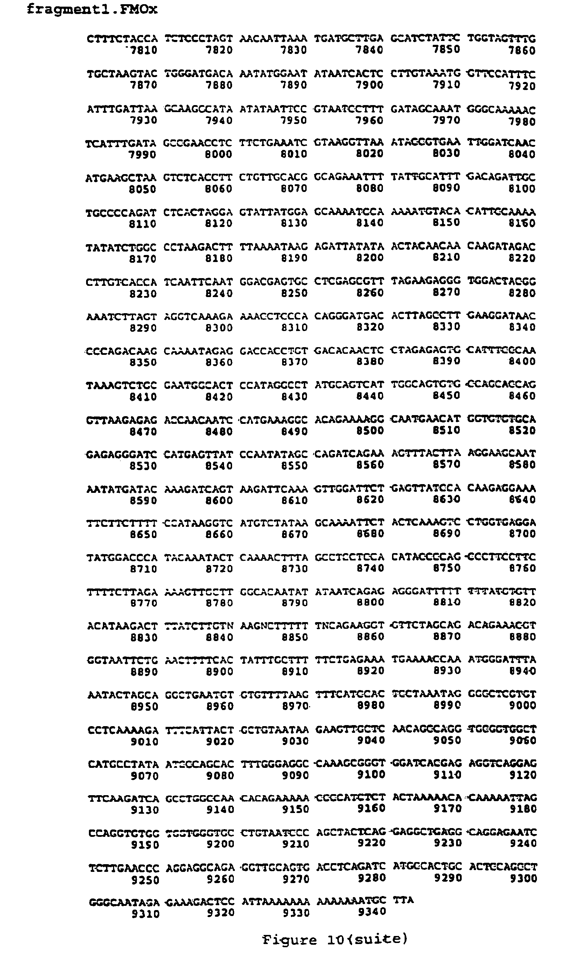

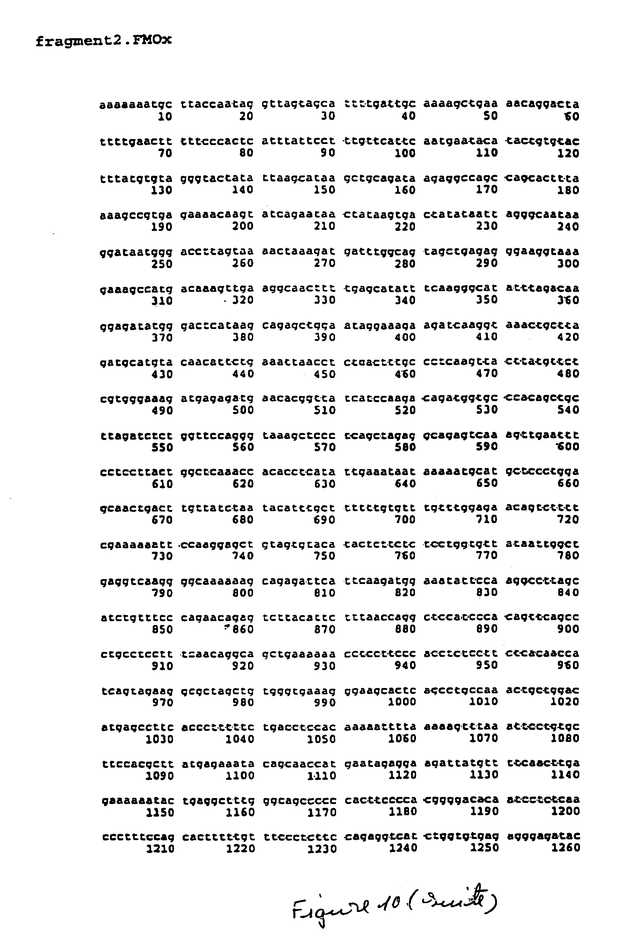

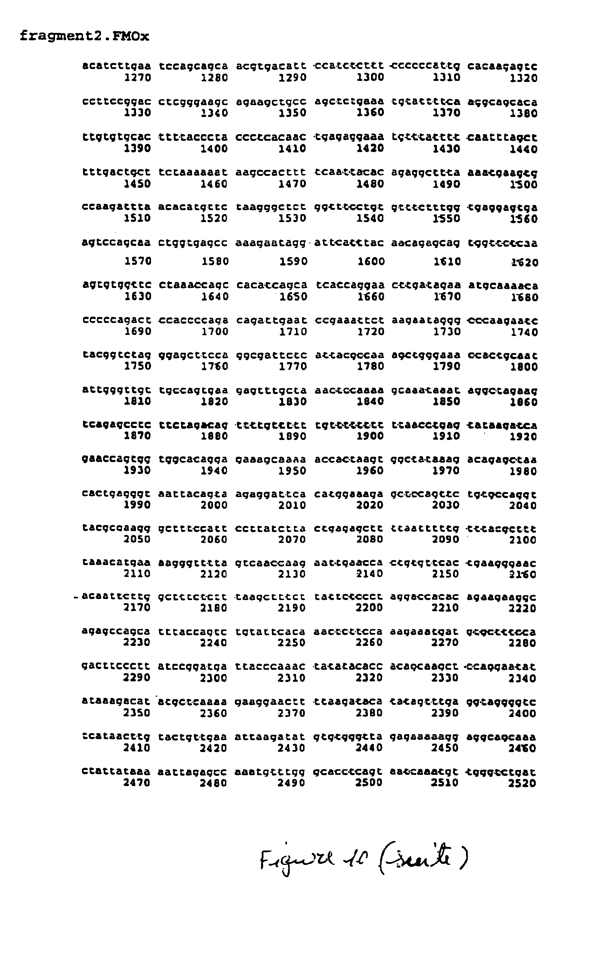

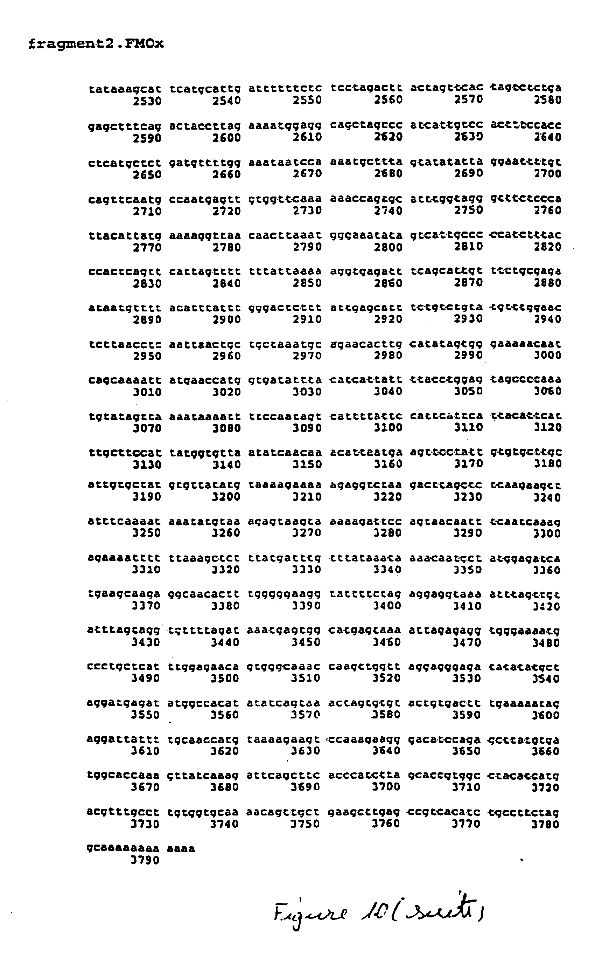

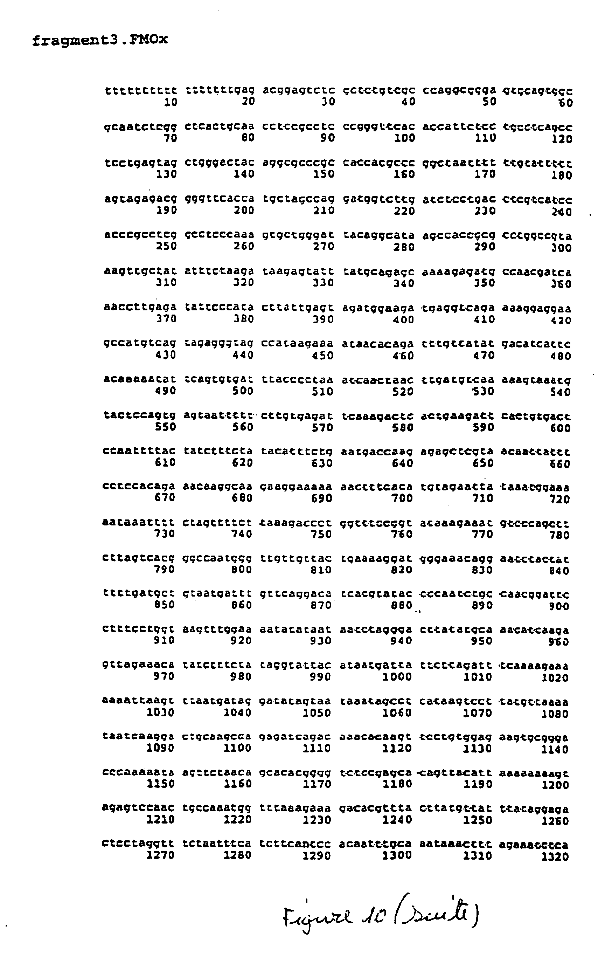

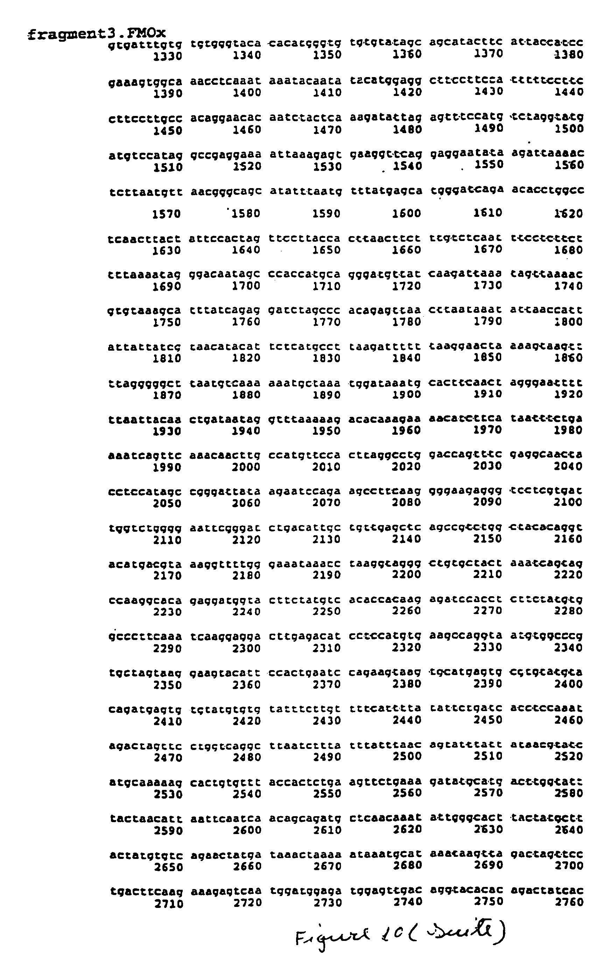

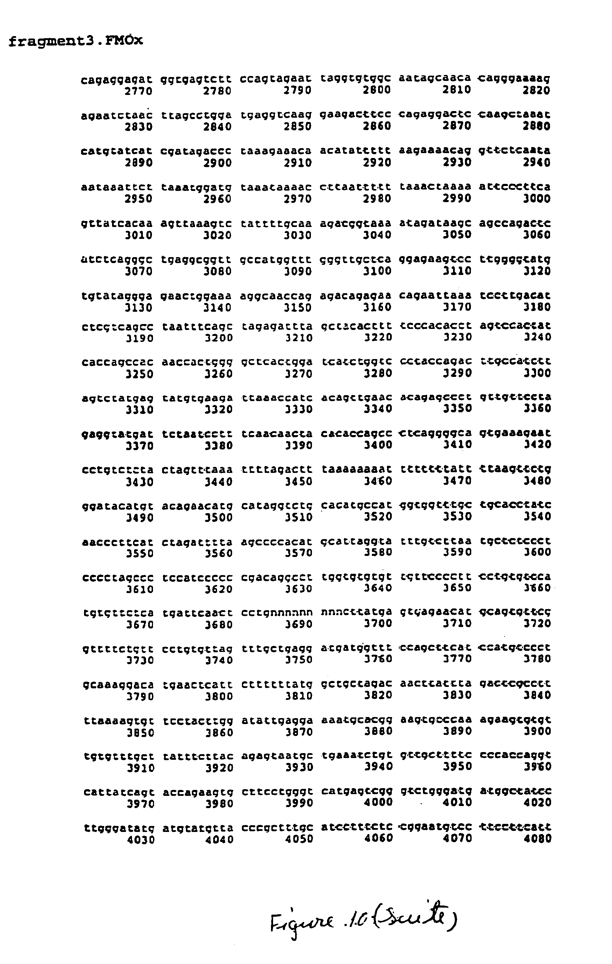

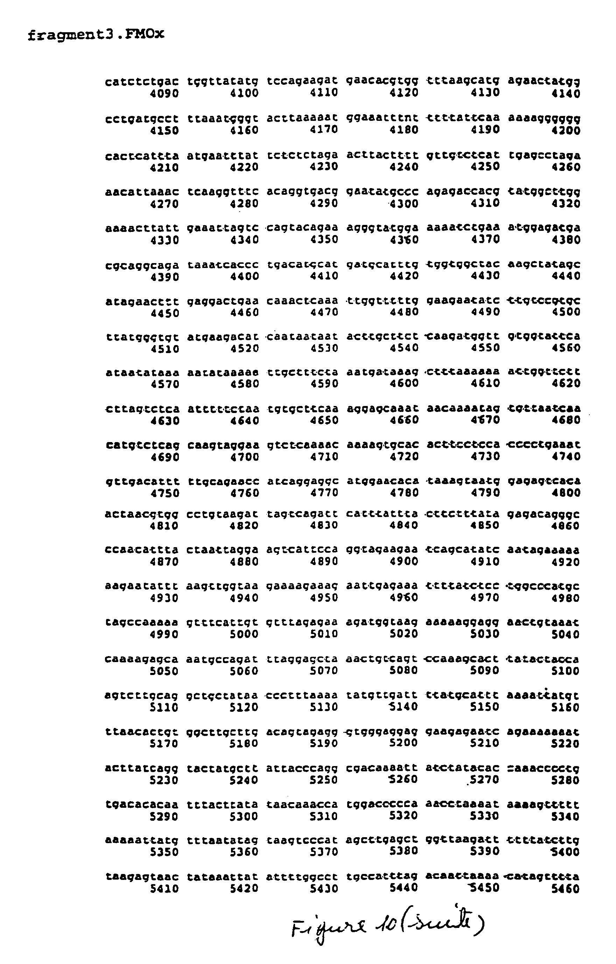

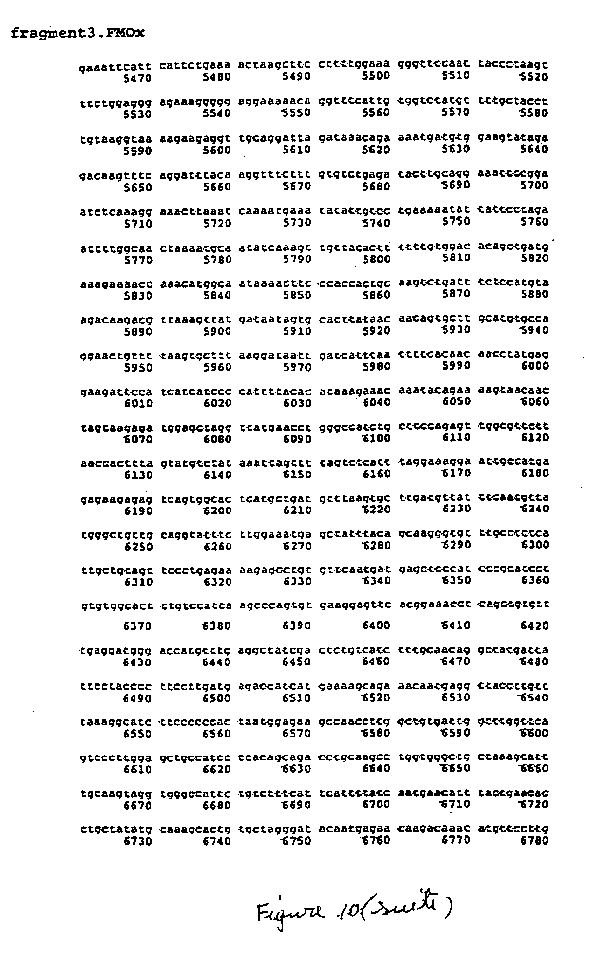

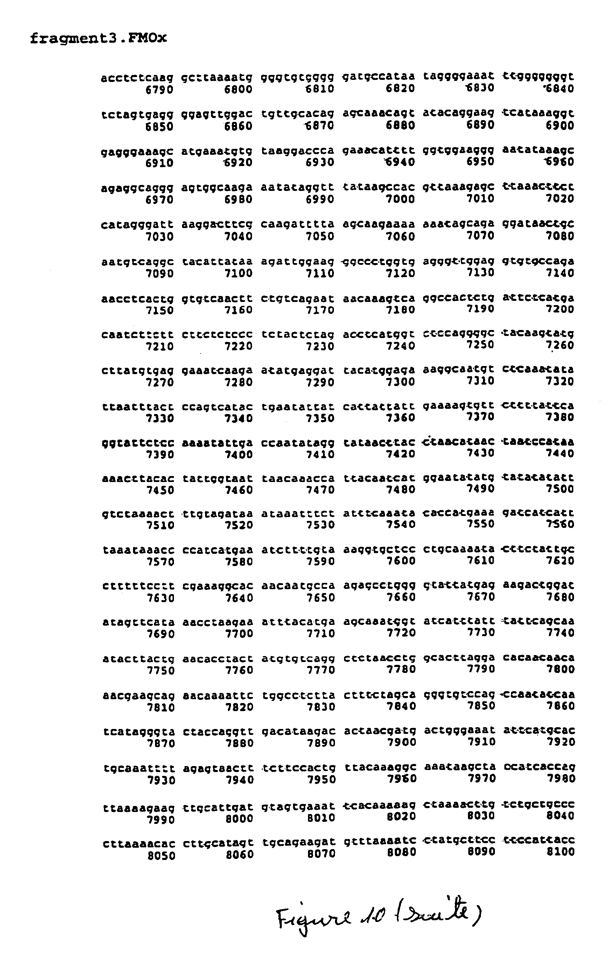

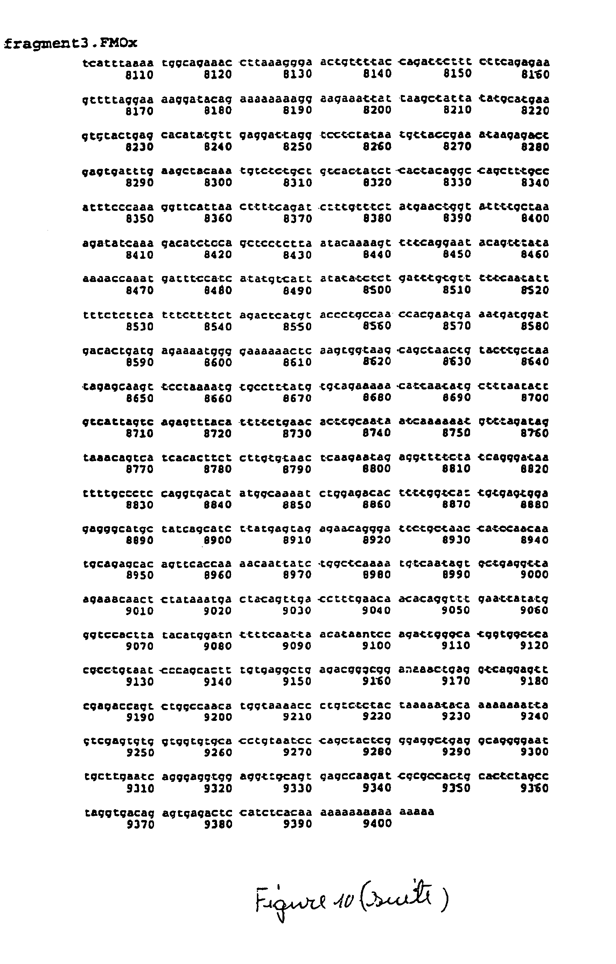

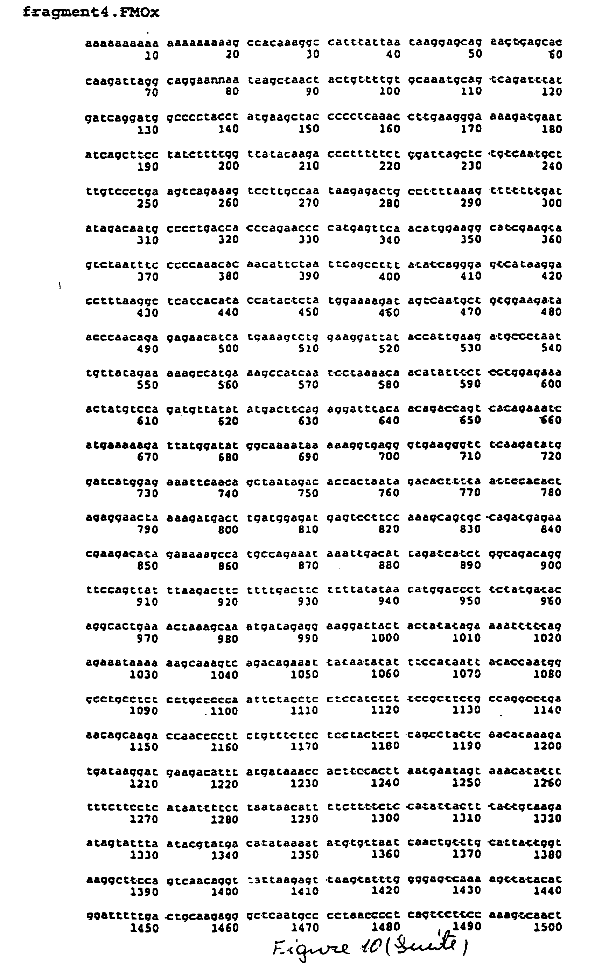

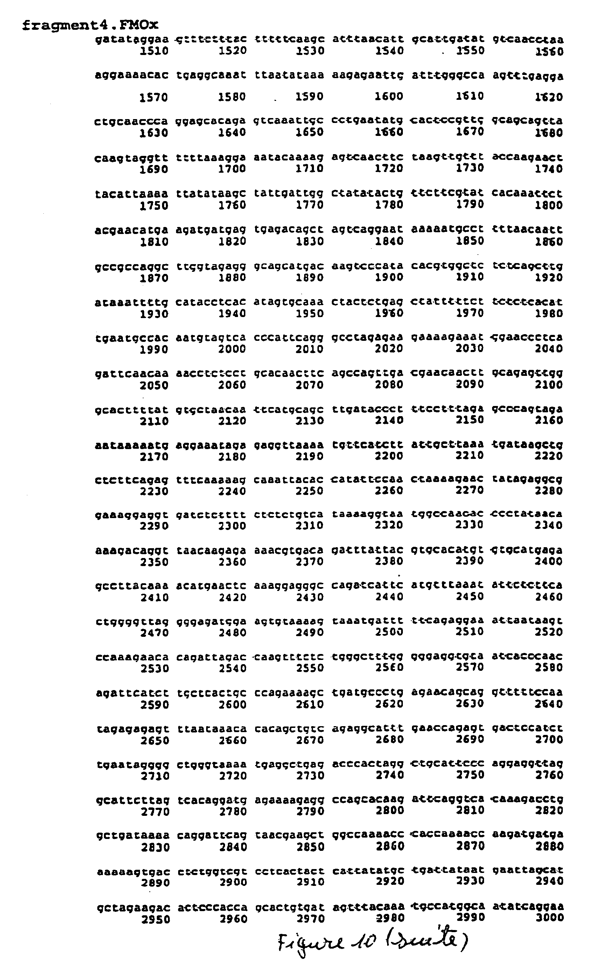

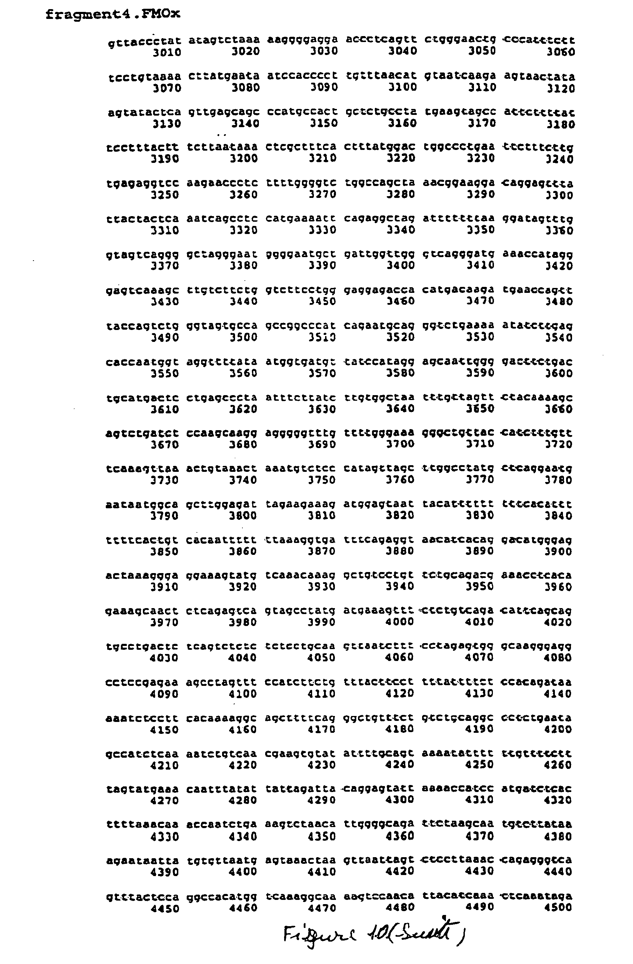

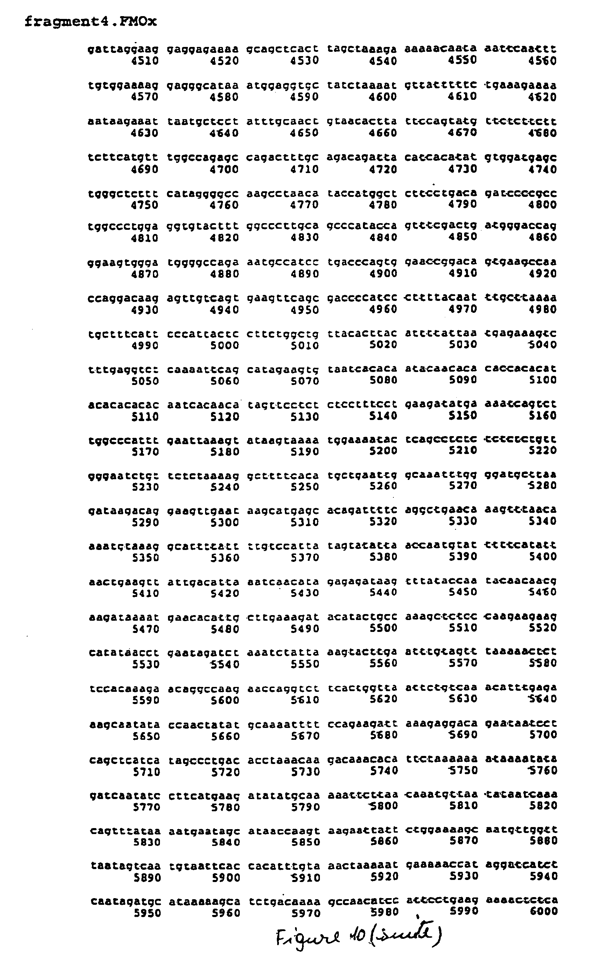

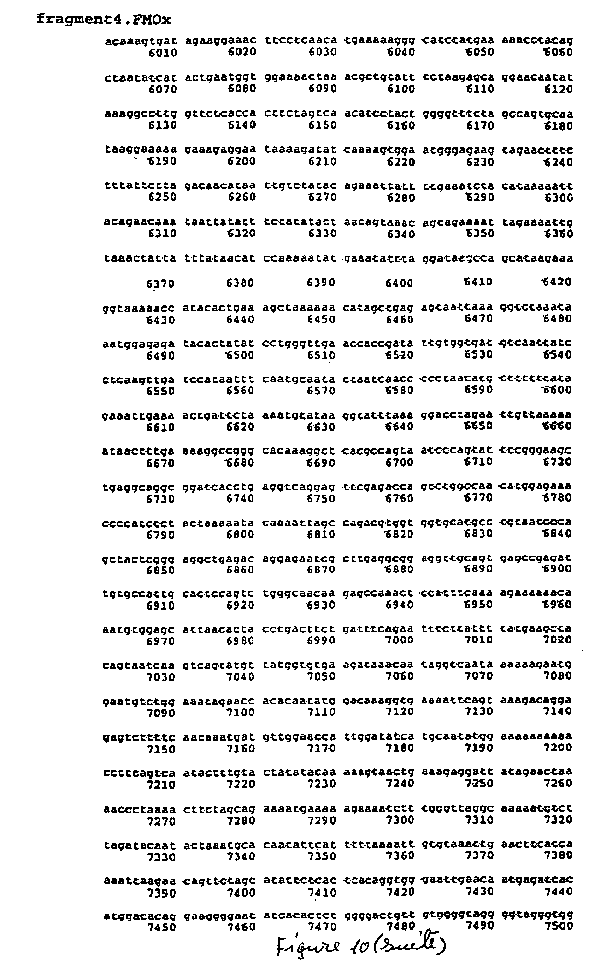

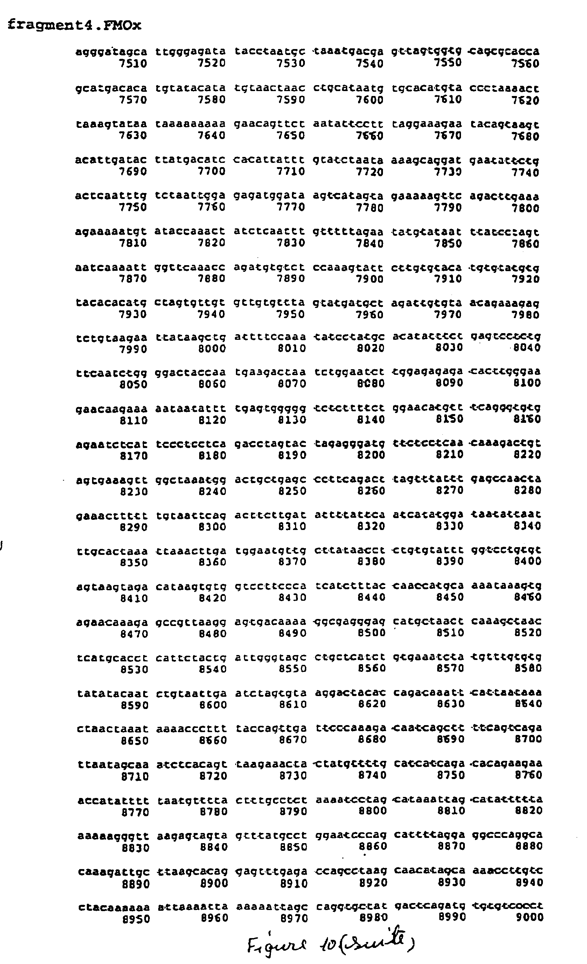

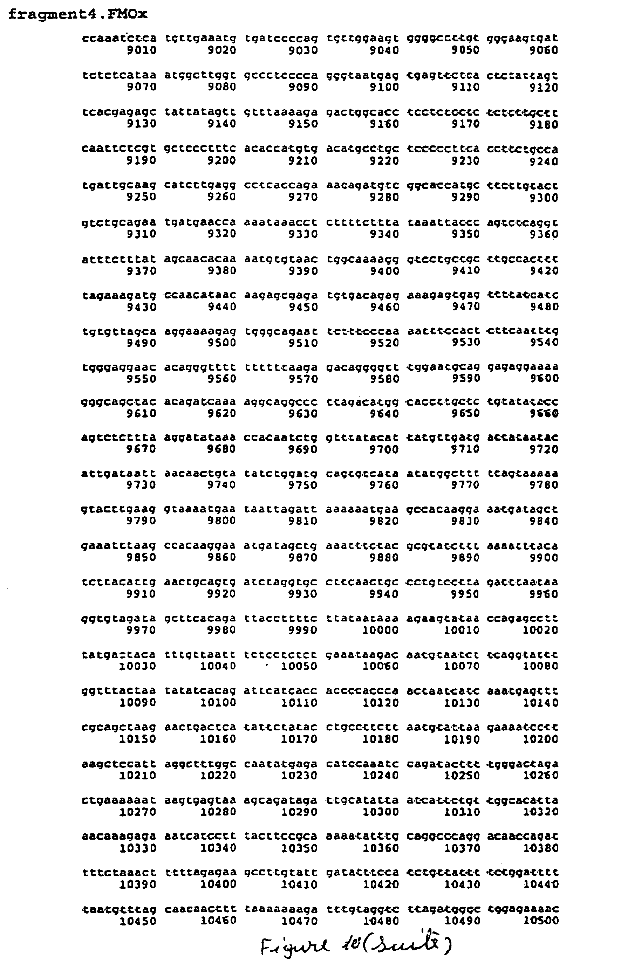

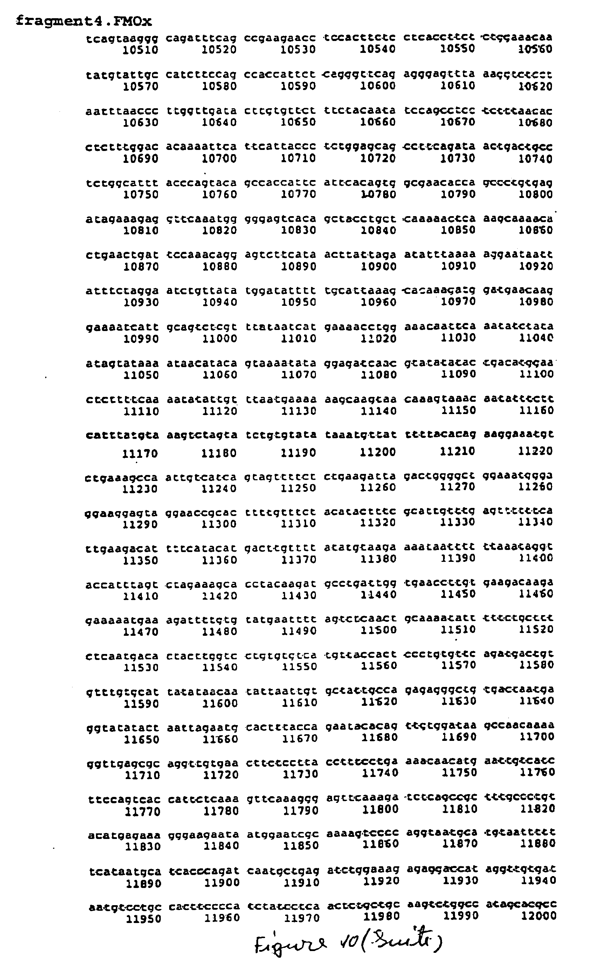

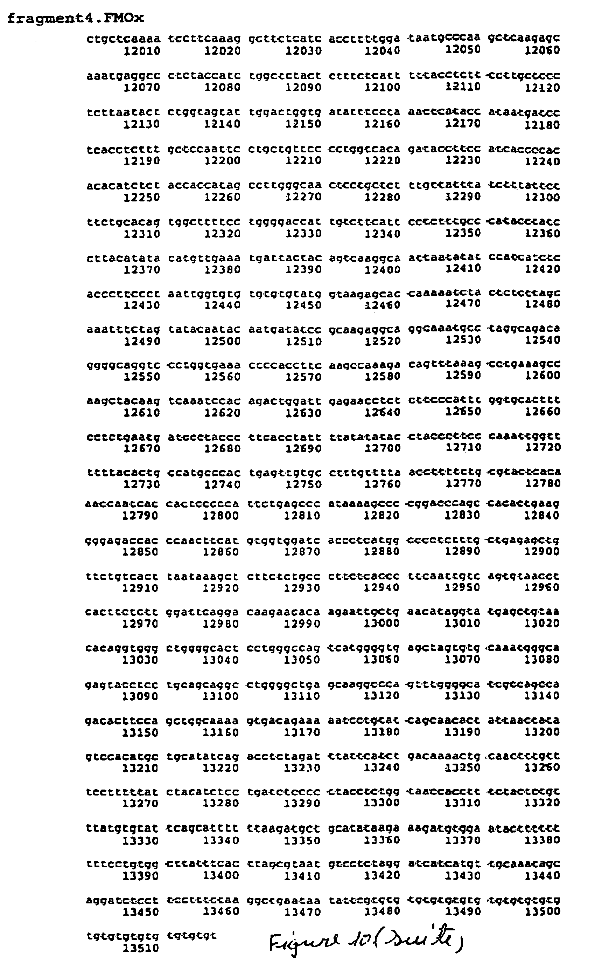

La présente invention décrit également un polynucléotide isolé, dont la séquence SEQ ID N° 4 est partiellement représentée sur la Figure 10, et qui code pour un polypeptide de séquence SEQ ID N° 6.

Ces deux séquences nucléotidiques sont celles de deux gènes codant pour de nouvelles enzymes de la famille des mono-oxygénases à flavine (FMO) humaines, respectivement hFM02 et hFMOx. Ceci a été établi par comparaison des séquences identifiées aux séquences déjà connues de FMO : des homologies structurales très fortes entre les deux séquences étudiées et celles des FMO, des homologies très fortes entre la première séquence et les FMO2 connues, notamment celle de macaque (FM02 de macaque : séquence GeneBank U59453), ainsi qu'une homologie non suffisante de la seconde séquence avec aucune des FMO déjà répertoriées chez l'homme ont permis de conclure.

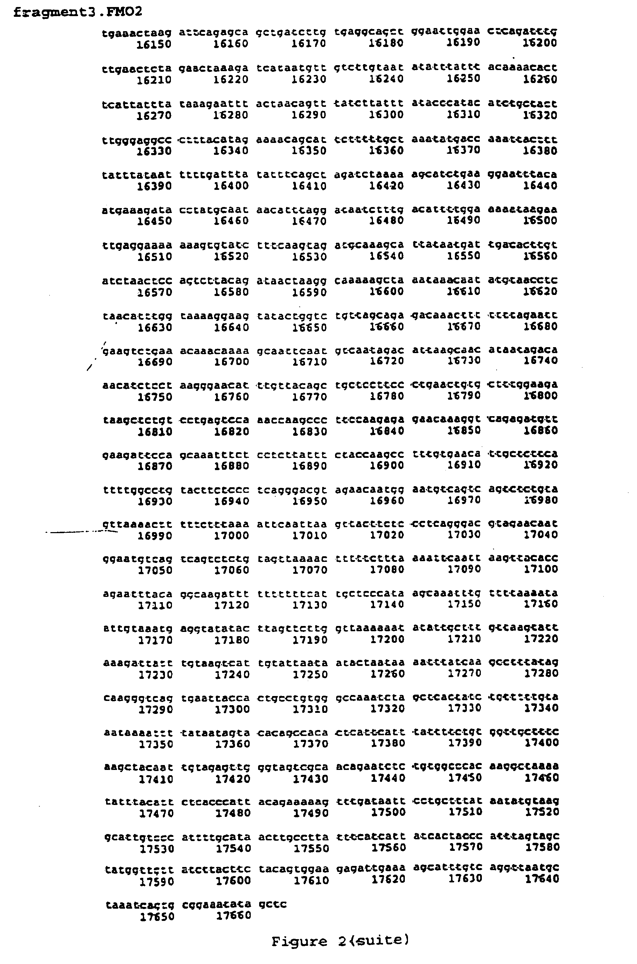

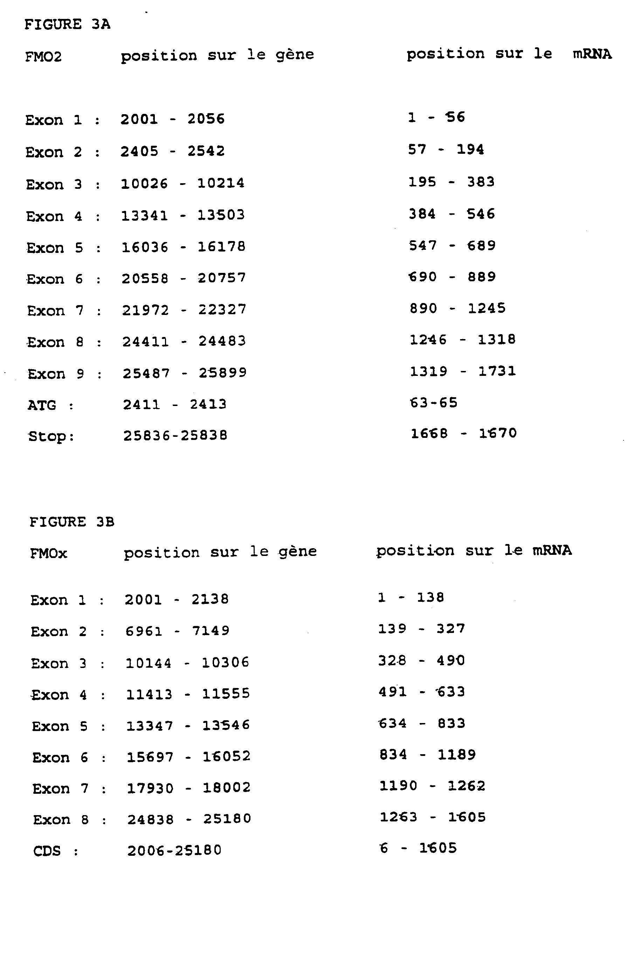

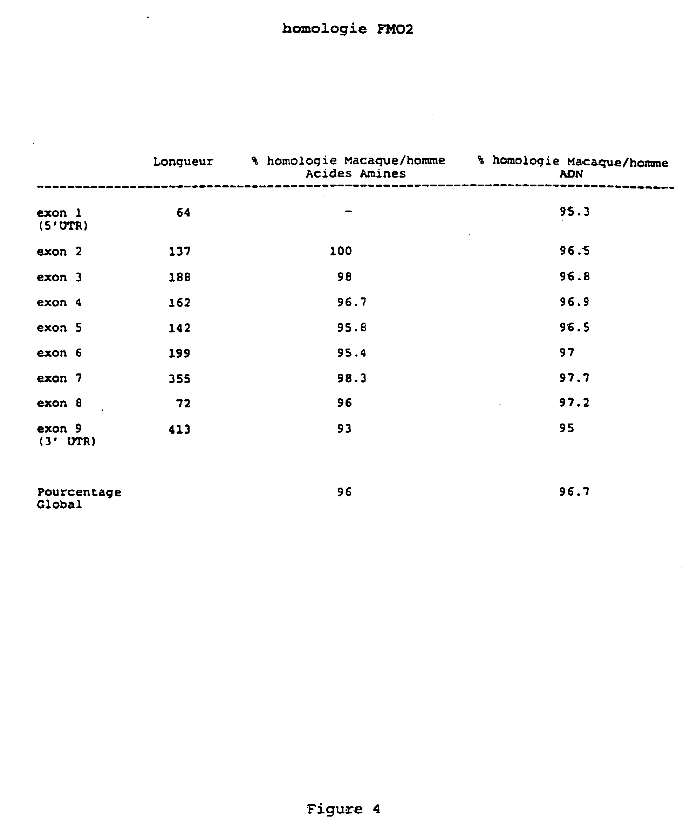

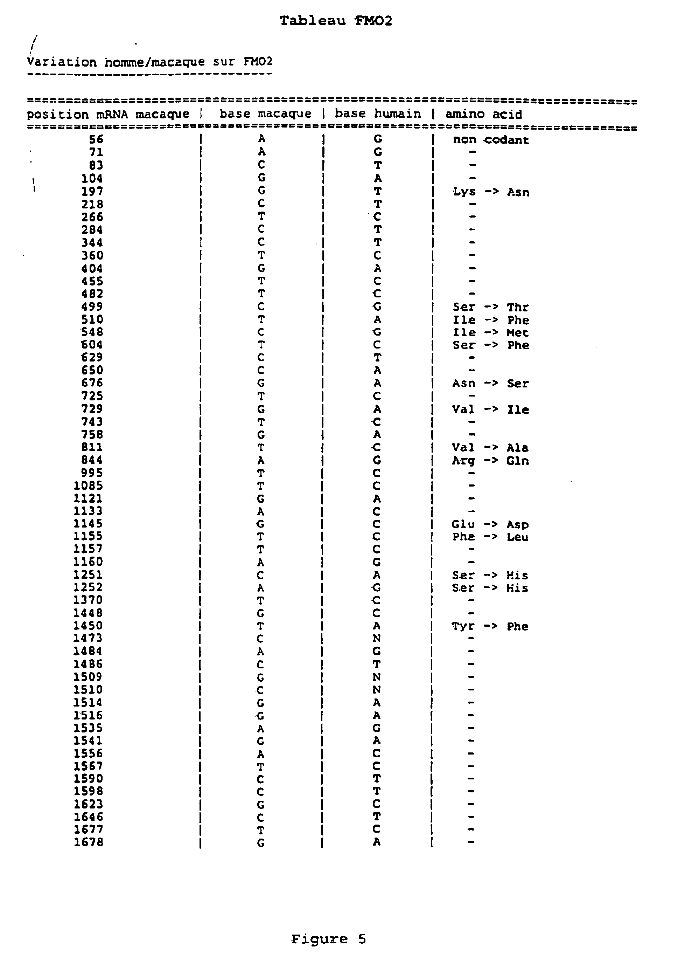

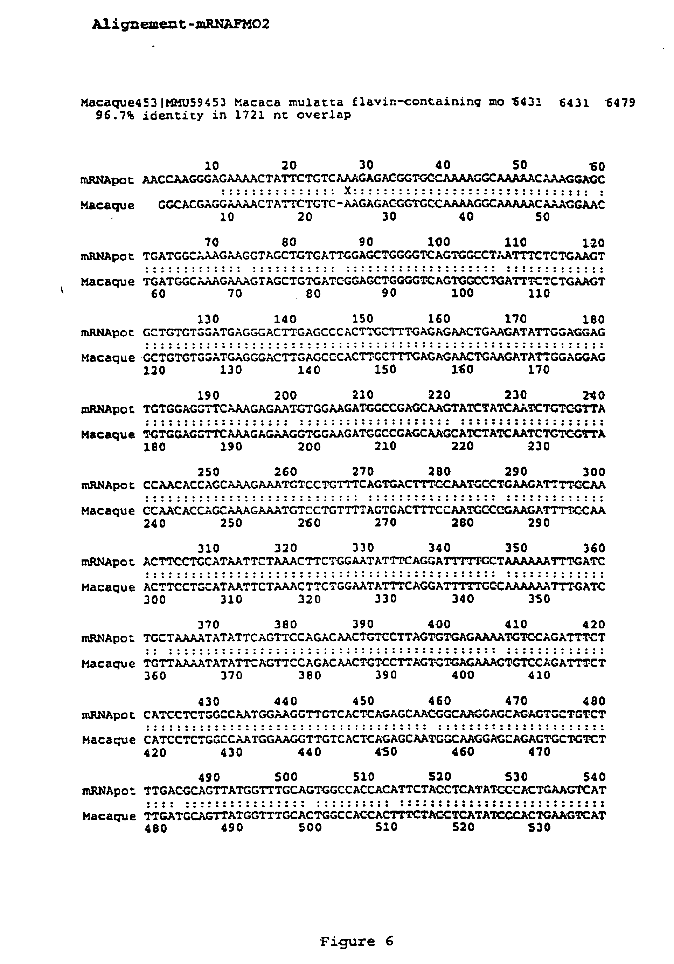

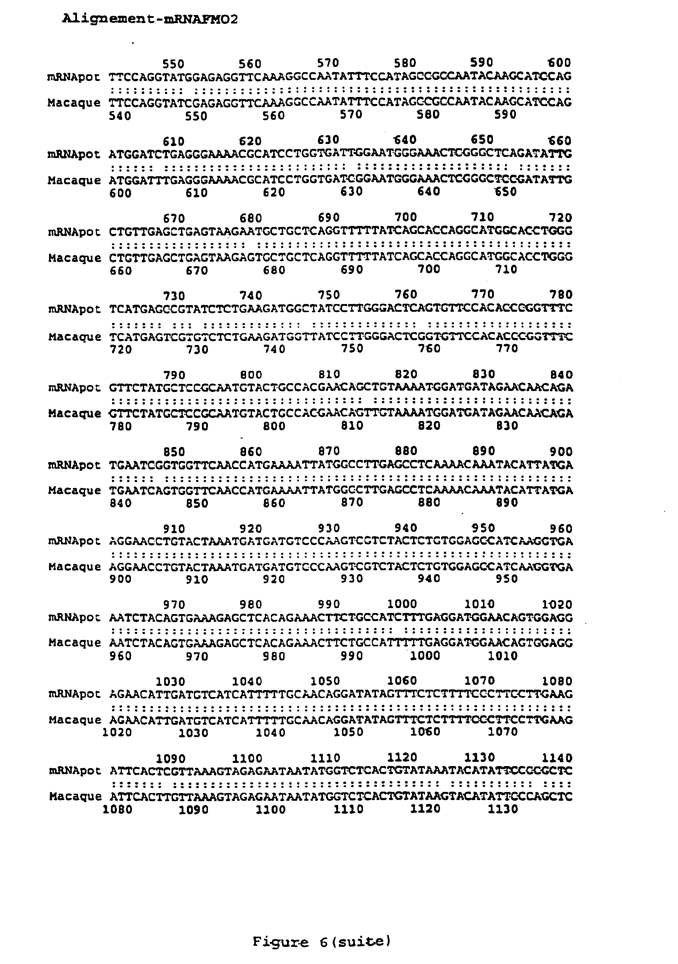

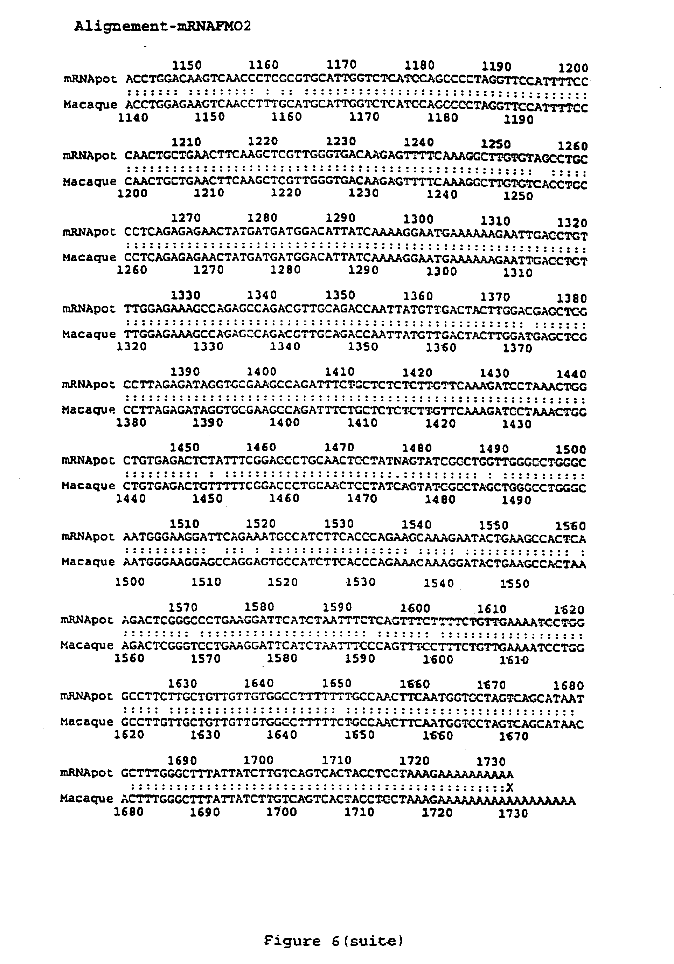

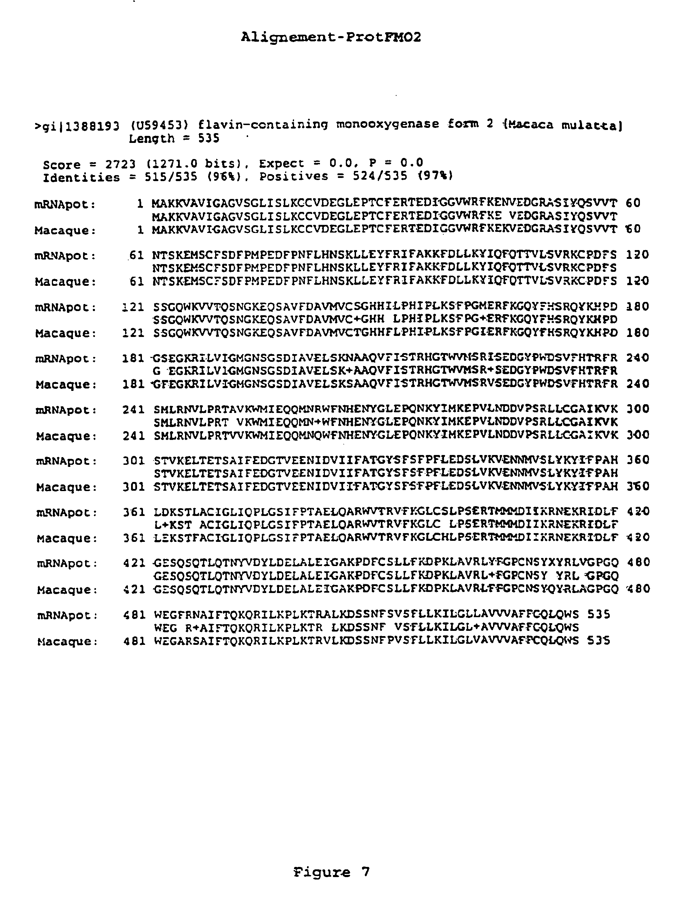

La structure exonique des gènes de la famille FMO déjà connus est entièrement conservée dans la séquence nucléotidique hFMO2 selon l'invention. Les séquences de chacun des 9 exons du polynucléotide selon l'invention (Figure 3) présentent des degrés d'homologie variant de 95 % à 98 % en ADN avec la séquence correspondante de l'ARN messager de la FMO2 de macaque (Figure 4). Les divergences entre les deux séquences nucléotidiques, ainsi que leur signification envers la séquence peptidique, sont présentées sur la Figure 5. La séquence polynucléotidique SEQ ID N° 1 selon l'invention code pour un polypeptide de séquence SEQ ID N° 3 de 535 acides aminés (Figure 1) ; la séquence SEQ ID N° 2 de l'ARN messager prédite, ainsi que la séquence polypeptidique de la protéine humaine sont homologues à 97 % avec celles de la FMO2 de macaque (Figures 6 et 7), ce qui a permis l'identification du polypeptide selon l'invention comme étant la FMO2 humaine. Le polypeptide de séquence SEQ ID N° 3, représenté à la Figure 1, présente également un haut degré d'homologie avec d'autres flavines-mono-oxygénases 2 de mammifères ; ses degrés d'homologie avec d'autres protéines de la famille des flavines-mono-oxygénases sont moins forts.

Comme mentionné précédemment, l'absence d'homologie suffisante entre les séquences correspondant à hFMOx séquences génomique (SEQ ID N° 4), d'ARN messager (SEQ ID N° 5), et peptidique (SEQ ID N° 6) - et les séquences des FMOs connues, a permis de conclure qu'il s'agit d'une nouvelle isoforme de FMO.

La présente invention décrit donc les séquences d'ADN ou d'ARN, l'ADN pouvant être génomique, ADN complémentaire ou synthétique, des FMOs, notamment de hFM02 et hFMOx, ainsi que les protéines correspondantes.

La présente invention décrit en outre des vecteurs de clonage et/ou d'expression contenant lesdites séquences nucléotidiques, des cellules transformées par ces vecteurs ou des animaux contenant lesdites cellules, ainsi que des méthodes de préparation desdits polypeptides sous la forme de polypeptides recombinants.

L'invention décrit aussi des méthodes de sélection de composé capable de moduler l'activité FMO.

L'invention décrit également des méthodes de diagnostic de prédisposition à des troubles liés à FMO ainsi que des compositions pharmaceutiques destinées au traitement et/ou à la prévention de ces troubles.

Un premier exemple de tels troubles pourrait être le glaucome primaire à angle ouvert (GPAO). En effet, d'une part Sunden et al. (1996), ainsi que les inventeurs (Belmouden et al., 1996), ont identifié la région chromosomique GLC1A, qui porte entre autres séquences de gènes celles connues de la famille des FMOs, en 1q23-25, comme liée à la survenue du GPAO juvénile (GPAO-J). D'autre part, un rôle possible des mono-oxygénases dans l'étiologie du glaucome a précédemment été suggéré (Schwartzman et al., 1987). En effet, des métabolites de réactions d'oxydation, en inhibant l'activité Na+, K+ ATPase dans la cornée, contribueraient à la régulation de la transparence de la cornée et de la sécrétion humorale oculaire; or, une opacité de la cornée et une hypertension oculaire sont les deux critères majeurs de diagnostic de glaucome.

Ainsi, un site d'hétérozygotie, présentant une ségrégation d'ordre -génotypique dans une famille étudiée pour la présence en son sein de nombreux membres atteints de GPAO-J, a été identifié par les inventeurs dans l'exon 8 du polypeptide hFM02 selon l'invention.

En poursuivant la recherche des polymorphismes présents dans des populations choisies de façon adéquate, et situés dans des séquences correspondant à celles portées par l'insertion du BAC 123H04M, ou plus généralement par les séquences FMOs, on pourra identifier notamment les mutations associées aux pathologies ou troubles liés à une altération de FMO.

Les différentes isoformes des FMOs semblent moins se distinguer par la spécificité tissulaire de leur expression, que par les substrats dont elles catalysent la transformation. Comme indiqué précédemment, l'expression des FMOs a été mise en évidence dans le foie, les poumons, les reins ou le cerveau.

L'effet pathogène d'un déficit fonctionnel d'une FMO pourrait résulter d'une capacité diminuée des tissus où elle s'exprime à résister au stress oxydatif.

Plus généralement, par leur rôle dans le métabolisme oxydatif et leur fonction de détoxification, les FMOs pourraient être impliquées dans toute pathologie dégénérative ou toxique, démontrée ou à prouver, notamment celles où une mort cellulaire programmée peut être mise en évidence, et les maladies dégénératives du système nerveux central.

De façon générale, les pathologies liées au fonctionnement des FMOs, sont rassemblées sous le nom de « troubles liés à FMO ».

Parmi les troubles liés à FMO, on peut citer par exemple, mais sans s'y limiter:
- oxydation de médicaments, substrats de FMO, en dérivés moins actifs, impliquant une perte d'efficacité dudit médicament ;
- non métabolisation de médicaments actifs sous forme de métabolites, perte d'efficacité dudit médicament ;
- non métabolisation de xénobiotiques toxiques et/ou carcinogènes, dont des substances exogènes présentes naturellement dans l'alimentation, telles que des alcaloïdes végétaux, ou des substances toxiques présentes dans l'environnement, telles que les pesticides ou les herbicides;
- métabolisation de médicaments en intermédiaires pouvant être plus réactifs, impliquant un surdosage avec possibilité d'effet secondaire ;
- métabolisation de xénobiotiques, dont les médicaments ou autres substances exogènes, en intermédiaires pouvant être potentiellement toxiques ; et/ou
- altération de la fonction physiologique dans laquelle est impliquée la FMO ; en particulier, l'altération du fonctionnement de FMO, pourrait être impliquée dans la symptomatologie du glaucome.

Par « FMO », on entendra désigner l'une quelconque des FMOs humaines connues, FMO1, FMO3, FMO4 et FMO5, ou nouvellement décrites dans la présente demande de brevet, à savoir FMO2 ou FMOx.

Certains de ces troubles pourront avoir une origine multigénique mais pour tous, les modifications d'une ou plusieurs FMOs contribuent à la survenue du trouble ou à son aggravation.

### Les séquences nucléotidiques

La présente invention concerne, tout d'abord, une séquence nucléotidique isolée, caractérisée en ce qu'elle code pour le variant de la protéine FMO2 humaine de séquence SEQ ID N° 3 qui présente un résidu lysine à la place d'un résidu acide glutamique en position 402 de la séquence SEQ ID N° 3.

La présente invention concerne également une séquence nucléotidique isolée selon l'invention, caractérisée en ce qu'elle code pour le variant de la protéine FMO2 humaine de séquence SEQ ID N° 3 qui présente un résidu lysine à la place d'un résidu acide glutamique en position 402 de la séquence SEQ ID N° 3 et qu'elle comprend une séquence choisie parmi :
a) la séquence nucléotidique allant du nucléotide en position 2001 au nucléotide en position 2056 de la séquence nucléotidique SEQ ID N° 1,
b) la séquence nucléotidique allant du nucléotide en position 2405 au nucléotide en position 2542 de la séquence nucléotidique SEQ ID N° 1,
c) la séquence nucléotidique allant du nucléotide en position 10026 au nucléotide en position 10214 de la séquence nucléotidique SEQ ID N° 1,
d) la séquence nucléotidique allant du nucléotide en position 13341 au nucléotide en position 13503 de la séquence nucléotidique SEQ ID N° 1,
e) la séquence nucléotidique allant du nucléotide en position 16036 au nucléotide en position 16178 de la séquence nucléotidique SEQ ID N° 1,
f) la séquence nucléotidique allant du nucléotide en position 20558 au nucléotide en position 20757 de la séquence nucléotidique SEQ ID N° 1,
g) la séquence nucléotidique allant du nucléotide en position 21972 au nucléotide en position 22327 de la séquence nucléotidique SEQ ID N° 1,
h) la séquence nucléotidique allant du nucléotide en position 24411 au nucléotide en position 24483 de la séquence nucléotidique SEQ ID N° 1 dans laquelle le nucléotide en position 24431 de la séquence SEQ ID N° 1 est une adénosine à la place d'une guanine,
i) la séquence nucléotidique allant du nucléotide en position 25487 au nucléotide en position 25899 de la séquence nucléotidique SEQ ID N° 1,
j) la séquence nucléique SEQ ID N° 1 dans laquelle le nucléotide en position 24431 est une adénosine à la place d'une guanine, et
k) la séquence nucléique SEQ ID N° 2 dans laquelle le nucléotide en position 1266 est une adénosine à la place d'une guanine.

L'invention comprend en outre une séquence nucléotidique isolée, caractérisée en ce qu'elle est choisie parmi les séquences codant pour un polypeptide comprenant le polypeptide de séquence SEQ ID N° 3 qui présente un résidu lysine à la place d'un résidu acide glutamique en position 402 ou complémentaire d'une séquence selon la présente invention.

Il doit être compris que la présente invention ne concerne pas les séquences nucléotidiques génomiques dans leur environnement chromosomique naturel, c'est-à-dire à l'état naturel, il s'agit de séquences qui ont été isolées, c'est-à-dire qu'elles ont été prélevées directement ou indirectement, par exemple par copie (ADNc), leur environnement ayant été au moins partiellement modifié.

Ainsi, il peut s'agir aussi bien d'ADNc que d'ADN génomique partiellement modifié ou porté par des séquences au moins partiellement différentes des séquences les portant naturellement.

Ces séquences pourront également être qualifiées de « non naturelles ».

Par « séquence nucléique », on entend un fragment d'ADN et/ou d'ARN naturel isolé, ou de synthèse, désignant un enchaînement précis de nucléotides, modifiés ou non, permettant de définir un fragment, un segment ou une région d'un acide nucléique.

Par « allèles », on entend désigner les séquences mutées naturelles correspondant à des polymorphismes pouvant exister chez l'être humain et, notamment, ceux qui peuvent conduire au développement de troubles liés à FMO.

Par « variant protéique », on entend désigner l'ensemble des protéines mutées pouvant exister chez l'être humain, qui correspondent notamment à des troncatures, substitutions, délétions et/ou additions de résidus d'amino-acides, ainsi que les variants artificiels qui seront néanmoins également appelés « variants protéiques ». Dans le cas présent, les variants sont liés en partie à la survenue de troubles liés à FMO.

Selon l'invention, les fragments de séquences nucléiques peuvent notamment coder pour des domaines de la protéine ou bien être utilisés comme sonde ou comme amorce dans des procédés de détection ou d'identification ou d'amplification. Ces fragments présentent une taille minimale de 10 bases et on préférera des fragments de 20 bases, et de préférence 30 bases.

Selon l'invention, l'homologie est uniquement de type statistique, elle signifie que les séquences présentent au minimum 80 %, et préférentiellement 90 %, de nucléotides en commun.

La Figure 1 représente la séquence SEQ ID N° 3, la Figure 2 représente partiellement la séquence SEQ ID N° 1 de FMO2, la Figure 10 représente partiellement la séquence SEQ ID N° 4 de FMOx, telles qu'elles ont été séquencées sur un génome d'un individu ne présentant pas de troubles FMO visibles.

La structure du gène de hFM02 est identifiée dans la Figure 3.

La séquence SEQ ID N° 4 de FMOx est partiellement représentée sur la Figure 10.

L'invention décrit également des fragments de ces séquences, en particulier des séquences codant pour des polypeptides ayant gardé tout ou partie de l'activité de la protéine FMO.

Certaines de ces séquences peuvent être identifiées en se reportant notamment à la Figure 3 qui schématise l'organisation de hFM02.

Ces séquences partielles peuvent être utilisées pour de nombreuses applications, comme cela sera décrit ci-après, notamment pour effectuer des constructions protéiques de type FMO ou de types différents, mais également pour réaliser par exemple des protéines FMO-like.

Si les séquences décrites sont, en général, les séquences normales, l'invention concerne ainsi une séquence mutée comportant une mutation ponctuelle.

La présente invention concerne ainsi une séquence nucléotidique mutée dans laquelle la mutation ponctuelle est non muette, c'est-à-dire qu'elle conduit à une modification de l'amino-acide codé par rapport à la séquence normale. L'invention décrit que certaines mutations peuvent porter sur des amino-acides qui structurent les protéines FMO ou les fragments correspondants de celles-ci, notamment dans les régions correspondant aux sites catalytiques, aux sites régulateurs ou aux sites de fixation des cofacteurs ; les mutations peuvent également porter sur les séquences impliquées dans le transport et l'adressage ; elles peuvent aussi en particulier supprimer les cystéines ou, au contraire, en faire apparaître, mais également changer le caractère de la protéine, soit sur le plan de la charge, soit sur le plan de l'hydrophobicité.

Ces mutations pouvent également intervenir dans les séquences promotrices et/ou régulatrices des gènes FMO humains, lesquelles peuvent avoir des effets sur l'expression de la protéine, notamment sur son taux d'expression.

Parmi les fragments nucléotidiques pouvant être intéressants, notamment pour le diagnostic, il faut citer également les séquences génomiques introniques du gène FMO, par exemple les séquences jonctions entre les introns et les exons.

L'invention comprend ainsi les séquences nucléotidiques selon l'invention, caractérisées en ce qu'elles comprennent au moins la mutation G.1263mac.A, telle qu'elle sera définie ci-après dans les exemples.

L'invention comprend également les séquences nucléotidiques comme amorce nucléique, caractérisées en ce que lesdites séquences sont choisies parmi les séquences SEQ ID N° 7, SEQ ID N° 8, SEQ ID N° 9 et SEQ ID N° 10.

L'invention concerne en outre les séquences nucléotidiques comme sonde spécifique, caractérisées en ce que lesdites séquences sont choisies parmi les séquences SEQ ID N° 11, SEQ ID N° 12, SEQ ID N° 13 et SEQ ID N° 14.

Les polypeptides codés par les séquences nucléotidiques selon l'invention, notamment le polypeptide de séquence SEQ ID N° 3 qui présente un résidu lysine à la place d'un résidu acide glutanique en position 402 font bien entendu partie de l'invention.

Dans la présente description, les termes de protéine, polypeptide ou peptide sont interchangeables.

La présente invention cite également les séquences nucléotidiques qui peuvent comporter des nucléotides non naturels, notamment des nucléotides soufrés ou de structure α ou β.

Il est dit dans la présente invention que les séquences concernent, bien entendu, aussi bien les séquences ADN qu'ARN, ainsi que les séquences qui s'hybrident avec elles, de même que les ADN double brin correspondants.

Parmi les fragments d'acides nucléiques intéressants, il faut citer en particulier les oligonucléotides anti-sens, c'est-à-dire dont la structure assure, par hybridation avec la séquence cible, une inhibition de l'expression du produit correspondant. Il faut également citer les oligonucléotides sens qui, par interaction avec des protéines impliquées dans la régulation de l'expression du produit correspondant, induiront soit une inhibition, soit une activation de cette expression.

Comme cela sera décrit ci-après, pour certaines applications, il peut être nécessaire de prévoir des constructions mixtes, protéine/ADN/composé chimique, notamment l'utilisation d'agents intercalants par exemple

Les protéines, polypeptides ou peptides correspondant aux séquences mentionnées précédemment, sont sous forme non naturelle, c'est-à-dire qu'elles ne sont pas prises dans leur environnement naturel mais qu'elles ont pu être obtenues par purification à partir de sources naturelles ou bien obtenues par recombinaison génétique, comme cela sera décrit ci-après.

L'invention cite également les mêmes polypeptides ou protéines obtenus par synthèse chimique et pouvant comporter des amino-acides non-naturels.

La présente invention cite les protéines recombinantes ainsi obtenues aussi bien sous forme glycosylée que non glycosylée et pouvant présenter ou non la structure tertiaire naturelle.

### Les vecteurs et les cellules

La présente invention concerne également des vecteurs de clonage et/ou d'expression comportant une séquence nucléotidique selon l'invention.

Ces vecteurs de clonage et d'expression pourront comporter des éléments assurant l'expression de la séquence dans une cellule hôte, notamment des séquences promotrices et des séquences de régulation efficaces dans ladite cellule.

Le vecteur en cause pourra être à réplication autonome ou bien destiné à assurer l'intégration de la séquence au sein des chromosomes de la cellule hôte.

Dans le cas de systèmes à réplication autonome, en fonction de la cellule hôte, procaryote ou eucaryote, on utilisera de préférence des systèmes de type plasmidique ou des systèmes viraux, les virus vecteurs pouvant être notamment des adénovirus (Perricaudet et al., 1992), des rétrovirus, des poxvirus ou des virus herpétiques (Epstein et al., 1992). L'homme de métier connaît les technologies utilisables pour chacun de ces virus.

Ainsi, il est connu d'utiliser comme vecteur viral des virus défectifs dont la culture est effectuée dans des cellules de complémentation, ceci évitant les risques éventuels de prolifération d'un vecteur viral infectieux.

Lorsque l'on souhaitera l'intégration de la séquence dans les chromosomes de la cellule hôte, il sera nécessaire de prévoir de part et d'autre de la séquence nucléotidique à intégrer une ou plusieurs séquences provenant de la cellule hôte afin d'assurer la recombinaison. Il s'agit là également de procédés qui sont largement décrits dans la technique antérieure. On pourra, par exemple, utiliser des systèmes de type plasmidique ou viral ; de tels virus seront, par exemple, les rétrovirus (Temin 1986) ou les AAV, Adenovirus Associated Virus (Carter 1993).

L'invention concerne également les cellules procaryotes ou eucaryotes transformées par un vecteur selon l'invention et ceci afin d'assurer l'expression d'une protéine FMO naturelle ou variante ou bien, par exemple, d'un de ses domaines.

Les animaux, caractérisés en ce qu'ils contiennent une cellule transformée selon l'invention, font également partie de l'invention.

L'invention comprend en outre un procédé de production d'un polypeptide selon l'invention, caractérisé en ce qu'on cultive une cellule selon l'invention et en ce que l'on récupère la protéine produite.

Comme cela a été indiqué précédemment, la présente invention concerne également les polypeptides, obtenus par culture des cellules ainsi transformées et récupération du polypeptide exprimé, ladite récupération pouvant être effectuée de façon intracellulaire ou bien de façon extracellulaire dans le milieu de culture lorsque le vecteur a été conçu pour assurer la sécrétion du polypeptide par le biais, par exemple, d'une séquence « leader », la protéine étant exprimée sous forme d'une pré-protéine ou pré-pro-protéine. Les constructions permettant la sécrétion des polypeptides sont connues, aussi bien pour des systèmes procaryotes que pour des systèmes eucaryotes. Certains des polypeptides FMO pourront comporter leur propre système de sécrétion ou d'insertion membranaire.

Parmi les cellules utilisables pour la production de ces polypeptides, il faut citer bien entendu les cellules bactériennes (Olins et Lee, 1993), mais également les cellules de levure (Buckholz, 1993), de même que les cellules animales, en particulier les cultures de cellules de mammifère (Edwards et Aruffo, 1993) mais également les cellules d'insectes dans lesquelles on peut utiliser des procédés mettant en oeuvre des baculovirus par exemple (Luckow, 1993).

Les cellules ainsi obtenues peuvent permettre de préparer des polypeptides naturels ou variants FMO, mais également des fragments de ces polypeptides, notamment des polypeptides pouvant correspondre aux différents domaines en cause.

L'invention comprend également les anticorps mono- ou polyclonaux dirigés contre les polypeptdides selon l'invention, de préférence, caractérisés en ce qu'ils sont obtenus par réaction immunologique d'un organisme humain ou animal avec un agent immunogène constitué par un polypeptide selon l'invention, notamment un polypeptide recombinant ou synthétique selon l'invention.

L'invention concerne également les anticorps selon l'invention, caractérisés en ce qu'il s'agit d'anticorps marqués, notamment pour l'imagerie.

Ces anticorps monoclonaux ou polyclonaux marqués et correspondant notamment à tout ou partie des protéines mutées pourront être utilisés par exemple comme agent d'imagerie, *in vivo* ou *ex vivo* sur des prélèvements biologiques (imagerie à l'aide d'anticorps couplés à une molécule détectable en imagerie de type PET-scan, par exemple).

### Les modèles cellulaires.

Dans le cadre de la présente invention, les cellules transformées ou animaux non humains selon l'invention pourront également être utilisées à titre de modèle pour la selection in vitro de produits impliqués directement ou indirectement dans l'activité de la FMO2 mutée ceci afin d'étudier les différentes interactions mises en cause. Ils pourront être utilisés également pour la sélection in vitro de produits interagissant avec la FMO2 mutée, à titre d'agoniste, notamment d'activateur enzymatique, ou d'antagoniste, notamment d'inhibiteur enzymatique.

Une autre application potentielle de la caractérisation de ces gènes est donc la possibilité d'identifier des composés, notamment protéiques, interagissant avec ces FMOs. Il peut s'agir aussi bien d'inhibiteurs que d'activateurs, de substrats ou de cofacteurs, par exemple. Leur identification permettra de les utiliser en fonction de leurs interactions avec la protéine normale ou la protéine variante. En particulier, on pourra chercher à isoler des agents ayant des effets différents sur les FMOs normales et variantes.

Il est décrit également que l'on pourra utiliser ces modèles cellulaires pour étudier le métabolisme de xénobiotiques, médicaments ou autres, par une FMO, normale ou variante. Ceci pourra être mis en oeuvre dans l'identification du pouvoir toxique de certains composés, dans la sélection et le développement de composés à toxicité réduite, ou à activité accrue, ou dans celui de FMOs modifiées, ayant un meilleur pouvoir de métaboliser les composés d'intérêt.

Ce type de modèle cellulaire peut être réalisé en mettant en oeuvre des techniques de génie génétique. Il s'agit, suivant le type de cellules que l'on désire utiliser, de cloner le gène en question sous sa forme normale ou sous sa forme mutée dans un vecteur d'expression, qu'il s'agisse d'un vecteur à réplication autonome ou d'un vecteur d'intégration, ledit vecteur comportant l'ensemble des éléments permettant l'expression du gène dans la cellule en cause, ou celle-ci ayant l'ensemble des éléments permettant l'expression de la séquence en cause.

On obtient ainsi des cellules eucaryotes ou procaryotes exprimant la ou les protéines FMO, normales ou variantes, qui pourront alors constituer des modèles permettant de tester tout à la fois les interactions de différents produits avec les protéines FMO ou leurs variants, ou de tester des composés, notamment des produits chimiques de synthèse, pouvant interagir avec le produit du gène FMO, normal ou muté, et ce en les ajoutant dans le milieu de culture desdites cellules.

Il faut, en particulier, remarquer que les produits en question pourront aussi bien être des agents à activité antagoniste qu'agoniste.

L'utilisation de modèles cellulaires en vue de tester des composés pharmaceutiques est bien connue, là encore il n'y a pas lieu de détailler ce type de modèle. On peut cependant citer, parmi les techniques utilisées, le « Phage Display» (Allen et al., 1995), et les méthodes de double-hybride (Luban et Goff., 1995).

Ces modèles peuvent être de type in vitro, par exemple des cultures de cellules humaines, soit en culture normale, soit éventuellement sous forme d'organe isolé.

La présente invention décrit également des organismes tels que les animaux, en particulier des souris, exprimant le phénotype correspondant à la FMO normale ou variante d'origine humaine. Là encore, ces animaux pourront être utilisés comme animaux modèles pour tester l'efficacité de certains produits pharmaceutiques.

### Méthode de diagnostic

La présente invention concerne des méthodes de diagnostic de prédisposition à un glaucome juvénile chez un patient, caractérisées en ce qu'on détermine à partir d'un prélèvement biologique dudit patient la présence de la mutation G 24431A dans au moins une séquence codant pour FMO2 par l'analyse de tout ou partie d'une séquence nucléique correspondant audit gène, la présence d'au moins une telle mutation étant indicative d'une prédisposition dudit patient) au glaucome juvénile lié à FMO2.

Il est important de préciser que la présente invention ne décrit en détail que hFMO2 et hFMOx, mais il est indiqué ici que méthodes de diagnostic et les compositions à visées thérapeutiques décrites concernent aussi bien les FMOs précédentes que FMO1, FMO3, FMO4 et FMO5. En effet, les FMOs en général interviennent dans le métabolisme des xénobiotiques et les troubles qui y sont associés, tels que, par exemple, les xénobiotiques et les troubles liés à FMO cités précédemment.

La mutation qui est recherchée pour l'invention est la mutation G.1263mac.A. (localisée sur la Figure 6).

Les séquences d'acides nucléiques analysées pourront être aussi bien de l'ADN génomique, un ADNc ou un ARNm.

Comme cela a été dit précédemment, parmi les troubles liés à FMO qui peuvent être mis en évidence, on entend plus particulièrement les pathologies associées au métabolisme de xénobiotique telles que citées précédemment, ou associées à la fonction biologique de FMO, mais il peut exister d'autres troubles qui pourraient être liés à une anomalie des FMOs.

Les outils de diagnostic basés sur la présente invention, bien qu'ils puissent permettre un diagnostic positif et différentiel chez un patient pris isolément, seront de préférence intéressants pour un diagnostic présymptomatique chez un sujet à risque, notamment avec antécédent familial, et il est possible également de prévoir un diagnostic anté-natal.

En outre, la mise en évidence d'une mutation spécifique peut permettre un diagnostic évolutif, notamment quant à l'intensité du trouble ou à l'époque probable de son apparition.

Les méthodes permettant de mettre en évidence la mutation dans un gène par rapport au gène naturel sont, bien entendu, très nombreuses. On peut essentiellement les diviser en deux grandes catégories, le premier type de méthode est celui dans lequel la présence d'une mutation est détectée par comparaison de la séquence mutée avec la séquence correspondante naturelle non mutée, et le second type dans lequel la présence de la mutation est détectée de façon indirecte, par exemple par la mise en évidence de misappariements dus à la présence de la mutation.

Dans les deux cas, on préférera en général les méthodes dans lesquelles tout ou partie de la séquence correspondant à FMO est amplifiée préalablement à la mise en évidence de la mutation, ces méthodes d'amplification pouvant être réalisées par des méthodes dites PCR ou PCR-like. Par PCR-like on entendra désigner toutes les méthodes mettant en oeuvre des reproductions directes ou indirectes des séquences d'acides nucléiques, ou bien dans lesquelles les systèmes de marquage ont été amplifiés, ces techniques sont bien entendu connues, en général il s'agit de l'amplification de l'ADN par une polymérase ; lorsque l'échantillon d'origine est un ARN il convient préalablement d'effectuer une transcription reverse. Il existe actuellement de très nombreux procédés permettant cette amplification, par exemple les méthodes dites NASBA « Nucleic Acid Séquence Based Amplification » (Compton 1991), TAS « Transcription based Amplification System » (Guatelli et al., 1990), LCR « Ligase Chain Reaction » (Landegren et al., 1988), « Endo Run Amplification » (ERA), « Cycling Probe Reaction » (CPR), et SDA « Strand Displacement Amplification » (Walker et al., 1992), bien connues de l'homme du métier.

Le Tableau 1 présente des séquences d'amorces utilisables pour amplifier les séquences intéressant la mutation G.1263mac.A.

Le réactif utilisé pour détecter et/ou identifier une mutation du gène FMO dans un échantillon biologique comprend une sonde dite de capture et/ou une sonde dite de détection, l'une au moins de ces sondes comportant une séquence selon la présente invention décrite précédemment.

### Recherche de mutations ponctuelles

De façon générale, plusieurs méthodes de détection peuvent être appliquées ou adaptées si nécessaire, après amplification des séquences d'intérêt par PCR. A titre d'exemples, on peut citer:
1) Séquençage: comparaison des séquences de plusieurs individus et/ou repérage d'un site d'hétérozygotie chez un seul individu.
2) « Single nucleotide primer extension » (Syvanen et al., 1990). Des exemples d'amorces utilisables pour détecter la mutation G.1263mac.A par cette méthode figurent dans le Tableau 2.
3) RFLP « Restriction Fragment Length Polymorphism ». Un exemple d'enzyme de restriction utilisable pour détecter la mutation G.1263mac.A. par RFLP est présenté sur le Tableau 3.
4) Recherche de « Single Strand Conformation Polymorphisms » (SSCP).
5) Méthodes basées sur un clivage des régions misappariées (clivage enzymatique par la S1 nucléase, clivage chimique par différents composés tels que la pipéridine ou le tétroxide d'osmium, etc.
6) Mise en évidence d'hétéroduplex en électrophorèse.
7) Méthodes basées sur l'utilisation en hybridation de sondes oligonucléotidiques spécifiques d'allèles:
   « Allele Specific Oligonucleotide » (ASO) (Stoneking et al., 1991). Des exemples de sondes utilisables pour la détection de la mutation G.1263mac.A. par ASO figurent sur le Tableau 4.
8) Méthode OLA « dual color Oligonucleotide Ligation Assay » (Samiotaki et al., 1994).
9) Méthode ARMS « Amplification Refractory Mutation System », ou ASA « Allele Specific Amplification », ou PASA « PCR Amplification of Specific Allele » (Wu et al., 1989).

Cette liste n'est pas exhaustive, et d'autres méthodes bien connues peuvent être utilisées.

### Recherche de remaniements , par exemple de type délétions

D'autres méthodes bien connues et basées sur les techniques d'hybridation à l'aide de sondes génomiques, de sondes ADNc, de sondes oligonucléotidiques ou de ribosondes peuvent être utilisées pour la recherche de ce type de remaniements.

Font donc, ainsi, également partie de l'invention, les méthodes de diagnostic d'une prédisposition à un glaucome juvénile lié à FMO2 mutée chez un patient selon l'invention, caractérisées en ce que ladite analyse est réalisée par hybridation, ladite hybridation étant réalisée de préférence à l'aide d'au moins une sonde oligonucléotidique spécifique de l'allèle, ou en ce que la présence d'une mutation est détectée par comparaison avec la séquence correspondante naturelle non mutée, ou en ce que ladite analyse est réalisée par séquençage, ou par migration électrophorétique, et plus particulièrement par SSCP ou DGGE, ou en ce que ladite analyse est réalisée par une méthodologie visant à détecter une troncation de la protéine.

Font aussi partie de l'invention, les méthodes de diagnostic d'une prédisposition à un glaucome juvénile lié à FMO2 mutée chez un patient selon l'invention, caractérisées en ce que tout ou partie de la séquence nucléique du gène FMO est amplifiée préalablement à la mise en évidence de la ou des mutations, de préférence l'amplification est réalisée par PCR ou PCR-like, les amorces choisies pour réaliser l'amplification étant de préférence choisies parmi les amorces selon l'invention.

Les réactifs pour détecter et/ou identifier la mutation du gène) FMO2 humain un échantillon biologique, caractérisés en ce qu'il comprennent une sonde dite de capture et/ou une sonde dite de détection, l'une au moins de ces sondes comportant une séquence selon l'invention, ou un anticorps selon l'invention, font également partie de l'invention.

### Méthodes basées sur la détection du produit du gène

La mutation du gène FMO2 humain est responsables d'une modification du produit de ce gène, modification utilisable pour une approche diagnostique. En effet, les modifications d'antigénicité peuvent permettre la mise au point d'anticorps spécifiques. Toutes ces modifications peuvent être utilisées en approche diagnostique, grâce à plusieurs méthodes bien connues basées sur l'utilisation d'anticorps mono- ou polyclonaux reconnaissant la protéine normale ou des variants mutés, par exemple méthode RIA ou ELISA.

Enfin, il est décrit ici qu'il est également possible de diagnostiquer une prédisposition à des troubles liés à FMO, chez un patient, en mesurant l'activité enzymatique de la (ou des) FMO à partir d'échantillons biologiques dudit patient. La mesure de cette (de ces) activité(s), par comparaison avec un étalon, interne ou externe, sera en effet indicative d'une prédisposition à l'un des troubles précédemment cités.

### Compositions thérapeutiques

La présente invention décrit également les traitements thérapeutiques, curatifs ou préventifs, de troubles liés à FMO.

On pourra utiliser les composés impliqués directement ou indirectement dans l'activité FMO, issus de l'utilisation des modèles cellulaires décrits précédemment.

On pourra particulièrement utiliser les composés capables d'interagir avec les FMOs, normales ou variantes, à titre d'agoniste ou d'antagoniste notamment.

La présente invention décrit également des compositions thérapeutiques comportant à titre de principe actif un composé capable de moduler l'activité FMO, il peut s'agir de composés à activité pro-FMO, notamment tels que décrits précédemment, ou des composés à activité anti-FMO.

De façon générale, on entendra par composé à « activité pro-FMO » un composé qui induira l'activité FMO, au contraire un composé anti-FMO aura tendance à réduire l'activité FMO. L'effet réel de ces types d'activités dépendra du type d'enzyme exprimée, normale ou pathologique.

De façon préférée, on pourra utiliser des compositions thérapeutiques dont l'activité sera différente envers les enzymes FMOs normales et variantes.

Il est tout d'abord possible de prévoir un traitement de substitution, c'est-à-dire des compositions thérapeutiques caractérisées en ce qu'elles comportent à titre de principe actif un composé à activité pro-FMO ; il pourra s'agir notamment de tout ou partie de polypeptides tels qu'ils ont été décrits précédemment, ou bien d'un vecteur d'expression de ces mêmes polypeptides, ou bien encore de composés chimiques ou biologiques ayant une activité pro-FMO, une activité FMO-like ou induisant la production de FMO.

Il est possible également d'utiliser des compositions thérapeutiques dans lesquelles le principe actif aura une action anti-FMO, en particulier anti-FMO variante. Dans ce cas il s'agit d'un traitement suppressif. Il pourra s'agir, par exemple, de composés interagissant avec lesdites enzymes, notamment des composés protéiques, et en particulier d'anticorps anti-FMO, notamment lorsque ces anticorps reconnaîtront les protéines variantes. Il pourra s'agir également de produits chimiques ayant une activité anti-FMO, notamment des antagonistes de FMO variante.

Parmi les nombreux composés pharmaceutiques utilisables, il faut citer plus particulièrement, les séquences anti-sens interagissant avec le gène FMO normal ou muté, ou bien les séquences sens agissant sur la régulation de l'expression de ces gènes, lesdits produits pouvant également interagir en aval des produits d'expression induits par les FMOs.

Il faut également mentionner les anticorps monoclonaux inhibant les FMOs, en particulier les FMOs mutées, et/ou inhibant les ligands correspondants et/ou les produits induits par l'activité FMO, qui peuvent donc avoir des activités pro ou anti.

Il est également possible de prévoir l'expression de protéines ou leurs fragments in vivo, notamment par le biais de la thérapie génique et en utilisant les vecteurs qui ont été décrits précédemment.

Dans le cadre de la thérapie génique, il est possible également de prévoir l'utilisation des séquences des gènes ou des ADNc précédemment décrits, « nus », cette technique a notamment été développée par la société Vical, qui a montré qu'il était, dans ces conditions, possible d'exprimer la protéine dans certains tissus sans avoir recours au support d'un vecteur viral notamment.

Toujours dans le cadre de la thérapie génique, il est également possible de prévoir l'utilisation de cellules transformées ex-vivo, lesquelles pourront être ensuite réimplantées, soit telles quelles, soit au sein de systèmes de type organoïde, tel que cela est également connu dans l'état de la technique (Danos et al., 1993). On peut également envisager l'utilisation d'agents facilitant le ciblage d'un type cellulaire déterminé, la pénétration dans les cellules ou le transport vers le noyau.

Ainsi, l'invention décrit également une composition thérapeutique, caractérisée en ce qu'elle comporte à titre de principe actif au moins un composé capable de moduler l'activité FMO, de préférence l'activité FMO2 et/ou FMOx.

L'invention décrit également une composition thérapeutique caractérisée en ce qu'elle comporte à titre de principe actif au moins un composé capable d'interagir avec FMO, de préférence capable d'interagir avec FMO2 et/ou FMOx, ou une composition thérapeutique caractérisée en ce qu'elle présente une activité différente sur FMO normale et FMO pathologique.

La présente invention comprend une composition thérapeutique caractérisée en ce qu'elle comporte à titre de principe actif au moins un agoniste de la FMO2 humaine mutée choisi parmi les composés suivants :
a) une protéine ou un polypeptide selon l'invention,
b) un vecteur d'expression selon l'invention,
c) une séquence nucléotidique selon l'invention,
caractérisée en ce que ladite séquence est une séquence sens induisant l'expression de ladite FMO2 mutée.

L'invention concerne en outre une composition thérapeutique caractérisée en ce qu'elle comporte à titre de principe actif un antagoniste de la FMO2 humaine mutée choisi parmi les composés suivants :
a) un anticorps anti-FMO selon l'invention,
b) une séquence nucléotidique selon l'invention, caractérisée en ce que ladite séquence est une séquence antisens inhibant l'expression de ladite FMO2 mutée,
c) une séquence nucléotidique selon l'invention,
caractérisée en ce que ladite séquence est une séquence sens inhibant l'expression de ladite FMO2 mutée.

L'invention décrit aussi une composition thérapeutique selon l'invention, caractérisée en ce que le principe actif est une séquence soluble interagissant avec FMO.

L'invention a également pour objet l'utilisation d'un principe actif capable de moduler l'activité de la FMO2 humaine mutée pour réaliser un médicament destiné au traitement et/ou à la prévention du glaucome juvénile, caractérisée en ce que ledit principe actif est choisi parmi les composés agonistes as antagonistes des compositions thérapeutiques de l'invention.

Sous un autre aspect, l'invention décrit également un procédé de biodégradation ou de biosynthèse de composé organique ou inorganique, caractérisé en ce qu'il met en oeuvre un polypeptide ou une cellule selon l'invention.

Les polypeptides à activité FMO pourront en effet avantageusement être utilisés pour biodégrader suivant les réactions d'oxydation, telles que décrites par exemple par Ziegler (Ziegler et al., 1993), les composés substrats de FMO, en particulier les composés tels que cités dans la présente description, ou être utilisés pour la biosynthèse de composé d'intérêt à partir desdits composés substrats de FMO, notamment pour la biosynthèse de médicament, d'additif alimentaire, de pesticide ou d'herbicide.

Les procédés d'élaboration de composé d'intérêt, caractérisés en ce qu'ils utilisent, un polypeptide ou une cellule selon l'invention, sont cités ici.

Les polypeptides ou cellules selon l'invention, pourront en effet avantageusement être utilisés in vitro pour déterminer la métabolisation potentielle du composé d'intérêt et pour analyser les métabolites éventuellement obtenus, leur toxicité et/ou leur activité. Les résultats obtenus permettront de confirmer le composé ou de le reformuler de manière à ce qu'il devienne ou pas substrat de FMO, ou à ce que les métabolites formés soient différents.

Enfin, l'invention décrit ici l'utilisation de polypeptide ou de cellule selon l'invention, pour la détoxification de composé xénobiotique, substrat de FMO. Ces composés xénobiotiques peuvent être présents dans l'environnement, tels que pesticide ou herbicide, présents naturellement dans les plantes comme certains alcaloïdes, ou peuvent correspondre à des composés pharmaceutiques.

D'autres caractéristiques et avantages de la présente invention apparaîtront à la lecture des exemples ci-après, faite en se référant aux dessins annexés suivants:
- Figure 1: Séquence polypeptidique correspondant à la séquence SEQ ID N° 3 prédite de hFMO2, homologue humaine de la FMO2 de macaque.
- Figure 2 : Séquence nucléotidique correspondant partiellement à la séquence SEQ ID N° 1 du gène codant pour hFM02, homologue humaine de la FMO2 de macaque.
   Au vu des homologies des ARN messagers connus de gènes de la famille des mono-oxygénases à flavine, ces gènes partagent la même structure exon/intron:
   exon1 : non traduit, variable en taille et en séquence,
   exon2 : début de la région codante, code pour les acides aminés 1-44,
   exon3 : acides aminés 45-107,
   exon4 : acides aminés 108-161,
   exon5 acides aminés 162-209,
   exon6 acides aminés 210-275,
   exon7 : acides aminés 276-394,
   exon8 :acides aminés 395-419,
   exon9 : acides aminés 420-535, fin du codant et région non traduite 3'.

   Les introns sont variables en taille et en complexité. Nous avons d'abord isolé la séquence de trois fragments du BAC 123H04M qui contiennent la totalité des exons de cet homologue.
   Fragment 1 : contenant les exons 1 et 2,
   Fragment 2 : contenant l'exon 3,
   Fragment 3 : contenant les exons 4 à 9.

   Les séquences de deux introns ont ensuite été complétées.
- Figure 3 :
   Figure 3A : Description de la structure exon/intron du gène codant pour hFMO2, homologue humaine de la FMO2 de macaque.
      Sont indiquées les positions des débuts et fins d'exons sur les séquences nucléotidiques SEQ ID N° 1 et N° 2.
   Figure 3B: Description de la structure exon/intron du gène codant pour hFMOx.
      Sont indiquées les positions des débuts et fins d'exons sur les séquences nucléotidiques SEQ ID N° 4 et N° 5.
- Figure 4 : Homologie entre le gène FMO2 de macaque et son homologue humain tel que présenté en figure 2.
   La région 5' non traduite diverge légèrement de la séquence de macaque tel que présenté en figure 2.
- Figure 5 : Relevé des positions variantes de la séquence de l'ARNm de hFM02 humain par rapport à la séquence homologue de macaque ; influence des variations sur la séquence protéique.
- Figure 6: Homologies entre les séquences d'ARMm de la FMO2 de macaque et de son homologue humaine.
   La position de la mutation G.1263mac.A est repérée par une flèche verticale.
- Figure 7 : Homologies entre les séquences peptidiques de la FMO2 de macaque et de son homologue humaine.

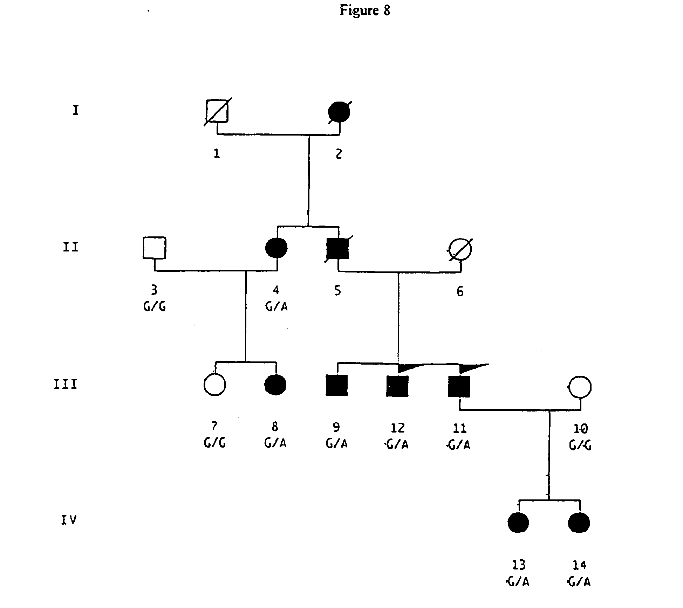

- Figure 8 : Analyse de la ségrégation du polymorphisme G.1263mac.A dans la famille étudiée.
   L'ADN génomique des individus 3, 4, et 7 à 14 a été amplifié par PCR, et la séquence des fragments obtenus analysée pour détecter des sites d'hétérozygotie ségrégeant avec la maladie.
   Les symboles pleins indiquent les individus atteints du GPAO juvénile. Les symboles barrés indiquent des individus non génotypés. Les individus 11 et 12 sont des jumeaux.
   G/G = homozygotes pour la base en position homologue de la position 1263 de l'ARNm de la FMO2 de macaque.
   G/A = hétérozygotes pour la base en position homologue de la position 1263 de l'ARNm de la FMO2 de macaque.

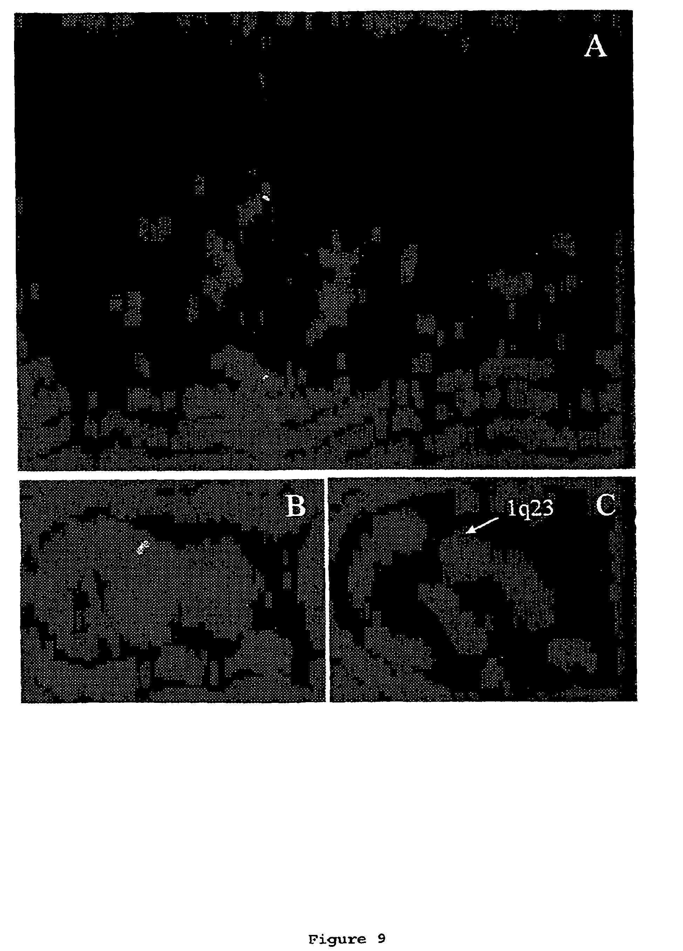

- Figure 9: Localisation chromosomique du BAC123H04M par hybridation in situ fluorescente.
   (A) Un signal spécifique est observé sur les deux chromosomes 1. Sur la photo (B) est représenté un seul des deux chromosomes 1. En (C), seules les bandes R de ce chromosomes sont observées, montrant que le signal de la sonde 123H04M est localisé sur la bande 1q23.
- Figure 10 : Séquence nucléotidique correspondant partiellement à la séquence SEQ ID N° 4 du gène codant pour l'isoforme hFMOx humaine.
   Ce gène présente un taux d'homologie de 75 % en ADN et de 70 % en acides aminés avec les ARNs messagers de mono-oxygénases à flavine présents sur le BAC 123H04M. Il est présenté, dans cette figure préliminaire, en quatre fragments :
   fragment 1: code pour l'exon 2 (premier exon codant),
   fragment 2 : exon 3,
   fragment 3 : exons 4 à 8,
   fragment 4 : exon 9.

### EXEMPLES

### Isolement du BAC 123H04M

Afin d'identifier un gène codant pour une nouvelle FMO, on a isolé un BAC (« Bacterial Artificial Chromosome ») correspondant à la région candidate précédemment localisée sur le chromosome 1. Une banque de BACs couvrant le génome humain complet a été préparée à partir de l'ADN d'une lignée lymphoblastique humaine dérivée de l'individu n° 8445 des familles du CEPH. Cette lignée a été utilisée comme source d'ADN de haut poids moléculaire. L'ADN a été partiellement digéré par l'enzyme de restriction BamH1, puis cloné au site BamH1 du plasmide pBeloBacII. Les clones ainsi obtenus ont été « poolés » et criblés selon une procédure d'analyse tridimensionnelle précédemment décrite pour le criblage des banques de YACs (« Yeast Artificial Chromosome » ) (Chumakov et al., 1992). Les pools tridimensionnels obtenus ont été criblés par PCR à l'aide des amorces encadrant le marqueur D1S3423 (WI-10286). Ce STS (« Sequence Tagged Site ») avait été précédemment localisé dans la région candidate. Un clone du BAC 123H04M a été ainsi isolé.

Après digestion par l'enzyme de restriction NotI, la taille de l'insert porté par ce BAC a été déterminée sur un gel d'agarose 0,8 % après migration par électrophorèse en champ alterné (CHEF) (4 heures à 9 Volts/cm, avec un angle de 100°, à 11°C en tampon 0,5xTAE). On a ainsi mis en évidence que le BAC 123H04M porte un insert de 180 kb.

### Localisation chromosomique du BAC 123H04M par hybridation in situ fluorescente (FISH)

La localisation chromosomique du BAC dans la région candidate 1q23-q25 a été confirmée par hybridation in situ fluorescente (FISH) sur chromosomes métaphasiques, selon la méthode décrite par Cherif et al., 1990. Le BAC 123H04M a été localisé plus précisément dans la bande 1q23 du chromosome 1 (Figure 9).

### Séquençage de l'insert du BAC 123H04M

Afin de séquencer l'insert du BAC 123H04M, on a préparé trois banques distinctes de sous clones à partir de l'ADN soniqué de ce BAC.

Après incubation une nuit, les cellules issues de trois litres de culture ont été traitées par lyse alcaline selon les techniques classiques. Après centrifugation du produit obtenu dans un gradient de chlorure de césium, 52 µg d'ADN du BAC 123H04M ont été purifiés . 7 µg d'ADN ont été soniqués dans trois conditions différentes, afin d'obtenir des fragments dont les tailles se distribuent uniformément de 1 à 9kb. Les fragments obtenus ont été traités dans un volume de 50 µl avec 2 unités de Vent polymérase pendant 20 minutes à 70°C, en présence des 4 déoxytriphosphates (100 µM). Les fragments aux extrémités franches résultant de cette étape ont été séparés par électrophorèse en gel 1 % d'agarose à bas point de fusion (60 Volts pendant 3 heures). Les fragments groupés selon leurs tailles ont été excisés et les bandes obtenues traitées par l'agarase. Après extraction au chloroforme et dialyse sur colonnes Microcon 100, l'ADN en solution a été ajusté à une concentration de 100 ng/µl. Une ligation a été effectuée, incubation une nuit, en mettant en présence 100 ng de l'ADN fragmenté du BAC 123HO4M et 20 ng d'ADN du vecteur linéarisé par digestion enzymatique, et traité par la phosphatase alcaline. Cette réaction é été réalisée dans un volume final de 10 *µ*l en présence de 40 unités/*µ*l de T4 ADN ligase (Epicentre). Les produits de ligation ont ensuite servi à transformer par électroporation, soit une souche XL-Blue (pour les plasmides multicopies), soit une souche D10HB (pour les sous clones issus du BAC). Les clones lacZ⁻ et résistants à l'antibiotique, ont été repiqués individuellement .en microplaques pour stockage et séquençage.

On a ainsi obtenu :
- 864 sous clones issus de l'insertion de fragments .de 2 à 3 kb au site SmaI du plasmide puc18;
- 1728 sous clones correspondant à l'insertion de fragments de 1, 5 à 2 kb au site BamHI (rendu franc) du plasmide BluescriptSK;
- 288 sous clones portant des fragments de 4 à 7 kb insérés au site PmlI d'un vecteur BAC modifié.

Les inserts de ces sous clones ont été amplifiés par PCR sur cultures bactériennes incubées une nuit en utilisant les amorces des vecteurs flanquant les insertions. La séquence des extrémités de ces inserts (en moyenne 500 bases de chaque côté) a été déterminée par séquençage automatique fluorescent sur séquenceur ABI 377, équipé du logiciel ABI Prism DNA Sequencing Analysis (version 2.1.2).

Les fragments de séquence provenant des sous-BACs ont été assemblés par le logiciel Gap4 de R. Staden (Bonfield et al., 1995). Ce logiciel permet la reconstruction d'une séquence complète à partir de fragments de séquences. La séquence déduite de l'alignement des différents fragments est la séquence consensus.

On a enfin utilisé des techniques de séquencage dirigé (marche systématique de l'amorce) pour parfaire les séquences et relier les contigs.

### Analyse des séquences

Les exons potentiels du BAC 123H04M ont été repérés par recherche d'homologie sur les banques publiques de protéines, d'acides nucléiques et d'EST (Expressed Sequence Tags).

### Banques de données:

On a utilisé des refontes locales des principales banques publiques. La banque de protéines utilisée est constituée par la fusion non redondante des banques Genpept (traduction automatique de GenBank, NCBI ; Benson et al., 1996); Swissprot, (George et al., 1996); et PIR/NBRF (Bairoch et al., 1996). Les doublons ont été éliminés par le logiciel "nrdb" (domaine public, NCBI ; Benson et al., 1996). Les répétitions internes ont ensuite été masquées par le logiciel « xnu » (domaine public, NCBI ; Benson et al., 1996). La banque résultante, dénommée NRPU (Non-Redundant Protein- Unique) a servi de référence pour les recherches d'homologies protéiques. Les homologies trouvées avec cette banque ont permis de localiser des régions codant potentiellement pour un fragment de protéine au moins apparenté à une protéine connue (exons codants). La banque d'EST utilisée est composée des sous-sections « gbest » (1-9) de Genbank (NCBI ; Benson et al., 1996). Elle contient tous les fragments de transcrits publics.

Les homologies trouvées avec cette banque ont permis de localiser des régions potentiellement transcrites (présentes sur l'ARN messager).

La banque d'acides nucléiques (autres que les EST) utilisée contient toutes autres sous-sections de Genbank et de l'EMBL (Rodriguez-Tome et al., 1996) dont les doublons ont été éliminés comme précédemment.

### Logiciels :

On a utilisé l'ensemble de logiciel BLAST (domaine public, Altschul et al., 1990) de recherche d'homologies entre une séquence et des banques de données protéiques ou nucléiques. Les seuils de signification dépendent de la longueur et de la complexité de la région testée ainsi que de la taille de la banque de référence. Ils ont été ajustés et adaptés à chaque analyse.

### Identification de polymorphismes génétiques associés au FMO en relation avec un polymorphisme phénotypique associé à la survenue du glaucome juvénile GFAO-J, maladie de transmission autosomale dominante (locus GLC1A)

### Détection de Polymorphismes/Mutations

### 1) Extraction de l'ADN

L'ADN est extrait du sang veineux périphérique après lyse cellulaire, digestion protéique, partition organique et finalement précipitation alcoolique.

Le sang (20 ml) est prélevé par ponction veineuse périphérique sur un tube contenant de l'EDTA.

Il est dilué avec un volume d'eau bidistillée. Après 10 minutes, les cellules sont collectées par centrifugation à 1600 g pendant 10 minutes. Cette manipulation est répétée.

Les cellules blanches sont lysées en présence de 20 ml de tampon CLB (Tris 10 mM pH 7.6, 5 mM MgCl₂, sucrose 0.32 M, Triton X-100 1 % (v/v). Les noyaux sont collectés par centrifugation à 1600 g pendant 10 minutes. Cette manipulation est répétée.

Les noyaux sont lavés une fois dans le tampon RSB (Tris 10 mM pH 8, NaCl 10 mM, EDTA 10 mM). Le culot est resuspendu dans 2 ml de tampon RSB auquel est ajouté du lauryl sulfate de sodium (1 %) et la protéinase K (200 mg/ml). Le mélange est incubé à 55°C pendant au moins 3 heures et régulièrement agité.

La solution d'ADN ainsi obtenue est ensuite extraite avec un volume de phénol équilibré avec un tampon 50 mM Tris pH 8. Cette opération est répétée et complétée par une extraction avec un volume de chloroforme /alcool isoamylique (24: 1 v/v).

L'ADN est précipité avec un volume d' isopropanol, rincé à l'ethanol (70 %), séché et enfin resuspendu dans 1 ml de tampon TE (Tris 10 mM pH 8, EDTA 0.5 mM). La concentration d'ADN est évaluée par mesure de l'absorbance à 260 nm en utilisant l'équivalence de 50 µg/ml d'ADN pour une unité d'absorbance. La concentration d'ADN est alors ajustée à 200 µg/ml.

### 2) Amplification de l'ADN génomique

Les amorces oligonucléotidiques utilisées pour l'amplification génomique des séquences exoniques dérivées du BAC 123H04M, telles que prédites par analyse informatique, ont été définies à l'aide du logiciel OSP (Hillier et al., 1991).

Toutes ces amorces contiennent, en amont des bases spécifiquement ciblées par l'amplification, une queue oligonucléotidique commune, destinée à permettre le séquençage des fragments amplifiés (PU pour les amorces en amont, et RP pour les amorces en aval ; séquences exposées sur le Tableau 5).

Les amorces oligonucléotidiques ont été synthétisées selon la méthode des phosphoramidites, sur un synthétiseur GENSET UFPS 24.1.

L'amplification de chaque séquence exonique prédite a été réalisée par réaction d'amplification en chaîne par polymérase (PCR), dans les conditions suivantes :

| | |
|---|---|
| Volume final | 50 µl |
| ADN génomique | 100 ng |
| MgCl2 | mM |
| dNTP (pour chacun) | 200 *µ*M |
| Amorce (pour chacune) | 7.5 pmoles |
| AmpliTaq Gold DNA polymerase (Perkin) | 1 unité |
| *Tampon de PCR | 1 X |

| | |
|---|---|
| * :(10X=0.1 M Tris HCl pH 8.3, 0.5 M KCl) | |

L'amplification est réalisée dans un thermocycleur Perkin Elmer 9600 ou MJ Research PTC200 avec couvercle chauffant. Après un chauffage à 94°C pendant 10 minutes, 35 cycles sont effectués. Chaque cycle comprend: 30 secondes à 94°C, 1 minute à 55°C et 30 secondes à 72°C. Un segment final d'élongation de 7 minutes à 72°C termine l'amplification.

La quantité de produits d'amplification obtenue est déterminée sur micro-plaque de 96 puits, par fluorométrie, utilisant l'agent intercalant Picogreen (Molecular Probes).

### 3) Détection des polymorphismes/mutations

- Séquence
   Les produits de l'amplification génomique par PCR ont été séquencés sur séquenceur automatique ABI 377, en utilisant des amorces fluorescentes marquées par les fluorochromes ABI (Joe, Fam, Rox et Tamra) et l'ADN polymérase Thermosequanase (Amersham).
   Les réactions ont été réalisées en microplaques de 96 puits, sur thermocycleur Perkin Elmer 9600, dans des conditions classiques de cycles de température :
   - 8 cycles: dénaturation: 5 sec. à 94°C; hybridation : 10 sec. ; élongation : 30 sec. à 72°C, puis
   - 13 cycles : dénaturation : 5 sec. à 94°C; élongation : 30 sec. à 72°C.

   6 unités de Thermosequanase, et 5-25 ng de produit d'amplification ont été utilisées par réaction de séquence.
   A l'issue des cycles d'amplification, les produits des réactions de séquence sont précipités dans l'éthanol, resuspendus dans du tampon de charge contenant de la formamide, dénaturés, et déposés sur gels d'acrylamide 4 %; les électrophorèses (2 heures 30 à 3000 Volts) sont conduites sur séquenceurs ABI 377 équipés des logiciels ABI de collection et d'analyse (ABI Prism DNA Sequencing Analysis Software, version 2.1.2.).
- Analyse des séquences
   Le GPAO-J étant une maladie autosomale dominante, les données de séquence obtenues ont été analysées afin de détecter la présence de sites d'hétérozygotie parmi les patients atteints de glaucome juvénile. Les sites d'hétérozygotie ont été confirmés après comparaison des séquences des deux brins d'ADN génomique de chaque individu concerné. Un site d'hétérozygotie est retenu comme mutation candidate responsable de la survenue de troubles liés à FMO s'il est présent dans une population de membres d'une même famille, alors qu'il est absent en général chez les contrôles non apparentés à la famille.
- Résultats
   Parmi tous les fragments d'amplification dérivés du BAC 123H04M étudiés, l'un d'entre eux présente un site d'hétérozygotie ségrégeant avec la survenue du glaucome juvénile dans un pédigree représenté sur la Figure 8.

Ce site d'hétérozygotie (G/A) est présent chez 7 patients atteints de GPAO-J tandis qu'il est absent de 3 patients sains homozygotes (G/G), tous issus de la même famille. De plus, 99 contrôles non apparentés sont de même homozygotes (G/G) pour ce site, indiquant que la fréquence de l'allèle A dans la population générale est inférieure à 0.005.

Le site est contenu dans l'exon 8 du gène codant pour la protéine hFM02 selon l'invention ; la mutation décrite transforme l'acide glutamique en position 402 de la séquence SEQ ID N° 1 de la hFM02 en lysine (Figure 1).

Il est surprenant de remarquer que le calcul des lod scores intégrant les données précédentes pour différentes hypothèses de fréquences de chaque allèle dans la population générale, indique une probabilité supérieure à 100 contre 1 que l'hétérozygotie (G/A) décrite soit liée au GPAO-J (Tableau 6). Cette probabilité est significative du fait que l'analyse a porté sur une seule famille.

Les amorces ayant permis l'amplification du fragment d'ADN contenant ce site d'hétérozygotie sont décrites dans le Tableau 1.

**Tableau 1 : Séquences des amorces utilisées pour amplifier la région exonique dérivée du BAC 123H04M et contenant un site d'hétérozygotie lié au GPAO juvénile**

| | |
|---|---|
| Locus du fragment : | FMO2/Exon 8 |
| Taille du fragment amplifié : | 420 |
| Amorces: Amont PU (SEQ ID N°7) : | 5'TCACATAGAGTGCTATGGGGG |
| Aval RP (SEQ ID N°8) : | 5'CTTAGGAAGAAGATAAAAATGCAAC |

**Tableau 2: Exemples d'amorces pour détecter la mutation G.1263mac.A par « Single Nucleotide Primer Extension »**

| | | | | |
|---|---|---|---|---|
| a) | SEQ ID N°9 : | 5' | AATGTCCATCATCATAGTTCTCT 3' | (antisens) |
| | | et/ou | | |
| b) | SEQ ID N°10 : | 5' | TAGGCTTGTGTAGCCTGCCCTCA 3' | (sens) |

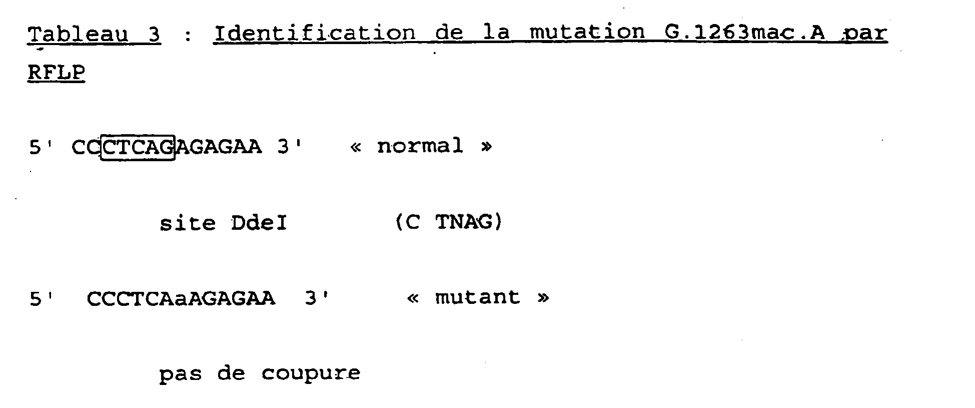

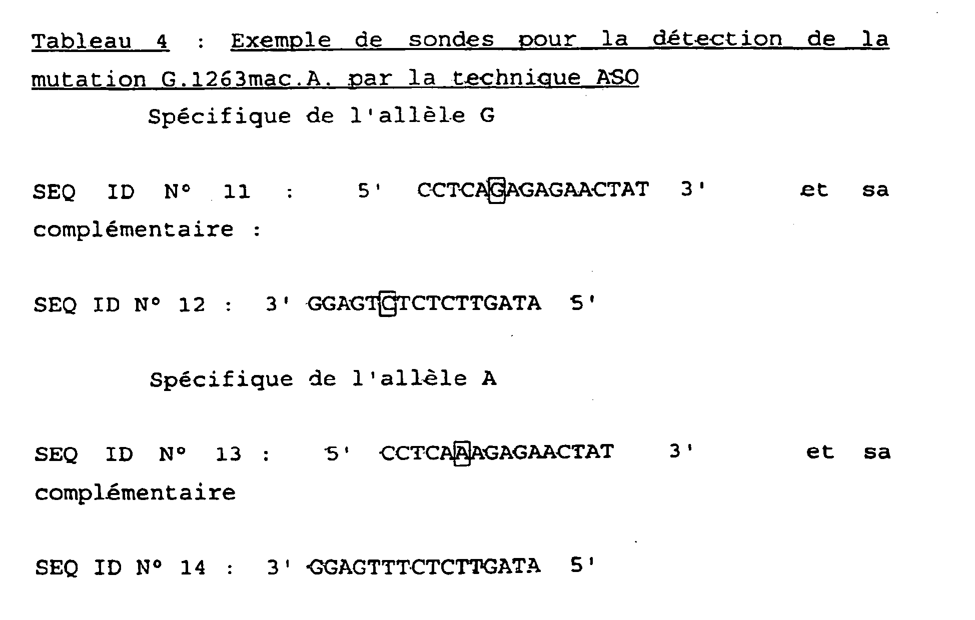

**Tableau 5 : Séquence des amorces utilisées pour le séquençage des fragments d'amplification à partir d'ADN génomique**

| | |
|---|---|
| PU | 5' TGTAAAACGACGGCCAGT |
| RP | 5' CAGGAAACAGCTATGACC |

**Tableau 6 : Lod score entre le polymorphisme G.1263mac. A et le GPAO juvénile dans la famille étudiée en fonction de la fréquence des deux allèles dans la population générale.**

| Fréquence de l'allèle rare (A) | Θ (taux de recombinaison) | Lod score |
|---|---|---|
| 0.01 | 0 | 2.07 |
| 0.001 | 0 | 2.10 |
| 0.0001 | 0 | 2.10 |
| 0.00001 | 0 | 2.10 |

### SEQUENCE LISTING

SEQ ID N° 1
SEQ ID N° 2
SEQ ID N° 3
SEQ ID N° 4
SEQ ID N° 5
SEQ ID N° 6
SEQ ID N° 7
SEQ ID N° 8
SEQ ID N° 9
SEQ ID N° 10
SEQ ID N° 11
SEQ ID N° 12
SEQ ID N° 13
SEQ ID N° 14

### RÉFÉRENCES

Allen J.B., Walberg M.W., Edwards M.C. & Elledge S.J. Finding prospective partners in the library: the two hybrid system and phage display find a match. TIBS 20: 511-516 (1995).
Altschul, Stephen F., Gish W., Miller W., Myers E. W., & Lipman D.J.
   Basic local alignment search tool.
   J. Mol. Biol. 215:403-10 (1990).
Bairoch A. & Apweiler R. The SWISS-PROT protein sequence data bank and its new supplement TREMBL. Nucleic Acids Res. 24: 21-25 (1996).
Belmouden A., Adam M.F., Dupont de Dinechin S., Brézin A.P., Rigault P., Chumakov I., Bach J-F., & Garchon H-J., 1996, Recombinational and physical mapping of the locus for primary open-angle glaucoma (GLC1A) on chromosome 1q23-q25. Genomics, sous presse.
Benson D. A., Boguski M., Lipman D. J. & Ostell J. GenBank. Nucleic Acids Res. 24: 1-5 (1996).
Bonfield J. K., Smith K. F. & Staden R. A new DNA sequence assembly program.
   Nucleic Acids Res. 23: 4992-9 (1995).
Buckholz R.G. Yeast Systems for the Expression of Heterologous Gene Products. Curr. Op. Biotechnology 4: 538-542 (1993).
Cashman J.R., Park, B.P., Berkman, C.E. & Cashman, L.E. Rôle of hepatic flavin-monoxygenase 3 in drug and chemical metabolism in adult humans. Chemico-Biological Interactions 96: 33-46 (1995).
Carter B.J. Adeno-Associated virus vectors. Curr. Op. Biotechnology 3: 533-539 (1993).
Cherif D., Julier C., Delattre O., Derré J. Lathrop G.M., & Berger R.: Simultaneous localization of cosmids and chromosome R-banding by fluorescence microscopy - Applications to régional mapping of chromosome 11. Proc.Natl.Acad.Sci. USA. 87: 6639-6643 (1990).
Chumakov I., Rigault P., Guillou S., Ougen P., Billault A., Guasconi G., Gervy P., Le Gall I., Soularue P., Grinas P. et al. Continuum of overlapping clones spanning the entire human chromosome 21q. Nature 359: 380-386 (1992).
Chumakov I.M., Rignault P., Le Gall I. et al. A YAC contig map of the human genome. Nature 377 supplt: 175-183 (1995).
Compton J. Nucleic Acid Sequence-Based Amplification. Nature 350: 91-92 (1991).
Danos O., Moullier P. & Heard J.M. Réimplantation de cellules génétiquement modifiées dans des néo-organes vascularisés. Médecine/Sciences 9:62-64 (1993).
Edwards C.P. et Aruffo A. Current applications of COS cell based transient expression systems. Curr. Op. Biotechnology 4: 558-563 (1993).
Epstein A. : Les vecteurs herpétiques pour le transfert de gènes - Médecine/Sciences 8: 902-911 (1992).
George D. G., Barker W . C . , . Mewes H. W, Pfeiffer F. & Tsugita A. The PIR-International Protein Sequence Database. Nucleic Acids Res. 24: 17-20 (1996).
Guatelli J.C. et al. Isothermal in vitro amplification of nucleic acids by a multienzyme reaction modeled after retroviral replication. Proc. Natl. Acad. Sci. USA 87: 1874-1878 (1990).
Hillier L. & Green P. OSP: a computer program for choosing PCR and DNA sequencing primers. PCR Methods Appl. 1: 124-8 (1991).
Hines et al., Toxicol. Appl. Pharmacol. 125, 1-6 (1994).
Landegren U., Kaiser R., Sanders J. & Hood L.A ligase-mediated gene détection technique. Science241: 1077-1080 (1988).
Lawton M.P., Cashman J.R., Cresteil T., Dolphin C.T., Elfarra A.A., Hines R.N., Hodgson E., Kimura T., Ozols J., Phillips I.R., Philpot R.M., Poulsen L.L., Rettie A.E., Shephard E.A., Williams D.E., & Ziegler D.M.: A nomenclature for the mammalian flavin-containing monooxygenase gene family based on amino acid sequence identities. Arch. Biochem. Biophys. 308:1, 254-257 (1994).
Luban J. & Goff S.P. The yeast two-hybride system for studying protein - protein interactions. Current Op. Biotechnology 1995, 6:59-64.
Luckow V.A. Baculovirus systems for the expression of human gene products. Curr. Op. Biotechnology 4: 564-572 (1993).
Olins P.O. et Lee S.C. Recent advances in heterologous gene expression in E. coli. Curr. Op. Biotechnology 4:520-525 (1993).
Park, S.B. et al., Chem. Res. Toxicol. 5, 193-201 (1992).
Perricaudet M., Stratford-Perricaudet L., & Briand P. : La thérapie génique par adénovirus - La Recherche 23: 471-473 (1992).
Poulsen, L.L. et al., Chem. Biol. Interact. 96, 57-73 (1995).
Rodriguez-Tome P., Stoehr P. J., Cameron G. N., & Flores T. P. The European Bioinformatics Institute (EBI) databases. Nucleic Acids Res. 24: 6-12 (1996).
Samiotaki M., Kwiatkowksi M. Parik J., & Landegren U. Dual-color detection of DNA sequence variants through ligase-mediated analysis. Genomics 20: 238-242 (1994).
Schwartzman, M.L., Masferrer, J., Dunn M.W., MoGiff J.C., Abracham N.G., 1987, Curr Eye Res. 6: 623-630.
Schwartzman M.L., Balazy M., Masferrer J., Abraham, N.G., McGiff, J.C., Murphy, R.C., 1987, PNAS USA 84 : 8125-8129.
Stoneking M., Hedgecock D., Higuchi R.G., Vigilant L., & Erlich H.A. Population variation of human DNA control region sequences by enzymatic amplification and sequence-specific oligonucleotide probes. Am. J. Hum. Genet. 48: 370-382 (1991).
Sunden S.L.F., Alward W.L.M., Nichols B.E., Rokhlina T.R., Nystuen A., Stone E.M. & Sheffield V.C. Fine mapping of the autosomal dominant juvenile open angle glaucome (GLC1A) region and evaluation of candidate gènes. Genome research 6: 862-869 (1996).
Syvänen A.C., Aalto-Setala K., Harju L., Kontula K., & Soderlund H. A primer-guided nucleotide incorporation assay in the genotyping of Apo E. Genomics 8: 684-692 (1990).
Temin H.M. : Retrovirus vectors for gene transfer. In Kucherlapati R., ed. Gene Transfer, New York, Plenum Press, 149-187 (1986).
Walker G.T., Fraiser M.S., Schram J.L., Little M.C., Nadeau J.G., & Malinowski D.P. Strand displacement amplification : an isothermal in vitro DNA amplification technique. Nucleic Acids Res. 20: 1691-1696 (1992).
Wu D.Y., Ugozzoli L. Pal B.K., Wallace R.B. Allele-specific amplification of b-globin genomic DNA for diagnosis of sickle cell anemia. Proc. Natl. Acad. Sci. USA 86: 2757-2760 (1989).
Ziegler, D.M., , Drug Metab. Rev. 19, 1-32 (1988).
Ziegler, D.M., Annu. Rev. Pharmacol. Toxicol., 33, 179-199 (1993).

## Revendications

1. Séquence nucléotidique isolée, **caractérisée en ce qu'**elle code pour le variant de la protéine FM02 humaine de séquence SEQ ID N° 3 qui présente un résidu lysine à la place d'un résidu acide glutamique en position 402 de la séquence SEQ ID N° 3.

2. Séquence nucléotidique isolée selon la revendication 1, **caractérisée en ce qu'**elle code pour le variant de la protéine FMO2 humaine de séquence SEQ ID N° 3 qui présente un résidu lysine à la place d'un résidu acide glutamique en position 402 de la séquence SEQ ID N° 3 et qu'elle comprend une séquence choisie parmi :
a) la séquence nucléotidique allant du nucléotide en position 2001 au nucléotide en position 2056 de la séquence nucléotidique SEQ ID N° 1,
b) la séquence nucléotidique allant du nucléotide en position 2405 au nucléotide en position 2542 de la séquence nucléotidique SEQ ID N° 1,
c) la séquence nucléotidique allant du nucléotide en position 10026 au nucléotide en position 10214 de la séquence nucléotidique SEQ ID N° 1,
d) la séquence nucléotidique allant du nucléotide en position 13341 au nucléotide en position 13503 de la séquence nucléotidique SEQ ID N° 1,
e) la séquence nucléotidique allant du nucléotide en position 16036 au nucléotide en position 16178 de la séquence nucléotidique SEQ ID N° 1,
f) la séquence nucléotidique allant du nucléotide en position 20558 au nucléotide en position 20757 de la séquence nucléotidique SEQ ID N° 1,
g) la séquence nucléotidique allant du nucléotide en position 21972 au nucléotide en position 22327 de la séquence nucléotidique SEQ ID N° 1,
h) la séquence nucléotidique allant du nucléotide en position 24411 au nucléotide en position 24483 de la séquence nucléotidique SEQ ID N° 1 dans laquelle le nucléotide en position 24431 de la séquence SEQ ID N° 1 est une adénosine à la place d'une guanine,
i) la séquence nucléotidique allant du nucléotide en position 25487 au nucléotide en position 25899 de la séquence nucléotidique SEQ ID N° 1,
j) la séquence nucléique SEQ ID N° 1 dans laquelle le nucléotide en position 24431 est une adénosine à la place d'une guanine, et
k) la séquence nucléique SEQ ID N° 2 dans laquelle le nucléotide en position 1266 est une adénosine à la place d'une guanine.

3. Séquence nucléotidique isolée, **caractérisée en ce qu'**elle est choisie parmi les séquences codant pour un polypeptide comprenant le polypeptide de séquence SEQ ID N° 3 qui présente un résidu lysine à la place d'un résidu acide glutamique en position 402 ou complémentaire d'une séquence selon la revendication 1 ou 2.

4. Amorce nucléique, **caractérisée en ce que** sa séquence est choisie parmi les séquences SEQ ID N° 7, SEQ ID N° 8, SEQ ID N° 9 et SEQ ID N° 10.

5. Sonde nucléique, **caractérisée en ce que** sa séquence est choisie parmi les séquences SEQ ID N° 11, SEQ ID N° 12, SEQ ID N° 13 et SEQ ID N° 14.

6. Polypeptide codé par une séquence nucléotidique selon l'une des revendications 1 à 3.

7. Polypeptide selon la revendication 6 de séquence d'acides aminés SEQ ID N° 3 qui présente un résidu lysine à la place d'un résidu acide glutamique en position 402.

8. Vecteur de clonage et/ou d'expression dans une cellule hôte appropriée d'une séquence nucléotidique, **caractérisé en ce qu'**il comporte une séquence selon l'une des revendications 1 à 3.

9. Vecteur selon la revendication 8, **caractérisé en ce qu'**il comporte les éléments permettant l'expression et/ou la sécrétion desdites séquences dans ladite cellule hôte.

10. Vecteur selon l'une des revendications 8 ou 9, **caractérisé en ce qu'**il s'agit d'un vecteur à réplication autonome.

11. Vecteur selon l'une des revendications 8 ou 9, **caractérisé en ce qu'**il s'agit d'un vecteur d'intégration chromosomique.

12. Vecteur selon l'une des revendications 8 à 11, **caractérisé en ce qu'**il s'agit d'un vecteur viral.

13. Vecteur selon la revendication 12, **caractérisé en ce que** le vecteur est réalisé sur la base d'un adénovirus, d'un AAV, d'un rétrovirus, d'un poxvirus ou d'un virus herpétique.

14. Cellule transformée par un vecteur selon l'une des revendications 8 à 13.

15. Cellule selon la revendication 14, **caractérisée en ce qu'**il s'agit d'une cellule procaryote.

16. Cellule selon la revendication 14, **caractérisée en ce qu'**il s'agit d'une cellule eucaryote.

17. Animal non humain, **caractérisé en ce qu'**il contient une cellule selon l'une des revendications 14 à 16.

18. Procédé de production de polypeptide selon les revendications 6 et 7 recombinant, **caractérisé en ce qu'**on cultive une cellule selon l'une des revendications 14 à 16 et **en ce que** l'on récupère la protéine produite.

19. Anticorps anti-FMO2 mutée humaine polyclonaux ou monoclonaux spécifiquement dirigés contre un polypeptide selon l'une des revendications 6 ou 7 comportant la mutation lysine en position 402 de la séquence SEQ ID N° 3.

20. Anticorps polyclonaux ou monoclonaux selon la revendication 19, **caractérisés en ce qu'**ils sont obtenus par réaction immunologique d'un organisme humain ou animal avec un agent immunogène constitué par un polypeptide selon l'une des revendications 6 ou 7 comportant la mutation lysine en position 402 de la séquence SEQ ID N° 3.

21. Anticorps selon l'une des revendications 19 ou 20, **caractérisés en ce qu'**il s'agit d'un anticorps marqué.

22. Utilisation d'un anticorps selon la revendication 21, pour la préparation d'une composition destinée à l'imagerie.

23. Utilisation de cellules selon l'une des revendications 14 à 16 ou d'un animal selon la revendication 17, pour la sélection *in vitro* de produits impliqués directement ou indirectement dans l'activité de la FMO2 mutée.

24. Utilisation de cellules selon l'une des revendications 14 à 16 ou d'un animal selon la revendication 17, pour la sélection *in vitro* de produits interagissant avec la FMO2 mutée.

25. Méthode de diagnostic d'une prédisposition au glaucome juvénile chez un patient, **caractérisée en ce qu'**on détermine à partir d'un prélèvement biologique dudit patient la présence de la mutation G24431A dans le gène FM02 par l'analyse de tout ou partie d'une séquence nucléique correspondant audit gène, la présence d'au moins une telle mutation étant indicative d'une prédisposition dudit patient au glaucome juvénile.

26. Méthode selon la revendication 25, dans laquelle la séquence d'acide nucléique analysée est un ADN génomique, un ADNc ou un ARNm.

27. Méthode selon l'une des revendications 25 ou 26, **caractérisée en ce que** ladite analyse est réalisée par hybridation.

28. Méthode selon l'une des revendications 25 à 27, **caractérisée en ce que** la présence d'une mutation est détectée par comparaison avec la séquence correspondante naturelle non mutée.

29. Méthode selon la revendication 27, **caractérisée en ce que** ladite hybridation est réalisée à l'aide d'au moins une sonde oligonucléotidique spécifique de l'allèle.

30. Méthode selon l'une des revendications 25 ou 26, **caractérisée en ce que** ladite analyse est réalisée par séquençage.

31. Méthode selon l'une des revendications 25 ou 26, **caractérisée en ce que** ladite analyse est réalisée par migration électrophorétique, et plus particulièrement par SSCP ou DGGE.

32. Méthode selon l'une des revendications 25 à 31, **caractérisée en ce que** tout ou partie de la séquence nucléique du gène FMO2 mutée est amplifiée préalablement à la mise en évidence de la mutation.

33. Méthode selon la revendication 32, **caractérisée en ce que** l'amplification est réalisée par PCR ou par une méthode choisie parmi la méthode NASBA (Nucleic Acid Séquence based Amplification), TAS (Transcription based Amplification System), LCR (Ligase Chain Reaction), ERA (Endo Run Amplifification), CPR (Cycling Probe Reaction) et SDA (Strand Displacement Amplification).

34. Méthode selon l'une des revendications 32 ou 33, **caractérisée en ce que** les amorces choisies pour réaliser l'amplification sont choisies parmi les amorces définies selon la revendication 4.

35. Réactif pour détecter et/ou identifier la mutation G24431A de la séquence SEQ ID N° 1 du gène FMO2 humain dans un échantillon biologique, **caractérisé en ce qu'**il comprend une sonde dite de capture et/ou une sonde dite de détection, l'une au moins de ces sondes comportant une séquence selon la revendications 5, ou **en ce qu'**il comporte un anticorps selon l'une des revendications 19 à 21.

36. Méthode de diagnostic d'une prédisposition au glaucome juvenile chez un patient, **caractérisée en ce qu'**on détermine à partir d'un prélèvement biologique dudit patient la présence de la FMO2 mutée correspondant à la mutation G24431A de la séquence SEQ ID N° 1 du gène FMO2 à l'aide d'un anticorps mono- ou polyclonal selon l'une des revendications 19 à 21.

37. Méthode selon la revendication 36, **caractérisée en ce que** la détection est effectuée par un procédé ELISA ou RIA.

38. Composition thérapeutique **caractérisée en ce qu'**elle comporte à titre de principe actif au moins un agoniste de la FMO2 humaine mutée correspondant à la mutation G24431A de la séquence SEQ ID N° 1, ledit agoniste étant choisi parmi les composés suivants :
a) un polypeptide selon la revendication 6 ou 7,
b) un vecteur d'expression selon l'une des revendications 8 à 13,
c) une séquence nucléotidique selon l'une des revendications 1 à 3, **caractérisée en ce que** ladite séquence est une séquence sens induisant l'expression de ladite FMO2 mutée.

39. Composition thérapeutique, **caractérisée en ce qu'**elle comporte à titre de principe actif un antagoniste de la FM02 humaine mutée correspondant à la mutation G24431A de la séquence SEQ ID N° 1, ledit antagoniste étant choisi parmi les composés suivants :
a) un anticorps anti-FMO2 mutée, selon l'une des revendications 19 à 22,
b) une séquence nucléotidique selon l'une des revendications 1 à 3, **caractérisée en ce que** ladite séquence est une séquence antisens inhibant l'expression de ladite FMO2 mutée,
c) une séquence nucléotidique selon l'une des revendications 1 à 3, **caractérisée en ce que** ladite séquence est une séquence sens inhibant l'expression de ladite FM02 mutée.

40. Utilisation d'un principe actif capable de moduler l'activité de la FMO2 humaine mutée correspondant à la mutation G24431A de la séquence SEQ ID N° 1, pour réaliser un médicament destiné au traitement et/ou à la prévention de glaucome juvénile, **caractérisée en ce que** ledit principe actif est choisi parmi un agoniste ou un antagoniste tel que défini dans les revendications 38 et 39.

## Claims

1. Isolated nucleotide sequence, **characterised in that** it codes for the variant of the human FM02 protein in sequence SEQ ID NO:3 which has a lysine residue instead of a glutamic acid residue in position 402 of sequence SEQ ID NO:3.

2. Isolated nucleotide sequence according to claim 1, **characterised in that** it codes for the variant of the human FM02 protein in sequence SEQ ID NO:3 which has a lysine residue instead of a glutamic acid residue in position 402 of sequence SEQ ID NO:3 and comprises a sequence selected from among:
a) The nucleotide sequence ranging from the nucleotide in position 2001 to the nucleotide in position 2056 of the nucleotide sequence SEQ ID NO:1,
b) The nucleotide sequence ranging from the nucleotide in position 2405 to the nucleotide in position 2542 of the nucleotide sequence SEQ ID NO:1,
c) The nucleotide sequence ranging from the nucleotide in position 10026 to the nucleotide in position 10214 of the nucleotide sequence SEQ ID NO:1,
d) The nucleotide sequence ranging from the nucleotide in position 13341 to the nucleotide in position 13503 of the nucleotide sequence SEQ ID N0:1,
e) The nucleotide sequence ranging from the nucleotide in position 16036 to the nucleotide in position 16178 of the nucleotide sequence SEQ ID NO:1,
f) The nucleotide sequence ranging from the nucleotide in position 20558 to the nucleotide in position 20757 of the nucleotide sequence SEQ ID NO:1,
g) The nucleotide sequence ranging from the nucleotide in position 21972 to the nucleotide in position 22327 of the nucleotide sequence SEQ ID NO:1,
h) The nucleotide sequence ranging from the nucleotide in position 24411 to the nucleotide in position 24483 of the nucleotide sequence SEQ ID NO:1 in which the nucleotide in position 24431 of the sequence SEQ ID NO:1 is an adenosine instead of a guanine,
i) The nucleotide sequence ranging from the nucleotide in position 25487 to the nucleotide in position 25899 of the nucleotide sequence SEQ ID NO:1,
j) The nucleic sequence SEQ ID NO:1 in which the nucleotide in position 24431 is an adenosine instead of a guanine, and,
k) The nucleic sequence SEQ ID NO:2 in which the nucleotide in position 1266 is an adenosine instead of a guanine.

3. Isolated nucleotide sequence, **characterised in that** it is selected from among the sequences coding for a polypeptide consisting of the polypeptide in sequence SEQ ID NO:3 which has a lysine residue instead of a glutamic acid residue in position 402 or complementary of a sequence according to claim 1 or 2.

4. Nucleic primer, **characterised in that** its sequence is selected from among sequences SEQ ID NO:7, SEQ ID NO:8, SEQ ID NO:9 and SEQ ID NO:10.

5. Nucleic probe, **characterised in that** its sequence is selected from among sequences SEQ ID NO:11, SEQ ID NO:12, SEQ ID NO:13 and SEQ ID NO:14.

6. Polypeptide coded by a nucleotide sequence according to one of claims 1 to 3.

7. Polypeptide according to claim 6 of amino acid sequence SEQ ID NO:3 which has a lysine residue instead of a glutamic acid residue in position 402.

8. Cloning and/or expression vector in an appropriate host cell of a nucleotide sequence, **characterised in that** it comprises a sequence according to one of claims 1 to 3.

9. Vector according to claim 8, **characterised in that** it contains elements allowing expression and/or secretion of said sequences in said host cell.

10. Vector according to one of claims 8 or 9, **characterised in that** it is an autonomous replication vector.

11. Vector according to one of claims 8 or 9, **characterised in that** it is a chromosome integration vector.

12. Vector according to one of claims 8 to 11, **characterised in that** it is a viral vector.

13. Vector according to claim 12, **characterised in that** the vector is formed on the base of an adenovirus, AAV, retrovirus, poxvirus or herpes virus.

14. Cell transformed by a vector according to one of claims 8 to 13.

15. Cell according to claim 14, **characterised in that** it is a prokaryote cell.

16. Cell according to claim 14, **characterised in that** it is a eukaryote cell.

17. Non-human animal, **characterised in that** it contains a cell according to one of claims 14 to 16.

18. Process for production of recombinant polypeptide according to claims 6 and 7, **characterised in that** a cell is cultured according to one of claims 14 to 16 and the protein produced is recovered.

19. Polyclonal or monoclonal human mutated anti-FM02 antibodies specifically targeted against a polypeptide according to one of claims 6 or 7 containing the lysine mutation in position 402 of the sequence SEQ ID NO:3.

20. Polyclonal or monoclonal antibodies according to claim 19, **characterised in that** they are obtained by immunological reaction of a human or animal organism with an immunogenic agent consisting of a polypeptide according to one of claims 6 or 7 containing the lysine mutation in position 402 of the sequence SEQ ID NO:3.

21. Antibodies according to one of claims 19 or 20, **characterised in that** they are marked antibodies.

22. Use of an antibody according to claim 21 for the preparation of a composition designed for imaging.

23. Use of cells according to one of claims 14 to 16 or an animal according to claim 17 for *in vitro* selection of products involved directly or indirectly in the mutated FM02 activity.

24. Use of cells according to one of claims 14 to 16 or an animal according to claim 17 for *in vitro* selection of products interacting with the mutated FM02.

25. Method of diagnosis of a predisposition to juvenile glaucoma in a patient, **characterised in that** the presence of the G24431A mutation in the FM02 gene is determined from a biological sample from said patient by analysis of all or part of a nucleic sequence corresponding to said gene, the presence of at least such mutation being indicative of a predisposition in said patient to juvenile glaucoma.

26. Method according to claim 25, in which the nucleic acid sequence analysed is a genomic DNA, a DNAc or an RNAm.

27. Method according to one of claims 25 or 26, **characterised in that** said analysis is carried out by hybridisation.

28. Method according to one of claims 25 to 27, **characterised in that** the presence of a mutation is detected by comparison with the corresponding natural, unmutated sequence.

29. Method according to claim 27, **characterised in that** said hybridisation is carried out using at least one allele-specific oligonucleotide probe.

30. Method according to one of claims 25 or 26, **characterised in that** said analysis is carried out by sequencing.

31. Method according to one of claims 25 or 26, **characterised in that** said analysis is carried out by electrophoretic migration and more particularly by SSCP or DGGE.

32. Method according to one of claims 25 to 31, **characterised in that** all or part of the nucleic sequence of the mutated FM02 gene is amplified prior to detection of the mutation.

33. Method according to claim 32, **characterised in that** the amplification is carried out by PCR or by a method chosen from among the methods NASBA (Nucleic Acid Sequence Based Amplification), TAS (Transcription based Amplification System), LCR (Ligase Chain Reaction), ERA (Endo Run Amplification), CPR (Cycling Probe Reaction) and SDA (Strand Displacement Amplification).

34. Method according to one of claims 32 or 33, **characterised in that** the primers chosen to carry out the amplification are selected from among the primers defined according to claim 4.

35. Reagent to detect and/or identify the G24431A mutation of the sequence SEQ ID NO:1 of the human FM02 gene in a biological sample, **characterised in that** it comprises a probe called a capture probe and/or a probe called a detection probe, at least one of said probes containing a sequence according to claim 5, or **in that** it contains an antibody, according to one of claims 19 to 21.

36. Method of diagnosis of a predisposition to juvenile glaucoma in a patient, **characterised in that** the presence of the mutated FM02 corresponding to the G24431A mutation of the sequence SEQ ID NO:1 of the FM02 gene is determined from a biological sample from said patient, using a monoclonal or polyclonal antibody according to one of claims 19 to 21.

37. Method according to claim 36, **characterised in that** the detection is carried out by an ELISA or RIA process.

38. Therapeutic composition, **characterised in that** it contains as its active principle at least one agonist of the mutated human FM02 corresponding to the G24431A mutation of the sequence SEQ ID N0:1, said agonist being selected from among the following compounds:
a) A polypeptide according to claim 6 or 7,
b) An expression vector according to one of claims 8 to 13,
c) A nucleotide sequence according to one of claims 1 to 3, **characterised in that** said sequence is a sense sequence inducing the expression of said mutated FM02.

39. Therapeutic composition, **characterised in that** it contains as its active principle an antagonist of the mutated human FM02 corresponding to the G24431A mutation of the sequence SEQ ID NO:1, said antagonist being selected from among the following compounds:
a) A mutated anti-FM02 antibody according to one of claims 19 to 22,
b) A nucleotide sequence according to one of claims 1 to 3, **characterised in that** said sequence is an antisense sequence inhibiting the expression of said mutated FM02.
c) A nucleotide sequence according to one of claims 1 to 3, **characterised in that** said sequence is a sense sequence inhibiting the expression of said mutated FM02.

40. Use of an active principle capable of modulating the activity of the mutated human FM02 corresponding to the G24431A mutation of the sequence SEQ ID NO:1, to produce a medication designed for the treatment and/or prevention of juvenile glaucoma, **characterised in that** said active principle is selected from among an agonist or an antagonist as defined in claims 38 and 39.

## Patentansprüche

1. Isolierte Nukleotidsequenz, **dadurch gekennzeichnet, dass** sie für die humane Variante des Proteins FM02 der Sequenz SEQ ID Nr. 3 codiert, die einen Lysinrest anstelle eines Glutaminsäurerests in Position 402 der Sequenz SEQ ID Nr. 3 aufweist.

2. Isolierte Nukleotidsequenz nach Anspruch 1, **dadurch gekennzeichnet, dass** sie für die Variante des humanen Proteins FM02 der Sequenz SEQ ID Nr. 3 codiert, die einen Lysinrest anstelle eines Glutaminsäurerests in Position 402 der Sequenz SEQ ID Nr. 3 aufweist und dass sie eine Sequenz umfasst, ausgewählt aus:
a) der Nukleotidsequenz von dem Nukleotid in Position 2001 bis zu dem Nukleotid in Position 2056 der Nukleotidsequenz SEQ ID Nr. 1,
b) der Nukleotidsequenz von dem Nukleotid in Position 2405 bis zu dem Nukleotid in Position 2542 der Nukleotidsequenz SEQ ID Nr. 1,
c) der Nukleotidsequenz von dem Nukleotid in Position 10026 bis zu dem Nukleotid in Position 10214 der Nukleotidsequenz SEQ ID Nr. 1,
d) der Nukleotidsequenz von dem Nukleotid in Position 13341 bis zu dem Nukleotid in Position 13503 der Nukleotidsequenz SEQ ID Nr. 1,
e) der Nukleotidsequenz von dem Nukleotid in Position 16036 bis zu dem Nukleotid in Position 16178 der Nukleotidsequenz SEQ ID Nr. 1,
f) der Nukleotidsequenz von dem Nukleotid in Position 20558 bis zu dem Nukleotid in Position 20757 der Nukleotidsequenz SEQ ID Nr. 1,
g) der Nukleotidsequenz von dem Nukleotid in Position 21972 bis zu dem Nukleotid in Position 22327 der Nukleotidsequenz SEQ ID Nr. 1,
h) der Nukleotidsequenz von dem Nukleotid in Position 24411 bis zu dem Nukleotid in Position 24483 der Nukleotidsequenz SEQ ID Nr. 1, in welcher das Nukleotid in Position 24431 der SEQ ID Nr. 1 ein Adenosin anstelle eines Guanins ist,
i) der Nukleotidsequenz von dem Nukleotid in Position 25487 bis zu dem Nukleotid in Position 25899 der Nukleotidsequenz SEQ ID Nr. 1,
j) der Nukleotidsequenz SEQ ID Nr. 1, in welcher das Nukleotid in Position 24431 ein Adenosin anstelle eines Guanins ist, und
k) der Nukleotidsequenz SEQ ID Nr. 2 in welcher das Nukleotid in Position 1266 ein Adenosin anstelle eines Guanins ist.

3. Isolierte Nukleotidsequenz, **dadurch gekennzeichnet, dass** sie ausgewählt ist aus den Sequenzen, die für ein Polypeptid codieren, umfassend das Polypeptid der Sequenz SEQ ID Nr. 3, das einen Lysinrest anstelle eines Glutaminsäurerests in Position 402 aufweist, oder komplementär ist zu einer Sequenz nach Anspruch 1 oder 2.

4. Nukleotidprimer, **dadurch gekennzeichnet, dass** seine Sequenz ausgewählt ist aus den Sequenzen SEQ ID Nr. 7, SEQ ID Nr. 8, SEQ ID Nr. 9 und SEQ ID NR. 10.

5. Nukleotidsonde, **dadurch gekennzeichnet, dass** ihre Sequenz ausgewählt ist aus den Sequenzen SEQ ID Nr. 11, SEQ ID Nr. 12, SEQ ID Nr. 13 und SEQ ID NR. 14.

6. Polypeptid, codiert durch eine Nukleotidsequenz nach einem der Ansprüche 1 bis 3.

7. Polypeptid nach Anspruch 6 mit der Aminosäuresequenz SEQ ID Nr. 3, das einen Lysinrest anstelle eines Glutaminsäurerests in Position 402 aufweist.

8. Klonierungsvektor und/oder Expressionsvektor in einer geeigneten Wirtszelle mit einer Nukleotidsequenz, die **dadurch gekennzeichnet ist, dass** sie eine Sequenz nach einem der Ansprüche 1 bis 3 aufweist.

9. Vektor nach Anspruch 8, **dadurch gekennzeichnet, dass** er die Elemente umfasst, die die Expression und/oder Sezernierung der Sequenzen in die Wirtszelle zulassen.

10. Vektor nach einem der Ansprüche 8 oder 9, **dadurch gekennzeichnet, dass** es sich um einen Vektor zur autonomen Replikation handelt.

11. Vektor nach einem der Ansprüche 8 oder 9, **dadurch gekennzeichnet, dass** es sich um einen Chromosomenintegrationsvektor handelt.

12. Vektor nach einem der Ansprüche 8 bis 11, **dadurch gekennzeichnet, dass** es sich um einen viralen Vektor handelt.

13. Vektor nach Anspruch 12, **dadurch gekennzeichnet, dass** der Vektor realisiert wird auf Basis eines Adenovirus, eines AAV, eines Retrovirus, eines Pockenvirus oder eines Herpesvirus.

14. Zelle, transformiert durch einen Vektor nach einem der Ansprüche 8 bis 13.

15. Zelle nach Anspruch 14, **dadurch gekennzeichnet, dass** es sich um eine prokaryontische Zelle handelt.

16. Zelle nach Anspruch 14, **dadurch gekennzeichnet, dass** es sich um eine eukaryontische Zelle handelt.

17. Nichthumanes Lebewesen, **dadurch gekennzeichnet, dass** es eine Zelle nach einem der Ansprüche 14 bis 16 aufweist.

18. Verfahren zur Herstellung eines rekombinanten Polypeptids nach den Ansprüchen 6 und 7, **dadurch gekennzeichnet, dass** eine Zelle nach einem der Ansprüche 14 bis 16 kultiviert wird und das produzierte Protein gewonnen wird.

19. Polyklonale oder monoklonale Antikörper gegen mutiertes humanes FM02, die spezifisch gegen ein Polypeptid nach einem der Ansprüche 6 oder 7 gerichtet sind, das die Lysin-Mutation in Position 402 der Sequenz SEQ ID Nr. 3 aufweist.

20. Polyklonale oder monoklonale Antikörper nach Anspruch 19, **dadurch gekennzeichnet, dass** sie erhalten wurden durch Immunreaktion eines humanen oder tierischen Organismus mit einem immunogenen Mittel, bestehend aus einem Polypeptid nach einem der Ansprüche 6 oder 7, das die Lysin-Mutation in Position 402 der Sequenz SEQ ID Nr. 3 aufweist.

21. Antikörper nach einem der Ansprüche 19 oder 20, **dadurch gekennzeichnet, dass** es sich um einen markierten Antikörper handelt.

22. Verwendung eines Antikörpers nach Anspruch 21 zur Herstellung einer Zusammensetzung, die zur Bilddarstellung vorgesehen ist.

23. Verwendung von Zellen nach einem der Ansprüche 14 bis 16 oder von einem Lebewesen nach Anspruch 17 zur in vitro-Selektion von direkt oder indirekt mit der Aktivität von mutiertem FM02 in Zusammenhang stehenden Produkten.

24. Verwendung von Zellen nach einem der Ansprüche 14 bis 16 oder von einem Lebewesen nach Anspruch 17 zur in vitro-Selektion von Produkten, die mit mutiertem FM02 wechselwirken.

25. Diagnoseverfahren einer Prädisposition für juveniles Glaukom bei einem Patienten, **dadurch gekennzeichnet, dass** man zu Beginn einer biologischen Voruntersuchung des Patienten das Vorliegen der Mutation G24431A im Gen FM02 durch die Analyse der gesamten oder eines Teils einer Nukleinsäuresequenz, die dem Gen entspricht, bestimmt, wobei das Vorliegen mindestens einer solchen Mutation kennzeichnend für eine Prädisposition des Patienten für juveniles Glaukom ist.

26. Verfahren nach Anspruch 25, worin die analysierte Nukleinsäuresequenz eine genomische DNA, eine cDNA oder mRNA ist.

27. Verfahren nach einem der Ansprüche 25 oder 26, **dadurch gekennzeichnet, dass** die Analyse durch Hybridisierung realisiert wird.

28. Verfahren nach einem der Ansprüche 25 bis 27, **dadurch gekennzeichnet, dass** das Vorliegen einer Mutation nachgewiesen wird durch Vergleich mit der entsprechenden natürlichen, nichtmutierten Sequenz.

29. Verfahren nach Anspruch 27, **dadurch gekennzeichnet, dass** die Hybridisierung realisiert wird mit Hilfe mindestens einer Oligonukleotidsonde, die spezifisch für das Allel ist.

30. Verfahren nach einem der Ansprüche 25 oder 26, **dadurch gekennzeichnet, dass** die Analyse durch Sequenzieren realisiert wird.

31. Verfahren nach einem der Ansprüche 25 oder 26, **dadurch gekennzeichnet, dass** die Analyse durch elektrophoretische Migration und im Spezielleren durch SSCP oder DGGE realisiert wird.

32. Verfahren nach einem der Ansprüche 25 bis 31, **dadurch gekennzeichnet, dass** die gesamte oder ein Teil der Nukleinsäuresequenz des mutierten FMO-Gens vorab amplifiziert wird, wie durch Mutation gezeigt wird.

33. Verfahren nach Anspruch 32, **dadurch gekennzeichnet, dass** die Amplifikation realisiert wird durch PCR oder durch ein Verfahren, ausgewählt aus dem NASBA- (Nucleic Acid Sequence based Amplification), TAS- (Transcription based Amplification System), LCR-(Ligase Chain Reaction), ERA- (Endo Run Amplification), CPR- (Cycling Probe Reaction) und SDA- (Strand Displacement Amplification) Verfahren.

34. Verfahren nach einem der Ansprüche 32 oder 33, **dadurch gekennzeichnet, dass** die zur Realisierung der Amplifikation ausgewählten Primer ausgewählt werden aus den Primern nach Anspruch 4.

35. Reaktives Mittel zum Nachweisen und/oder Identifizieren der Mutation G24431A der Sequenz SEQ ID Nr. 1 des humanen FMO2-Gens in einer biologischen Probe, **gekennzeichnet dadurch, dass** es eine Einfangsonde und/oder eine Nachweissonde umfasst, wobei die mindestens eine dieser Sonden eine Sequenz nach Anspruch 5 umfasst, oder **dadurch**, dass es einen Antikörper nach einem der Ansprüche 19 bis 21 aufweist.

36. Diagnoseverfahren für eine Prädisposition für juveniles Glaukom eines Patienten, **dadurch gekennzeichnet, dass** man zu Beginn einer biologischen Voruntersuchung des Patienten das Vorliegen des mutierten FM02 entsprechend der Mutation G24431A der Sequenz SEQ ID Nr. 1 des FMO2-Gens mit Hilfe eines mono- oder polyklonalen Antikörpers nach einem der Ansprüche 19 bis 21 bestimmt.

37. Verfahren nach Anspruch 36, **dadurch gekennzeichnet, dass** der Nachweis durch ein ELISA- oder RIA-Verfahren erfolgt.

38. Therapeutische Zusammensetzung, **dadurch gekennzeichnet, dass** sie als aktives Mittel mindestens einen Agonisten des mutierten humanen FM02, entsprechend der Mutation G24431A der Sequenz SEQ ID Nr. 1, aufweist, wobei der Agonist ausgewählt ist aus den folgenden Zusammensetzungen:
a) einem Polypeptid nach Anspruch 6 oder 7,
b) einem Expressionsvektor nach einem der Ansprüche 8 bis 13,
c) einer Nukleotidsequenz nach einem der Ansprüche 1 bis 3, **dadurch gekennzeichnet, dass** die Sequenz eine Sense-Sequenz ist, die die Induktion der Expression des mutierten FM02 induziert.

39. Therapeutische Zusammensetzung, **dadurch gekennzeichnet, dass** sie als aktives Mittel einen Antagonisten des mutierten humanen FM02, entsprechend der Mutation G24431A der Sequenz SEQ ID Nr. 1, aufweist, wobei der Antagonist ausgewählt ist aus den folgenden Zusammensetzungen:
a) einem Antikörper gegen mutiertes FM02 nach einem der Ansprüche 19 bis 22,
b) einer Nukleotidsequenz nach.einem der Ansprüche 1 bis 3, **dadurch gekennzeichnet, dass** die Sequenz eine Antisense-Sequenz ist, die die Expression des mutierten FMO2 inhibiert,
c) einer Nukleotidsequenz nach einem der Ansprüche 1 bis 3, **dadurch gekennzeichnet, dass** die Sequenz eine Sense-Sequenz ist, die die Expression des mutierten FM02 inhibiert.

40. Verwendung eines aktiven Mittels, das in der Lage ist zum Modulieren der Aktivität von mutiertem humanem FM02, entsprechend der Mutation G24431A der Sequenz SEQ ID Nr. 1, zum Realisieren eines Medikaments, das vorgesehenen ist zur Behandlung und/oder Verhinderung von juvenilem Glaukom, **dadurch gekennzeichnet, dass** das aktive Mittel ausgewählt ist aus einem Agonisten oder Antagonisten nach einem der Ansprüche 38 und 39.


### LISTE DE SEQUENCES

(1) INFORMATIONS GENERALES:
   (i) DEPOSANT:
      (A) NOM: GENSET
      (B) RUE: 24 RUE ROYALE
      (C) VILLE: PARIS
      (E) PAYS: FRANCE
      (F) CODE POSTAL: 75008
   (ii) TITRE DE L' INVENTION: SEQUENCE NUCLEOTIDIQUE CODANT POUR UNE FLAVINE MONOOXYGENASE,PROTEINE CORRESPONDANTE ET LEURS APPLICATIONS DANS LE DOMAINE DU DIAGNOSTIC ET THERAPEUTIQUE
   (iii) NOMBRE DE SEQUENCES: 14
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
   (vi) DONNEES DE LA DEMANDE ANTERIEURE:
      (A) NUMERO DE LA DEMANDE: FR 9615032
      (B) DATE DE DEPOT: 06-AUG-1996
(2) INFORMATIONS POUR LA SEQ ID NO: 1:
   (i) CARACTERISTIQUES DE LA SEQUENCE:
      (A) LONGUEUR: 26016 paires de bases
      (B) TYPE: nucléotide
      (C) NOMBRE DE BRINS: double
      (D) CONFIGURATION: linéaire
   (ii) TYPE DE MOLECULE: ADN (génomique)
   (xi) DESCRIPTION DE LA SEQUENCE: SEQ ID NO: 1:
   CATCAGTTAT CCCTGGAGGA TAACTAAGCC ATCTGCCTCC ATCATCTTTT AAGGGTTCAG    60
   TCAGTTTAAA ACTTTGCTTC TATACCTAGG TATTTTCTTT TCTCTGTATG TTGGTCAGGT   120
   ACAATTATTT TTAACAGGGC TTCCATCAAT ATCATAACTA CCTAGAGAAG ACATTGCAAA   180
   GATAAAATTG GAGAATTGTT AACAGGCTGT TAACAAAATG TGTACCCAAC TGCCAATGAA   240
   GTGGCTTGAT TTTTTTCTTT TTTTAAAATT TTTCTTTTGT ATCCTTTTAT TTTATTTACT   300
   TATTTTTTAG AGACACAGTC TCGCTCTGTT ACCCAGGCTG GCGTACAATG GCACAATCAT 360
   AGCCCACTGC AGTCTCGACC TCCAGGGCCT CAAGCAATCC TGCCACCTCA GCCTTCCAAG 420
   TAGCTGAGAC TACAAGTGCA TGCTGCCATG CCTGACTGAT TTTTTGTTTT TTGCAGAGAT 480
   GAAGTCTCAC TATGTTGCCC AGTCTGACCT TGAACTCCTA GCAATACCCT AGCCTGGCCT 540
   CCCAAACTGC TGGGATTACA GGCATGAGCC ACTGTGCCTG GCCTTTTTTC ATTTTAACTG 600
   AGAAATGTGT TCAGCTCTTT TGTTCCTTAG TCATTGATCA TCACTTTTGT TATATCTGTT 660
   AGTCTTGTCA TAGAGTTGCT GCACTTATTA CACAGAGAAG GCCTTTTATC ACGACCAATT 720
   TATTTTAGGA AATTTCAGGG AAAACGTTTT TCTAGAACAC CTTATTTGAC ATTATAAAAC 780
   AACTCTTCAC TCTTGCACTC CAGACCTCCC TTTCCAGTTT TCTTTTTCTC CATAGTGGTC 840
   ATCACCACTT GTTTTATTTT ATTGATGGGC TGTCTGGCTC CCTCAACTGC AAAGTAAACT 900
   CCACAAAGGC AGAGAGTTTT GTCTCTTTTA TTCATTGCTG TACCTGCATC ACTTAGAAAG 960
   TTTCTGGCAC CTAGGAAGTG TTCAGTAAAT ATTTATTGAA TAAGTTTATG TAAAACGTCT 1020
   CAGACTCCTT AGAGAAACTG GTCTTTTGGG GTTGGAGAAT AAAGTTCTTT ACCTCATCAG 1080
   TTAGACTCTA TCTAAGGTAC ACGAGGGCTT GCTAGTCTCC TAAGTTAGTC TGCTAATAAA 1140
   TGTTAACCCT AATAACTGAA ATTATTAGCA GAGGTAATTA TCCAGTTCTA TATCAAGGCA 1200
   AAAAGACAGC AGTGGATAGA AAGATCTTAG AAGTCCCACT AGGTTCATCC AAGCCAGCAT 1260
   ACACATAGGC AGAAAAATCA AAATAAGATA TGAGCCTGGA CAGGGTGAGC AATCTGGGAA 1320
   AAGATGAACA CAGTATGCTA GGACCCAGAA ATCATCAAGT CTATGAAAAC TAAGCCAGAA 1380
   CACAAATGTG AATTCCATAA GATCAGGAAC ATAATCTGTC TTGTTCATCC AGGCATGGTA 1440
   ATCTGCCAGA AATAGTGCTT AACTGCAAGA ACTGAATATT TGTTAGATAA TTAAAGCATC 1500
   AACTAAATGA GATTCATGCA ACCATGAAAA ATGCTGCTAT AGGTACACAA TATTGATATA 1560
   CTAGAAAGTT AAAAAATCAA GTTGGAAATT AGACTATTCC ATTTCTGTTT GTGTGTATGT 1620
   ATCTACAAAT AGGTGGAAGG ATATACCAAA ATGTCAACAG CAGTTACCTC TGGGTGGTGA 1680
   GGAGTAATCT TAACCTTGTT ATTTATCCCT ATATGTTCAT TTGTGAATGA ATATTTATTA 1740
   CATCATTATA AAAAGGATTT TTAAACTATC TGTATGTTTA AGAGTATATG TTGCTACTAT 1800
   GTAAGAGTAT ATGCTGTTAC TGTAAAGACA TTGCATTACT ACTGTTGACC TCAGAGCACG 1860
   CGCCTCTTGC CTAATTCTAG GACTCCTAAC TAAGTCTTTG GAGTTTCAGC TGGAAGAATG 1920
   CTGGAGGAAT ACGGAACTCC TCCCATTTCT CACAGCCACC TCCAACTCTT AAAAACGCTT 1980
   CCAACTGCCT CCCAGCACAC AACCAAGGGA GAAAACTATT CTGTCAAAGA GACGGTGCCA 2040
   AAAGGCAAAA ACAAAGGTAA GGATGATCGC TGGGGAAAGA AGCTGAAAAG GAAAAGCTCA 2100
   GAACTCTAGC TGGAAATTTG GCTCACATCC CTAGTATGTT ACTGCATAGT CTGGCTTTGT 2160
   TCAATGGGTC GCTTTTAAAT ATTAAAGCTA GATGTAAGCA AGGTTTGCAA CAAAGTCCAT 2220
   AAGAAACTCA GCTTTTCTCA AAGGCAAGAA GAGAGCAGGA TTTTTGACTG GCTCTTTATT 2280
   CAATAGTGCT GCTTATTAAA TTACCACTGC TACAATGTTT AAAGCCAATT ACCTGAGCAC 2340
   ATCATAAGGA TTCTCTTACC GGTTGTCCCA GTTAAGTAAT GTTGATTGAT CAACTCCTTG 2400
   ACAGGAGCTG ATGGCAAAGA AGGTAGCTGT GATTGGAGCT GGGGTCAGTG GCCTAATTTC 2460
   TCTGAAGTGC TGTGTGGATG AGGGACTTGA GCCCACTTGC TTTGAGAGAA CTGAAGATAT 2520
   TGGAGGAGTG TGGAGGTTCA AAGTAAGTGA GATTTTCTTG GGTCTTGAAC AGGTTGTGTT 2580
   GTTATTTCAG GGTGAATCAC AGTTACTGAT GGGTCATATT GAGAAATTTA TTAAACAACT 2640
   CTGATCAGAT TTTATTTCTA TTTATTGATG TGGCCATAAT GGAACTGAAG TCATAGGCTG 2700
   GCATCTCTCC CCCAGTCAAT ACTAACCCAA CCCAGGTAGC TGACCCAGGC ATGTAAAAGA 2760
   TCTCTTCTTT TGGATTCAGC AATTGTCTTA CAGCCCATAC TTCTGTCATT CTTTAATACG 2820
   CTAATATTAG AGAACATTTT ACAAAAATAG AAGTAACAGG GATTCTTCTC AAGATATCAC 2880
   TTCTGTTTCA ATTATTAAAC CAAATGCTTC TTTAGAGACC ATGCTCTTAT CATTACTATT 2940
   TTTCTCTGAC AAATGAAGCA TGTTTGTTTA CTGAGCTTTA TCAATGACAT TCTAGTATAA 3000
   CTGCTGTGAA ACTCTTTGTT AAATATGTTT TATTAAATTT ATTCTATTAA TCAAACCAAA 3060
   ATATTGATAA TGCTATTTGT CTGTATTAGT CCATTCTCAT GCTGCTATGA AGAAATACTG 3120
   AGACTGGGTG ATTTATAAAG GAAAGAGGTT TAATTGACTC CCAGTTCCAC AATGCTGGGG 3180
   AGGACTCAGG AAATTTACAA TCATGGCAGT GGGAAAGAGA GGTGCTGAGC AAAGGGGGAA 3240
   AAGCCCCTTA TAAAACCATC AGATCTCATT AGAACGCACT CACTATCATG AGAACAGCAT 3300
   GAGGGTAGCT GCCCCCTTGA TTCAATTACC TACCCCCACC AGGTCCTTCC CAAGACATGT 3360
   GGGGATTGTG GGAACTACAA TTCAATATGA GATTTGGATG GGGACACAAA GCTAAACCAT 3420
   GTCACTGTCC TTAAAAATTT GTATAAAACT TAGAAAGTTG CATAGATAGC TATAAGGAGT 3480
   TACAATTATT CCTTCCCACA ACCTCTCAAT AGGTAGTAGC TTACCACCTT CTAGCTGTGA 3540
   GATCTTGAGC AAGTTATTTA CATCCTGTGT TTCAATTTAC TCAGTTATAA ATGGATATAA 3600
   TAACAGGAAA GTGTGATTAT CTCATAGTGC TATTTTGAAG ATTAAGGGAG ATAATTCATA 3660
   TAAAGAACTT AGATAAGTTC CGGACTCATA GAGTTCAATA AATGTTAGCT ACTAATAATA 3720
   ACTATATATT TTATAGATGA GCAAACTGAA AGTGAGGGAG GTTAAGTGAG ATGGCCAGGG 3780
   CCACACAACT GGAGGAACTG GGCTTCAAAC CACGGCCTAC GTGACTTCTA AACAGATAAG 3840
   CCCTGACTTA CAACCATGCC CTAACTTGCA TTCTTGCTCA AAAAGATTAA ACAAAAGTTT 3900
   AAGTTCAGAA CCCAAAAGCA ATGACTTTAG AATTATGTAA TCAGGTATCC CTGAGATATT 3960
   AAAACACATA AGAATATTCC AAATGGGAGC AAAAGGTTTG AATACATGAA AATCAAACTC 4020
   ATATCAGCAG AGACCATATA AAGGGCTCTC ACTGCAGGCT GACTAGTTAG GAGGATGGCA 4080
   AGGTGATCCA GGACCTGCGC ATGCTTTGTC AGTTCAAATT GAATCTCATG CCAACAGCGA 4140
   TCTTTTTTAA CATGTAACAT TAGGTGTCTC AGGTACACAT GACCATAAAC CACACCTGGA 4200
   GGGTTTCTTT TATTTTCTTT TTAATATTTT TCTGAGACAG GGTCCTACTC TGTCACCCAG 4260
   GCTACCATGC CCAGCCATGG AGAGTTTCTT AAAGATACTG ATTCCTTTGG TTAAAGCTGC 4320
   CACCAAAAAA AAAAAAAAAA AAAAAAAAAA ATACTGATTT GTGGGCACTC CATGCCAAAT 4380
   CTATGGAATC AAAATCTTCT GGGGGTTTTT AATAAACATC TCAAATGAAT GCTATGATAA 4440
   GACAAATTTG GTAATTGTTA CACAAACACC TAATTTAAAA ATCTGATCAT TCTACTATCT 4500
   AAACACACTC AGAGTTAATG AGGGAGAAGG GAGAAATTGA TTCTTCTGTA AGACAGGTAG 4560
   CTTTGCAAAA AGGAAAACAG CTTAAATCAC ATTCATTTCT TATTAAAAGC TGATGATTAA 4620
   TATCATTTTA GTTTTTCCTG GGATGGTGAT ATAATATGGT GGTCATTCCT GTCTTAACCA 4680
   AAGATATTTT TGTCCACTCT AGGTTCACAT GTAGATTTCA GCTGGAATTT TTTTTTTTTT 4740
   TTTTTTTTGC TCCCAGGTAG ATTCTTAACC TAAACAAGAA ATGTAGAAAT TACAGTTGGT 4800
   CCTTGGTATA TGCAGGAGAT TGGCTCCACA ACCTCCCTCC CCCAGTATAC CAAAATCCTT 4860
   GCATACTCAC ATCCCACAGA TTTATTGTCA GCAAAAGAGA TGAGAGTTAG TTTGAACAGT 4920
   CTGCCAACAA TATGATTTGA TGAATTCTAG GAAGGTATTT TCTGCAGTAA AATATTTCTC 4980
   CAACTATCCT TTTGCCAGTA TCTAAAATTT CAGATTAGAG ATAACTTCCT ATTCACTAGA 5040
   AAAACTGGAT TAAAACCTGA TTAATTAGGC TTTATTGAAT ATTAAGGGTT AAGTATATAA 5100
   CTGTGGAACT TGTAACAGTA TCACATTTCA AATTTCTCTT AAAACTATAT CCAATAGAGG 5160
   AATGTAAACT ATTGTCTCCA CTCAACGAAG TCAAAGAGTC CAAAGAGTCT CCCTGCAGAG 5220
   TGAAACATAA AATAAGCAAA ATTTCATAGG CTGCCTGCAC TACGGCTATG TGAGGGTTTT 5280
   GGTTACCAGG TGACTGGGAG TTTCCAAGAA GGATGCTGGG AGCCCCATGC TCTTCCCTGG 5340
   GAAACTTTGC CTTTTCACTA CTCTACCATC CAGAAGCAAT TTTTTAAATG GGTTATTTAT 5400
   TAATTTTCGT ATTTACACAA CTCCTACTGA GATTACTTAA CATATTTGGT GGTGACAAGT 5460
   TAACAATAAA TAAGTAAATT TAAGAATCCT TGTCCTATAC CCAACCCAGA CAATAGAGTT 5520
   CTTCCAGACT CTCCAGCACC CCCTAGTGGC ACATATGGAC CATGGGACGG GTAGGTAATT 5580
   AGCATATATT TTTCGTTCTG TTTCCAGCAA CGGGAAGCAC TTGGCAAGCA TCACCTTCTT 5640
   TTCTTCGCAA TACTGCTAGG AAGTATGTAT TATGATTATC TTTATTTACA TATTAAGAAG 5700
   AAACAGTTTT CAGATAAAGA ATTTGCTCAG GGGAACATAG GTGGCGGGAG AAAAAAAACG 5760
   AGGGTTTACA ATTTCGGAGC TCTCACACTT AATAACCTTG CTGAAGTATT GATAGAGGAA 5820
   AACATGATCT TCTTTCAGCC GCTAACCTTC TCTGTTTCCT TTATTGTTCC TAATACCTTG 5880
   TATTCACGTG GGAGTTACCA TGTACATTTT TTTTCCTGTG GGTTTTCTTT TAATATTTGG 5940
   ATTTGGATCT CCTCCTTTTC CAGATGTATA TGTTTAGTTA TTTTAATTTT CATGTAATAC 6000
   TCTCTAGACA TATCTCAATC TTGGTTTTCT TCCTCTAAGT TCAATCTGAA ATATCACTTT 6060
   CTCTCTTAAA TTTGGCTCCC CCAAGATCCA ACATTCCAAA CATATTGCCA ATGAGTGTAT 6120
   ACCTTTTAGC TTGAAAGCAG CAGAAAAAAA GTGGTAAATA CCTGAGCCAG GGAACTTAAT 6180
   TAGGGGGTTC TATCAGTGAT CAAGGCCAGT GATCAAGGGA GACACCAGCC TAATGAAAGA 6240
   TGACAGAAGA TAGCAATACT CTAATAGAGA TGTGGTTCAC AAAGTTCATT GTGCAGAAGC 6300
   AGCTAGGGAG AGCTTCTAAA ATACAGAAAT CTGAGCCCGT CTTTTTTCTT TTCTTTTTTT 6360
   TTTTTTTTTT TTTGAGATGG AGTCTCGCTC TGTTGCCCAG GCTGGAGGGC AGTAGCGCAA 6420
   TCTCAGCTCA CTGCAACCTC CATCCCCCGG GTTCAAGCGA TTTAGCTGGG ATTACAGCCT 6480
   TGTGCCACCA CACATCTGGA CCCATCTTCT AATGCAACTG GTCCACTGAC TGGCATTTGG 6540
   GAATTGCAAT TTTGCCTCTA ATTGTAGGAC AAGGAAGTAA GAAGAGTTTT AATCATATTC 6600
   AATTCAAGTA ATGGAGCAGA TAGATGTAAG GTCCATCCGA AAGAGTGAAA TGATAGAATC 6660
   ACAGAATATT CTTAAAGAAA GGCAATTTTA TTCTTTCTAA CTGCTTATGG TAACTACCCA 6720
   TGAAAGCAAA AATATTGATT GGTAAGGGTC AATATAATGA TGTTTCACGA AGAAAAAGTT 6780
   TAATTTGTAA GTTTTTGTAA TTCACATTTA TAATAAATAA ATCTGTTTCT GCTTTATAAA 6840
   TTTCCTCACT TGAGTAGATT AAATATTACC CTTATAATCT TCTTTAAACT TACTGTTTAC 6900
   AACCTTTTTA TTGTCATGAA GTCAAACATA AACTTCAATT CAGCTCGTGA TCAAAAGATC 6960
   ATAAATTCTA AATAAGTGCT ATCTGAATTA ACTTGGTTTG CTAGAGTTTT CTGACATTCT 7020
   GAAAATTCTA TATTAGAAGA ATTCTTTATT ATATGATAAT TTATGTTAAA CAAATTATAG 7080
   CAAATTCTAC ACATAAGGAA ATTCAGACTA TATTTATGCT TAATTATCCA GGCAGTAGTA 7140
   GTACTTAAGT AAATATGTGA GTTAAATTTA TCTGTTTTGA AAACTGTGCC TCTGTCCTCC 7200
   TCTTGATTGA CAATAAACCC TCTGTCTCCA CTTTCACATC TCCAAAGTTC AAGTGCATTT 7260
   TAATACAATA TAACAATAAG CACCATAAAG ATATAAACTA TGTTTGTACT GTTAGCATCT 7320
   TATCCCTAAA TCCAAGCTCA GGCCCTGGTC AGTTCAAGCA TTTGATACAT ACTTGTCTAT 7380
   TAAATCAACA TTAATCATCT CTTCATAACT AGGAAAACTA GGCCAATTTT ACCCAGATTT 7440
   GTCTAAATAC ACAGATGCCT ACTTCAGCAA ACTAAATGTA GAAGGAAGCA CATATGAAGA 7500
   CAAGGGGGTC TTTTTTAGCT GCTATTTACC AATTAACCCA ACAATAAAAG TTTATCACTT 7560
   GGCTGGGCGC GGTGGCTCAC GCCTGTAATC CCAGCACTTT GGGAGGCTGA GGCAGGTGGA 7620
   TCACCTGAGG TCGGGAGTTC GAGACCAGCC TGACCAAAAT GGAGAAACCC CAACTCTACT 7680
   AAAAATACAA AATTAGCCGG GCATGGTGGC GCATACCTGT AATCCCAGCT ACTCAGGAGG 7740
   CTGAGGCAGG AGAATCACTT GAACCCAAGA CGGGGAGGTT GCAGTGAGCC GAGATCATGA 7800
   CATTGCACTC CAGCCTGGGC AACAAGAGCA AAATTCTGTC TCAAAAAAAA AAAAGGGATT 7860
   ATCACTTGAT CTTCAGAAAA ATAGTGAGGT CATTATTGTT TGCTGACAGA CTACACAAGT 7920
   AAAATCTCCC AAAGGCCAGT TTTGCCCTGG CCCTAAGATT ACTGTAGGGC CTCAGACATC 7980
   AAATCAGTTC TTCTCATCAC TCAAAATTCC CTTAAAATTG ACCTGACAGA GAAGCCAACC 8040
   ACATTTTTAA GCCAAATTGT TGGGTCTTTT AAAAACTAGC ATTTTGGCTG TAGTATAACA 8100
   GTCTTAGTTT AACTGATTCA AAACTATGGC TGGCTTAGTA AATTTAACGC TAGTGGCCAA 8160
   TAATAACAGA AAAGAGATAA ATATTCTTAA GTATGTATTT TGAGCCAGGG ATTCTGCTAA 8220
   GTACTTTATT CACTCTCATT AAAGCCTTGA AACAATTGTT GCATGTTTAA GTTATTAATG 8280
   AGCCCCATTT TACAGAGGAA AATGAGGAAA CTGACCTATG TAACTTGCTC ATGGTCACAA 8340
   GCCATTAAAG GTGGCAGAAT TAGGATATCA ATCCAGTCGG TGTGACTCCA GAACCCTCCT 8400
   ATTTACTCTA TACTACTCAT AAAATTATTT GGTCTTGGGG CTGGGCGCAG TGGCTCATAC 8460
   CTGTAATCCC AGCACTTTAG GGGGTTGAGG TGGGTGGATT GCTTGAGCTC AGGAGTTTTA 8520
   GACCTGCCTG GGCAACATGG TAAGACCTCA TCTCTACAAA AAAAAAAAAA AAAAAAATAC 8580
   AAAAAATTAG CCGGTGTAGT GGCACGCGCC TGTAGTCCCA GCTACTTGGG AGGTTGAGGT 8640
   GGGAAGATCA CCTGAGCCCA GGAGGTTGAT GCTGCGGTGA GCCATAATCA TGTCACTGCA 8700
   TTCCAGCTTG GGCCACAGAG TGAGACCCTG TCTCAAAAAT AATAATAATA ATCTGGTCTT 8760
   GAGAAAAAAT AGTATTTTTT TCTTCATAAA ATATTTTCCA TTTTGAGAAC TTGATTAAGA 8820
   AACTCATTGT CTTGCCAATG ACATTACATT CAATCATGCT GAAACATCCA GAAATAGTTT 8880
   ACACATCAGT TTGACATCAG TATTATGCAA TTTGAAGCCA CTGTTTGAAA ATAAAAACAC 8940
   TGTACCGTGA TTTGTTTATC CAGAGTTCAG ATTATTATAT CCTTGTATAT GAGACAGAAA 9000
   CCCCCTTGTA TTCTAGTGCA AACTCTCTTT GGATCTTAAT ATGTATAGTT AACAATAATA 9060
   CCATACTACA TTCTAACTAC CTAGAAAGCT AGCATACCTT AACCTGATTA ACTTTTAGCA 9120
   AGTTACTTGA AATTATAGCA AAGTTACCAT TTAAATCTTG ATTCTGGCCA GGTGCAGTGG 9180
   ATGAACCAAG CATGGTGGTG CCTGTAATCC CAGCACTTTG GGAGGCTGAG GCGGGTGGAT 9240
   CACGAGGTCA GGAGATTGAG ACCATTCTGG TTAACACAGT GAAACCATCT CTACTAAAAA 9300
   ATACACACAA AAAAAATTAG CCGGGCGTGG TGGCAGGCAC CTGTAGTCCC AGCTACTCAG 9360
   GAGGCTGAGG CAGGAGAATG CCGTGGACCT GGGAGGCGGA CTTGCAGTGA GCCAAGATCA 9420
   CGCCACTGCA CTTCAGCCTG GGTGACAGAG CGAGACTCTG TCTCAAAAAA AAAAAAAAGA 9480
   TTTGATTCTA TCAGTCTACT CACCTTTATA GCTTGACAAT GATTGATTTG TGTAAAAGGA 9540
   TTCAAATCAA AATTTGCAAA CTCCCTTCCT CCAAAGGTAC TCATTTTATA ATACTGAAAT 9600
   TCTCTATTAT GTTCTCTGCC CAGTGTCCCA GGGTTTATTG GTTTCTAAAG AGGTAGTGGG 9660
   TATATACAGC CTCCCCAAGG GGAATTTAGG AAGTAAGCTG GTTGTCACAA AGACTGGCAT 9720
   TAAATAGGTA GAGACCTAGG ATGCTAATAT CTTGCAATGT GCCAAAATAA TTGTGCCTGT 9780
   CCCCAACCTC ACCATTGCCA ATATTACCCC TACCCCTCAC AGTGAGCGTC ACAGGCAGGC 9840
   AACAAACTGG TGTCGTCACA GAATGATTGA TGGAACACAT AGACTGCATT CATTACCTAA 9900
   ACATTGTCGT CACACTGCAG CAACCAAAGA CAATCGCATT ACCCAGGGGT TAGATGTAGG 9960
   AAGAGTAAAA AACAAAAAAT TTTTGAATGC GTAATTATCA CTAATTATTT TATTTGATGC 10020
   TTCAGGAGAA TGTGGAAGAT GGCCGAGCAA GTATCTATCA ATCTGTCGTT ACCAACACCA 10080
   GCAAAGAAAT GTCCTGTTTC AGTGACTTTC CAATGCCTGA AGATTTTCCA AACTTCCTGC 10140
   ATAATTCTAA ACTTCTGGAA TATTTCAGGA TTTTTGCTAA AAAATTTGAT CTGCTAAAAT 10200
   ATATTCAGTT CCAGGTATTG TATTTTTGGG GAAATGGGTT TCTCTGCATT AGTTCAGCTC 10260
   ATATTTAGAT AGAAAAGTTA CTCTGATAAT GAAAGCAATT ATGAATGAAG TATCCCATTC 10320
   TAAGTATTTG TTGAAATATA ACAGCCTCAT ATAAAACCCA AAAAGTAGTG TCATTACCCT 10380
   TGGTATTATA GATTATATAC ATTAATTGAA GAGGAAAATC ATCTGTTAAA ATTAAAGGTT 10440
   TGAATAATAA TATATTGATG TCAAAACTTT TTTTTTTTTT TTTCTCCCTG AGACAGAGTC 10500
   TCACTCTGTT GCTCAGGCTG GAGTGCAGTG GCATGATCTC AGCTCACTGC AACCTCTGCC 10560
   TTCCAGGCTC AAGTGATTCT CCTGCCTCGG CCTCCAGAGT AGCTGGGATT ACAGGCACAC 10620
   ATCACCATGC CTGGTTCATT TTTGTATTTT TGGTAGGGAC GAGGTTTCAC CATTTGGGCC 10680
   AGGCTGGTCT CGAACTCCTG ACCTCAAGTG ATCCACCCGC CTCGGCCCCC CAAAGTGCTG 10740
   GGATTACAGG TGTGAACCAC CACACCCAGC CTCAAAAATT CATTTAAACT AATATCTGTT 10800
   ATCATTGAAT ACACCTAGCT TCATTTGCCT TGAAAGGGCG TATACCAAAA TTAAATTGCT 10860
   GTTTTGTTTT CTTAGCTTCT TCATAGAAAT GGGATTTCTT AGATGTGTAT TAAATAAATT 10920
   CATTGGTCTC TGTTCATACT AGAAGGCTGT GGGAAGTATT TGCTTATCAT TTTTTTCTGA 10980
   ATGCAATCTC TTACAACCTA AAGATGGCCA GATCATTTTG AAAAACACTT GGAATTACCT 11040
   TTTCCTGTGC TTCCTCAAAA TCAACAAAAA GCAATATTTT AATTAAGCAT GCTGAATTTT 11100
   TATCAATGGT CTATACTTTG AGAAATAGCT ACTATGCTTA GAAAATAAAA TATAAATCAC 11160
   ATTTCTTGGC CAGGTATGGT GATTCATGTT TGTAATCCCA GCACTTTGGG AGGCTGAGGC 11220
   AGGAAGATCA CTTGAACCCA AGAGTCTGAG ACCAACCTGG GCAATACAGT GAAAATCTGT 11280
   CTCTACAAAA AATTTTTAAA AGATTATCCA GGCATGTTGA TACCCACCTG TGGTGCCAGC 11340
   TATTCTAGAC TGAGAAGGGA GGATCGCTTG AGCCTGGGAG GTCAAAGCTG CAATAAGTGG 11400
   TGATTGTGCC ACTGCACTCC AGCCTGGGCA ACAGTGTGAG ACCCTGTCTC AAAGTAAATA 11460
   ACTAACATTT CTGGATAAAT AACTGTTAGT GAGGCTTATT TTTAATACAT GTCATTTTCT 11520
   TAGTAATTCT AATACTAGGC TTATATAATA TCAACTTACA ATAGTAAATT TTGGTGAAAA 11580
   TTTGTATTTA TAAATTCCAT TAAAATGTCC AGTTCTACCT AATGTAGTTT TTCACCAATT 11640
   CCTGGTAGAT CTAACTTGTG AATAACAGAT TATGTATACC AGAAGGTTTT GTAACTTTGT 11700
   GCACTTAACT ATCAATCTAC TTAACAAATA TATTGCCTTT TTATGATATA TAACTTCTAT 11760
   TCCATTCTTT TAAAGATCAT GTTAGAGTCG CAAGGAAGTC ATTTCTCTTG GTTATTGTGT 11820
   TACTGCTACT TTTGTTTCTT GGAGAGTGAA GAGGGGTTGG GAAGAAAGGT TTCTGTTTAT 11880
   TGGTCTCTGA GTTGGTGTAA GTCATAGGTG TTAGAGCTCA ACTCGAGAAG CAGGCAAACT 11940
   GTAACAAGCC CTGTTGCTTA TGATTGTCAA TGTAATCTAC ATCAGTGCTT CTCAAACTTT 12000
   AATGTGGACA TGAATCACCT GGATATCTTG TTAAAAATGT AGGTTCTAAT TTAATAGGTA 12060
   TGGGGTAAGT TCTGAAATTC TGCATTTCTG ACAAGCTTCC AAGTGATACT GAAGATCCTG 12120
   ATCCTCAAAT CACATTTTGA ATAGCAAGGA TCTACAGCAC TTAGTTAATA TACTACTTTG 12180
   AACTACCATC TGAAATCTTT TCTTTCATCT GAAAACTGCC CAGATATTTA AAGCCCTTTT 12240
   ACAAGATTTC TACTAATATT CCATATACAT TTTTAAATTG AGACAGCTTA AAAATTACCA 12300
   ACCCAGCAGT TGGAAAAATA TCTGAAAATT TGAGATATAT AAAAGACTAA AATACTTGCA 12360
   AATGAGAAGC ATGCCATTCC TCTAGCATTA TAAACTTTGC TTCCACTTGA CATCGTTTCT 12420
   TAATCCAGCA GATATGAAAC ATTTATGTAC AATTTTAAAA ATTAACAGAC CTCCAGTGAG 12480
   CTACATTTAA AAAAATCAAT GAACCAATAA ATCATTTTAT TCAAATAAGA TCATGAACTG 12540
   TCTTGCTCAC ATGATGTACT CTGTTTTAAA AATAGCAAAT GTTAAAAACT ATCATTCAGT 12600
   GGAATGCTGA CCATGTGTCA GGCACTCTGC AAAGTGTTTT GCGTGAAATA TCTTCTCTAA 12660
   TACAAAGTCC ACAAAGAGGC GGCTACATAA AACGTTCCTG ACATATGCCA ATTGCATGAT 12720
   CACTTGAATT ATTGGTTTGT TTCCTTGTTC AGATTATCAA ATAACAAACA GAGAGAAGTT 12780
   CTTTAAAAGA AAAGATATAT ATTTGGTGAT AGAGCATTGT AATGAGAATG TACATGCCAT 12840
   GGTAAACTAT TTGTGTATTC AGGGAGTTAA AGGAAGACAA AGGTTTTTAA ATGGGGAAAA 12900
   AATACAATTA CATAATTGTT TTGAAATAAT TATATAAAGA GCAATAACAA GGGTGATGCC 12960
   AGTCTGAGAT TGGACAGTTA CTGAGCAGAT GTTCTTGTAG AAGTCATTTT TGTGTAAGAT 13020
   TATGATGGTC TTTGTGTAAG GTGGTGGTTT TTGTAGTTTT TGTTATCAGG CACACATCAT 13080
   GAGAACCCGC TCTTTCTGGC CTTTCCCAAT TCTATTTGTC GGGTTTCTTA ACATTAGTGA 13140
   CTCCATCTAG ATTCTGACAG TTTTCATGAG AACTTGCTTT TCTTTTCTCT CTCAAGTCCT 13200
   TATTCAGTAT TCAGCACCCT TAACAGATTA GTCCCACTGC TGAGTCAGGC CTCTTGCATG 13260
   AAGCAGCAAT GAGAAAGACA CACTTGGCCA ATGTTATCCT GGAGTAATTC TCAATGATGC 13320
   CTTCTCTGTG TTTCTTCAAG ACAACTGTCC TTAGTGTGAG AAAATGTCCA GATTTCTCAT 13380
   CCTCTGGCCA ATGGAAGGTT GTCACTCAGA GCAACGGCAA GGAGCAGAGT GCTGTCTTTG 13440
   ACGCAGTTAT GGTTTGCAGT GGCCACCACA TTCTACCTCA TATCCCACTG AAGTCATTTC 13500
   CAGGTGAGAC CCGCTGGGAT TCCCAGCTTT TTGGAGTAGG TTTCCAGGTA CTTTATATGT 13560
   AGTTTGGATT GACAAGCAGG ATTCATTGCT GCAACTGGGC AGAACTTGGC TCAATAAGAT 13620
   TGAGACAGAG CTAGAAAGAT GAAAGACACC AAACATCATC TTTGTTTCTA TTGGCCTCTG 13680
   AGTCTTCATC ACACATAGAT CTCAGAGCCA ACTTCCTTGG AAGTCACTAA GTCCTTGGCA 13740
   TAATTTTAGA GAATTCACAT CAAACTGGTT CTCTGTTGGA GAGGCCCTTT TAGCCATGTG 13800
   CCTGCGTTGG CCTTTTTCTA CCCTGCCAAA CACCGAGCCT TTTTCACAGG GCCATACTCA 13860
   CACACAAGGG GAGAGCTCCT AGAAAGAAAT GCTTTGCAAG TTAGTGATGG GGAGAGAAGT 13920
   GCAGGAATAG AACCCTGCAT CCAGCTGTTC TGGTCCACCC AAGTCTTTCC TCAGAGAACA 13980
   CACTTCTTTC CCAAGGCCCT TAGGAAAATA TGTAATATAG TGGTTCATAG TCCAGGCCTC 14040
   ATATTAGAAT CACCTGGGGA GCTTCTAAAG CCCTGATGGC CTGGAGACCT ACCCCCAAAG 14100
   ATTCAAACAC TATGGAGTAG GGTTAGAGCA ATGAAAGTTT GCTCAGGTGA TTTTAATATA 14160
   CAGTCAGGAT TAAGGCCTGC TCATCTAAAG CAATTGTTCT CAAATAGAGT CACCTGGAGG 14220
   GCTTTTGAAA GCACAAATTG CTAGGCCCCA CCCTCCATAT TTCTGATTCA ATAGGTGCTA 14280
   TGGCTTGAAT GTCCTGTCCA AAACTCATAT TGAGATTAAT CCCCAATGGG GCAGTATGAA 14340
   GAGGTGGGGC CTTTAAGAGG TGATTGAGTA GTAAGAGCTC TGCCCTCAAG AATGGATTAA 14400
   GCCATTTGTG GATAAATAGG TTAATGGATT ATTGGGTTAC ACAGGAGTGG AACTGGTGGC 14460
   TTTATAAGAA GAGGAAGAGA GACCTGAGCT AGCATGTTAG CATGCTTGGC TCCCTCACCA 14520
   TACAATGCCC TATGCTGCCT TGGGACTCTT CAGAGTCCAA ACCAGCAAGA AGGCTTTCAG 14580
   CAGACGCAGC CCTTCAACCT TGACTTCTCA GCCTCCACAA TTGTGTGCCA GAAGAAATAA 14640
   CTTCCTTCCC CTATAAAATA TTCGGTTTCA GATATTTTGT TAAAAACAAT AGAAGACAAA 14700
   TTAAGACAGT AGCTCTGGCA TGAGGCTGAG AATTTGCATT TCTAACACCA GGCAATGCTG 14760
   ATATTGCTGG CCATGTGACC ACACTTTGAG AACCAATAAT CTAAAGATTC TTTCAAGCAA 14820
   CCCCACCATC AATGGCAAAT ACTTTATAAA GTCATGTGTT TCCGTGAAGT GTAAAAGTAG 14880
   TAACTAGGAA AGGACACAGA AGAAGCTTGT CTGTGATTAA CCACCAGCAA GTCACTGATT 14940
   TACACAATAT GGAAACCAAC TCCTATGTGC CTGGTTTTTA GTTTTAGTTT TTGTTTACTT 15000
   TTTGAAAATA AGATTGCTAA ATTGTATTCT AACTATTACA CAATTATAAT AATAGCACTT 15060
   CATAATGTGC TTAAGAAATA TTTAAGAGTA TCTGATAAGT GATTTTTTTT TTTTTTGAGA 15120
   TGGAGTCTCA CTGTCACCCA GACTGGAGTG CAGTGGCACG ATCTCAGCTC ACTGCAACCT 15180
   CCACAACCTC CATCTTCCAG GTTCAAGCAA TTCTCCTGCC TCAGCCTCCC AAGTAGCTGG 15240
   GATTACAAGT GCACGACCAC CCCTGGCTAA TTTTTGTATT TTTAGTAGAG AGAGCTTCAT 15300
   CATGTTGGCC AGGCTGGTTT CAAATTCCTG ACCTCAGTTG ATCCGCCTGC CTTGGCCTCC 15360
   CAAAGTGCTG GGATTACAGG TGTGAGCCAC CACACCTTGC CTAATATGTG ATATTAAAGG 15420
   GTCAAATGTC ATTATATAGT CCAAAATAGT ATATAATAGG CAGGCAGAAG ACAGTATCTG 15480
   GTCCTGCTGT GTTCATCACC ATTTATTTGT CTCTGATAGA GACAAACTGC AGCCGTAAGC 15540
   TGCAGCCTCT GAAATAAAAA ATCAACCCCT TTGGTCCTGT TTTTTTGTTT GTTTTTTGTT 15600
   TTGTTTTGGT GTTGTGACAG TCTCACTCTG TCACCCAGAC TGGAGTGCAG TGACTCAATC 15660
   AGGGGTCACT GCATTCTTTA CTTCCCAAGC TCAAGCAATC TTCCCACCTC AGTCACCCGA 15720
   GTAGCTGGGA CCACAGGCAT GCACAACCAT GCCCAGCTAA TTTTTGTATT TTTTGTAGAT 15780
   ACAGGGTTTC ACTATGCTGC TCAGGCTGGT CTCAAACTCC TGGGCTCAAT CAACCTGCCT 15840
   AGGCCTCCCA AAGCGCTGGG ATTACAGGCC CCACCTGGTC TGGTACCTAA ACTTTCTTAT 15900
   GTGCTTTACT CCTATAGAGA AGAGGCAAAA CAATTATTAA CTCCAGAAAG GAAAAGCTGG 15960
   CAATGCAGTT TTATTGAAAT TAGCTTGACA TAGTTGCTCT GGAGCTCACA GACTTCTCTC 16020
   TTCTTCCCCC TGAAGGTATG GAGAGGTTCA AAGGCCAATA TTTCCATAGC CGCCAATACA 16080
   AGCATCCAGA TGGATCTGAG GGAAAACGCA TCCTGGTGAT TGGAATGGGA AACTGGGGCT 16140
   CAGATATTGC TGTTGAGCTG AGTAAGAATG CTGCTCAGGT GTGATGCTCT CTGCTTACCA 16200
   TGTACCTGGA GGGGAGGAAG TGGGGATGCC ATACTGGAGA ACCCCAGCCA TATAATCGCG 16260
   GCTCCAATCC TCATTAACTA GTTGGTTGGT AGCGCATTGT GGCATCATAG AAAATCTGGA 16320
   AGTCAAGAAA CCACTTTACC TCCTAGCTCT GTCACTAACC AGCCATGAAT CCTAGAGTGA 16380
   TTCATTTCAC TTCTCTGGGA GATGGCTCCC TCATTTTTAA AATGGGAACT TTTGACCAGA 16440
   TGATTTTCCA TATAAGAGGC CTTTCATCAA CATGGCTCAC TGCAGCCTTG ACCTCCTGGG 16500
   CTCCAATCTT CCTGTCATCT CAGCCTCCTG AGTAGCTGGG ACTACAGGCA CATGCCACAC 16560
   CACACTCAGC TAATTTTCAT ATATTTGTAG AGATGAGGGT CTTGCCATGT TGCCCAGGGT 16620
   AGTCTAAAAC TCCTGAACTC AAGCAATCTG CCCGCCTCAG CCTCCCAAAG TGCTGGGATT 16680
   ACAGGCATGC ACAACCACAC CCAGCCAAGA GGCCTTGTTT CTACCTGGAT GTTTAATGAG 16740
   AGGTTAATCT GTTCATATTC TGGAGGGTGG CTTTTAGAAA TTTAGTGTGT ATTTGAATTA 16800
   TATTTGAAAT ATAGATAACC TTCAGTTACC CAAATATTAT GAAAAGAAAG ATTAAATAGA 16860
   TAGTAGGTCT CTCAACTAAA ATCATAGATA TTTAGGTGCT TCCTGAGGCC TTCTAACCAC 16920
   TGTCTTCTTT GCACCTGCTC AGGAATGACA CCAGCTGAGC TGCCAAAGAG TCAAACATTC 16980
   ATTACATGAT GATGCTGCTG ACAGTGGTGG TCAGGAATAG CAAAAACTAA ACTCCTTCTG 17040
   CAAGGACAGA CCTAGGCAAA GAAGGGAAAA TCACTAAACA TCCTTTCCCA AAGTATTGCC 17100
   TCTCAAGAAG GCCTGAACCA GATGCCCAAT CACTCTTACC CTAGCTCTTT CAGCCTGATG 17160
   TCTCTGGCCA CCCAGGGCTT ACCATGGCCC TGTGCACAAC CAACAAATCA TTTCCATGCT 17220
   AAGTCTTACA CTTTCAGGAC TCTAGATACC CAGTGGCAAA AGTTACAAGC AAACATGACA 17280
   CCCGCCCAGC AGGTTAATGA AGGGGTTATA CTGGGACCTG TCAGAGTCAT CTATCAGTCA 17340
   GTTAGTTAGT GCCAGCCCGG GAACAGAGCA GGTCACTAAC ACCGGAAAGA GACTTACTAG 17400
   ACCCAATAAG TCTTCACTTT GTGAAAATAA ACCTCTTGTC ACTTATCACC TCAGTGTGAA 17460
   GAACAAGTGA GGAGGCAGGA ACTGTGACAG CCTGGAGAAG AGCAGAGCTG GAAAATGAGA 17520
   GTACCAGCTC TAGGCTCTTT CATGCTACGA ATACCCGCAA AGCCTTAGGA ACAGAGTGTA 17580
   ATGGGGCAGT ATGTGAGGAG CTAATATAGC AGTCAGCCAA GTGAAGATCC ATCCTAGACT 17640
   ACTTCACGTT GTCAGACCAG TGATTTGGAT TTAGATCTCT TCATTCCAAA GATATCAAAT 17700
   CTTAGATGGC AAGAACCAGT TCCTTGTATG GGTCTTGCCC TACAGGAAGA CTTATGGTGT 17760
   GAGATTCAAT ATTAAGAAAC TACCTTGGCT CTATTTGCAT GCCTTACAGC TTCTTAAACA 17820
   ATCTTTTGCA CAGAGTGCAA AAGACTTTGT TTCCATCTCC CTCTATCAGT GTAAATGCCA 17880
   CTAGATGCCC CCTTTTTAGG AGGTACTTCA CTTTGAGGTC AATCATCTTT AAAACAGAGC 17940
   CTCAGTAAAT TCTGGGGCTA TGCATGTGAT ACATCAGCTA CATAATAGAT TCCTCCTAAA 18000
   TATAATGTTA TAATCATACA TTTCCAGGAT TATACTCATT CATCTGCACT AATCTCTTCA 18060
   ATATTTATTA GAGTAACAAC ATAAATCTAT AACTATGATA AAAGCTCTTA CACAGAGTAA 18120
   TATACTCTCA AGCCTTCTGT GAAAAGACTA ACCAGAGACT TTACAGGAGC TATACATGCT 18180
   AGGAACGGAA CTAGGCGCAT CTGCAAAACT TGAAATTACA ACCTGAACTC ACCAAAATTC 18240
   TGAGTGTGCA CTGCTCTGTT AAAAGAAATT CACCTTCATA AGGTTACAGC ACCCTCTACC 18300
   ACAATCCAAA AGCACCACTC AAGATCATAT GGGATGGTGC TGCATCATTG TATTAGTCCA 18360
   TTCTCAACGC TGCTATGTAG ACATACCCGA GACTGGGTAA TTCATAAAGA AAAGAGGTTT 18420
   AATTGACTTA CAGTTTGGCA TGGCTGGGGA AGCCTCAGGA AACTAACAAT CATGACGGAA 18480
   TGCACCTCTT TACGAGGCTG CAGGAGAAAG AATGAGAGCG ACTGGGGAAC CCCTTATAAA 18540
   ACCATCAGAT CTCGTGAGAA CTTACTCCCT ATTAGGAGAA CAGCATGGCA GAAACCTCCC 18600
   CCATGATTGA ATTATCTCCA CCTGGTCCTG CCCTTGACAC GTGGGGATTA TTATAATTTA 18660
   AGGTGAGATG TGGGTAGGGA CACACAGCCA AACCATATTA GTCATTTACA TACTTCTGAC 18720
   CAAAAACCAA ATCTCTGGCC TTTGACCTAA AACATGCGTC TCAGAGAAAG CAGCCTGAGC 18780
   CTAAATCCTC ATGTTTCTCT CACTGTTGCA GCTAGTGTCA TTAAGGCAGG TTAGACCACC 18840
   CTGCTGTAGG GAGGGTCACA ACAGAAAAAG AGTGAATCAA ACGGGCAGAG CATACCATTT 18900
   GAAACATGGT TTGCTCCTGA GAAAGAAGAG GGGACAGTAA GTAATGGAAA GAGACACTAA 18960
   TGAAAATATT TTTGTATCTA ATATCTAATC AAAGTATTGC CAAGTCAGCC TATAAGGGCA 19020
   ACGGCAGGAG AAATTCAGAA CATAGGTATA TACCACACAC AGACCAGCAA TATAGGAATG 19080
   CTTGGTATAG GTGCTACTTC ACAAGCTAGG AATGTAAGGC CCATCCCCAC AAAATTTGTC 19140
   TCCAAATTCT GGTTTACTCC AGACATAAGG CACTGTATGA AACTCCTCTC TTCCAGCCTA 19200
   ACTTTATAAC TTAACAGCTA GCAGTACTTA TCACTTGCCA GGCAATATTT CAAGTACTTT 19260
   ATATATACCA CCTCATTTAA TCTACACAAG AATGCCATGA GGTAGGTACT GTTAATACCC 19320
   CCATTTTACA GAGAGAGAAA CTGAGGCACA GAGAGATTGA AATAATTCAA CCATGGCAAC 19380
   ACAGATTGAA ATAGTTCACC CACAGTAGTG TGATTGGGAT TCAAACCCAA GCAGTCTGTA 19440
   TCCAAACCTC TCAAGTAAAT TGGTTACCTT GCAAGTGAAT CTTATGTGTT TATCAAGTAT 19500
   AGCCTTAAAC AAAAACTTAT TGCATGGTAT GTAAAAATTT AAGAAGCAGT TCAAGTATGC 19560
   ATTTGGCCAA TGGGGGAGTA ACAGCAAACA CAGCAAAATA TACATTTGAA AAGAGATTAA 19620
   ATGTACATTT TGGAAACAAG GGAAATCTTA ATAAACAAGG TAAAGAATAC ACCTGAAAGA 19680
   GGATTCAGAT GTGCACTTGA AGAGAAAGAG AATCACAGTA TAAGTTCAGA GTTTTTAACT 19740
   TTTAAAATAC ATTACAAGCA CTGTGTCTCA TGCCTGTAAT CCCAGCACTT TGGGAGGCTG 19800
   CGGCAGGAGG ATTGCTTAAG CCCAGAAATT TGAGACCGAC CTGGGCAACA TAATGAGACC 19860
   GTCTCTACAA AAAAATTGTT TGAATTAGCT GGATGTGGTG GTACATGTCT GATACTGAGG 19920
   TGGGAGGATC ACTTGAGCCT GGGAGGTCGA GACTGCAATG AGCTATGACT GCACAACTGC 19980
   AGTCCAGCCT GAGTGACAGA GCAAGACCCT GTCTCACACA CACACACACA CACACACACA 20040
   CACACACACA CAAAATAAAG TCTTTTAAGT ATGGAAGGAA GATTATTTCC CCTGTTATTC 20100
   TCCATCCAGG GATATTCAGA TGCATATACA CTTATACTTG TGTAGTCACT AGGCTATAAT 20160
   CGCACATTTC CAAGGATTAT AATCATTCTA CCTGCACTAT AGAAGAAACT TAGGTGAGTG 20220
   GAAAACATGA GAGGAGGGAG GGAGGAACTT TCTCTTAAGG AGCAGCAAAC CACAACTGTA 20280
   AACATGGGAA AGACTTGTGG ATTTTATCAT CAGAGTTAGC CCAAAGACTT TCTCGTGTCT 20340
   CCATGAAGTT CTCAAGATTT TGTTGCAGTC TTCCTGCATC AGTGTAAATG CCACTGGGTA 20400
   CCCCTATTTA GGAGGTACTT TACATTGAGG TCAATCATCT TTAAAACAGA ACCTCTGTAA 20460
   ATTCTGGGGC TACACATGTG ATACATGACC TTCATAGTAG ATTCCTCCTA AACGGGACAA 20520
   TGCCCTAATT TAAACTGCAT TTCTTTTTGC TTGCCAGGTT TTTATCAGCA CCAGGCATGG 20580
   CACCTGGGTC ATGAGCCGTA TCTCTGAAGA TGGCTATCCT TGGGACTCAG TGTTCCACAC 20640
   CCGGTTTCGT TCTATGCTCC GCAATGTACT GCCACGAACA GCTGTAAAAT GGATGATAGA 20700
   ACAACAGATG AATCGGTGGT TCAACCATGA AAATTATGGC CTTGAGCCTC AAAACAAGTA 20760
   GAGTTATTTT GCTTTTTTAA TGGTATACTC GTTGGTGAGC AAAGTTGTCT GAAGGTGTCT 20820
   CCCTTAACAA AGATTCAAAT TGCTAACACG GTAGTTAAAA CTACAATCTA ACAATATGAG 20880
   TATCTTATAG GTCCTGGAGT TTAGCTTCTA AATTTGGTCT GTATGCCTTT AAAAAATACT 20940
   TAAGAAGATG AAGCAGAAGT GTTATAAGCT GCTCCAGAAA GCAAAACTAG GGGAGAACTT 21000
   TCTAATACCC AGAGTTATCT AACATTGGAG AAAACTGTTT CAAGAGATTA CGACCTGCCT 21060
   TTCAGAGGGG TGTGGTGGGA AACATGTAAT TCTCCATCTA ATAATTTATG CTTTGCTAAC 21120
   CCTATAGCAT GAAGGTTCTT CCCATGGGAA ACCTTTGAAA ACACATTCCT TTTTCTTTGC 21180
   TAAAAGACAA ATCTCTGTTG ACGTCAAAGT TATATGTCAG TGATTTAAGC ACAAGCAAAT 21240
   GTTATGAATG GTTCTTTTGC TTTAGTTGTT ACAGGCTTCT TCCCTTAAAA AAACAGAAGA 21300
   GCTTTAGAAT CTTTTAACAA ATGCCTGCCG TGCAACTACC ATATTCTAAG ATCTGACATA 21360
   AGTGCCACGT ATCGTCTATT AAAAAAAGAA AAAGAAAATG TTCTCAAATC TACAAAAAAA 21420
   ATAAGCGGAC TTTGCATCAA CATCCATGCT ATTACTAACA GAGACTCCAT GGATATTTGG 21480
   GATTAACAAA TATCACCAAA CCTAATTTTA TACATTAATT TTCACATTGA TCCCTTCATA 21540
   GATTTCAAAA CTAGTGGAAA TTTAGCAAAT TTTTTCTTAT GATCAAATAG GGGTTAAATA 21600
   AAACAGCAAA ATAATAAAAG CTAGATAGCA TGAAAAAGGT TAAAAACAGA AATGGTATAA 21660
   TAACCACCAT AATACTTGGG GATTGACCAT AGGCACAGGC ATTTTGTCTA AGCCCTTGGG 21720
   GATGCTTCCT TCCTTAAAAT CTCTTTCACT CACGTTGCCT ACATGTTTTC CCTTATTTAT 21780
   TGACAAGAGA TATTTGTGAC ATGAGAATTA AGTCAGAAAA TAAGGATTTG CACAGACAAC 21840
   CAGTTAAGTT AGAGTTTTAC AGATATTTGA AAAGCCCTTT TATTTTCAGA GCCGTACCCC 21900
   AAAAATATCA AGAGGGTTCA AGATTCCTCA GCAAATGATC CTTCAGAATG TTTTTCTTCT 21960
   GTATGTCTCA GATACATTAT GAAGGAACCT GTACTAAATG ATGATGTCCC AAGTCGTCTA 22020
   CTCTGTGGAG CCATCAAGGT GAAATCTACA GTGAAAGAGC TCACAGAAAC TTCTGCCATC 22080
   TTTGAGGATG GAACAGTGGA GGAGAACATT GATGTCATCA TTTTTGCAAC AGGATATAGT 22140
   TTCTCTTTTC CCTTCCTTGA AGATTCACTC GTTAAAGTAG AGAATAATAT GGTCTCACTG 22200
   TATAAATACA TATTCCCCGC TCACCTGGAC AAGTCAACCC TCGCGTGCAT TGGTCTCATC 22260
   CAGCCCCTAG GTTCCATTTT CCCAACTGCT GAACTTCAAG CTCGTTGGGT GACAAGAGTT 22320
   TTCAAAGGTA AGTGTGTAGG CAGGTGAGTG GCTAAGCGTT TCAGATCTGG TGAAGTTTAT 22380
   CAATAATGAT AAGAAGGTTG CCTGAGATAA AAAGGTTGCC AAGAAAAAGT TTGACAACCT 22440
   TGGCTGCTCT CACAAGACTA ACATTCTAAA AAGTTACTGG AGAATTCAAA GAATAACAAA 22500
   TACAGGAATT TAGTAATAAT AAATACCTGC AATCATCCTT TTAAAATATT AGACAGTCAA 22560
   GAGAATTTCA ACTGGCATAA AGCTAAGTGC ATGTTAACTT TTCTTTGAAT CGTGAGAGAT 22620
   AAGTTTAAGA AAAAGATCTG TCTCCTGGTT TTACCTCTGT GTTGTTTAAA AATTCCTCAG 22680
   CATATCTGCA AATCAATTTA ACTCTTAATA CTTGAGCAGC TCAACCTCAC AAATCCCTAC 22740
   AAGTTATAAA ATTATTAAAA GGTTTCTTTC TGGGTGTCTG TGTAGCACTT CATACTCCTC 22800
   AGAACGGTGT TACCTCCCTG CCTCCAGGGT TCAATTCTGT TCAGCAAAAG CTTACTGAAT 22860
   ACCTTGCCCT GTGCTGGGAA CTGGTGGGAC AGAGAGAAAT TTAAACAGAT CATTTCAACA 22920
   TAACATGACA AATGCTTTGA TTGAATAATA TATGGAGTGT TCAGGGAAGG AGAGAAAGGG 22980
   CACTTATCAT GGTAGAATAA GGGAAGGGCA CATGATAAAG GAAAACGTCC TGGATAACTG 23040
   CATTTCTCAG GGGCAGAAAA GGGGATTGCC TGAACAAAAG CATAGAGTCA ATGATGCATA 23100
   TGGAAGGGCA CATGCTATTT GACATTGCTA GAGCATGACG TATGAGGCAG AGAGAGATGA 23160
   GCCATTACTC TTGGAGAAGA AGGAGACAGG ACACAGGAAT TTTTTAAGAC ATGCTATGGA 23220
   GCTTAGATTA TAAATTATAG ATCAGTTCTT CCCAAATATG GCTACATATG AAAATCATCT 23280
   GATGGATCCT TAGGGACCCT GATTAAGTAA GACTGGCCAA GGGACCTGGA ATCTGCATTT 23340
   TAGAAAGCTC TTCAGCCCGG GGCACCAATG AAGGGTTATA AGCAAGGAAC AGGCATTAGC 23400
   AGATTTACAC TTCAGATAGA TTGTTTCAGC AGTAGTGTGG AATATAGATT TGAAAGTGGG 23460
   GAAAGACTAC AGCCTCAGGG ATGAAAGAGA AAGCTACTGA AATAGCCTAT GCTAAAATAT 23520
   GATGCATCCT GGGCCAGGGC AGAGATACAA AGTGGAAAGG AAGCCATAAA TGTGAGAAAT 23580
   CATTAAGGGA AAAATCAGCA TGACATTATG ATTGGTTCAA TGTGGGAAAG TCAGAGAAAT 23640
   AGAGAGGAAT CTAGGAGGAC TTACAGATCT CTGGCATTGG AAACCAGGTG GACAGTAGTG 23700
   CTGTGAATAC AGAGGGGGTG TGCAGAAAAT GATGCAAGTC TGGACAGGAG GGCTTCAGTG 23760
   AGGAGCTCAG GTCTGGACTA CTTGAACATG AGATGTCTGA TGACTCTAGG CAAGGGGACT 23820
   TGACCATATT TCAACACATC CAAAGCTCAG GGGACACTTG TGGGCAGGCG ATGGAGTCAT 23880
   GAGCACACAG TAATAACTTC TGCATCAATC TTTCCCTATC TCTACTGCCC TACTCTCATC 23940
   TCTCACCAGG TTTATTTCAA CAGCCTCTTT ACTGGTCTCC CCAGCTTTGG GCTTGCCTCC 24000
   CTGGAGTCCA TTTTCCTAAA TTCAGCAGCC AGACAGATCT TTCCAAAAAA TAAATCTGAT 24060
   CTTCTCACTT CATTCAGAAT ACTCTTCCAC TGATTTGATT TGGGGCCTCC TGTCACCTTC 24120
   AGGATAGAGC CCAAACCACT AGTCATGGCT GCCAGGCTCC CAGACACACT TGCCTTTTCC 24180
   AGCCTCTTCT CTTGGCCCTC TCCACTTGTA GTCCATGCCG TAGACTGTGC ACCCTGGACA 24240
   GTGTCACATA GAGTGCTATG GGGGTGGCAC CCCCTGAAGT TCAACAGCAC GGAAGCCCTG 24300
   ACTGGTATGA CATGGTTCAA TGTCCAGAGT TTAATTTTAA GAATCAACAA CTAGACAAAG 24360
   TAATGATATT GACTCAAACT TACTATTCAA ACCAACCTTT TATTCCTTAG GCTTGTGTAG 24420
   CCTGCCCTCA GAGAGAACTA TGATGATGGA CATTATCAAA AGGAATGAAA AAAGAATTGA 24480
   CCTGTAAGAA TTTTTTTTAA TTCTTTACAT GAAGCAGTGT TTCTCAAAGT ACAGTGATCT 24540
   AACTACTTAC AAGAACCACC TAGCTGCCTG ATAAAATGCA AATTTCTGGG CTATAGCCCA 24600
   GATGATTGAA TCAGAAACTC CGTGTGTGAG GCTAAAAAGT TGCATTTTTA TCTTCTTCCT 24660
   AAGCGATTCT TATACATACT AGGTTAAGAA CCAAATACTT AAAGATAAGA ATTGTACCAA 24720
   ATCAGAGCAC TTCTCCTTGG CTTAATTTCA TTTCAGTTTT ATATGATGCC TATGTCAGAT 24780
   TCCATAACTT CTCAAGCCAC CTACACTCTG TGGTTAGAGA GGGAATGGGA TGAGACAGTG 24840
   GTGGTGATAG TAGCTTGAAT AGCTGTGAAA AGTTAGAGAA TCCCCATCAG AATAAATTAG 24900
   GAAGGGGTTG GTGTGAAGGT TCAAGGATTT GTACTTTGTG ATGAGGTAAA ATGAGGTTCA 24960
   ACAGTGATCG AGTACCCTTG GAAAGTTGAT TTGGGGCTTA CATCAGGTGT AAAGAGTTTT 25020
   CTCATGTTCA AATTCAAATT TACCTAAGAT TGATTGAGTA TCTACTATAC GCCATCCAGA 25080
   CTGCCAGGTA CTTTAGTAAT TTAACAAGCA AATATTAAGC ATCTCCTTTG AGCAAGACAC 25140
   CAAGCTATGC TTTCATATGC ATTATCTCAT GAATTCCTGC AGCGGCCCTG GCTAGCATGT 25200
   ACTTGCCTGG AGATTTGCCA CCGCTTAAAA AATGCCAAAC AATGGTTACC AATCTTGTCA 25260
   CATTTCTAGA GCATCCATGA ATTCATGGCT CTTTATTTGA GGGCGTATTC TCAATCTGAG 25320
   ATATGAGCCT CCTGGTATGA TAAACTCAAA CTTTCCACCA GAGATTCATT GAAAACTCAT 25380
   TCACATATTC ACTCATTCCT TCATTCCTTT AGCAGTTTTG AATGCCTAAT ATTCTAGAAA 25440
   ACTTAGAACA TTCTGTGAAC ATTCCCTTTT TACTTTCTTC ACTAAGGTTT GGAGAAAGCC 25500
   AGAGCCAGAC GTTGCAGACC AATTATGTTG ACTACTTGGA CGAGCTCGCC TTAGAGATAG 25560
   GTGCGAAGCC AGATTTCTGC TCTCTCTTGT TCAAAGATCC TAAACTGGCT GTGAGACTCT 25620
   ATTTCGGACC CTGCAACTCC TATTAGTATC GCCTGGTTGG GCCTGGGCAA TGGGAAGGAG 25680
   CCAGAAATGC CATCTTCACC CAGAAGCAAA GAATACTGAA GCCACTCAAG ACTCGGGCCC 25740
   TGAAGGATTC ATCTAATTTC TCAGTTTCTT TTCTGTTGAA AATCCTGGGC CTTCTTGCTG 25800
   TTGTTGTGGC CTTTTTTTGC CAACTTCAAT GGTCCTAGTC AGCATAATGC TTTGGGCTTT 25860
   ATTATCTTGT CAGTCACTAC CTCCTAAAGA AAAAAAAAAA GGCTAGAAGA AAAAACATTA 25920
   CATTCATGTT CTAATTATAG ATTTTAGAGT TAGGTAGTAC AGGTAAGGGG GAAATTGTAA 25980
   AGAATTAGCA GAATTAGGCA TATGTACAAA ACCAAA 26016
(2) INFORMATIONS POUR LA SEQ ID NO: 2:
   (i) CARACTERISTIQUES DE LA SEQUENCE:
      (A) LONGUEUR: 1731 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 2:
   AACCAAGGGA GAAAACTATT CTGTCAAAGA GACGGTGCCA AAAGGCAAAA ACAAAGGAGC   60
   TGATGGCAAA GAAGGTAGCT GTGATTGGAG CTGGGGTCAG TGGCCTAATT TCTCTGAAGT  120
   GCTGTGTGGA TGAGGGACTT GAGCCCACTT GCTTTGAGAG AACTGAAGAT ATTGGAGGAG  180
   TGTGGAGGTT CAAAGAGAAT GTGGAAGATG GCCGAGCAAG TATCTATCAA TCTGTCGTTA  240
   CCAACACCAG CAAAGAAATG TCCTGTTTCA GTGACTTTCC AATGCCTGAA GATTTTCCAA  300
   ACTTCCTGCA TAATTCTAAA CTTCTGGAAT ATTTCAGGAT TTTTGCTAAA AAATTTGATC  360
   TGCTAAAATA TATTCAGTTC CAGACAACTG TCCTTAGTGT GAGAAAATGT CCAGATTTCT  420
   CATCCTCTGG CCAATGGAAG GTTGTCACTC AGAGCAACGG CAAGGAGCAG AGTGCTGTCT  480
   TTGACGCAGT TATGGTTTGC AGTGGCCACC ACATTCTACC TCATATCCCA CTGAAGTCAT  540
   TTCCAGGTAT GGAGAGGTTC AAAGGCCAAT ATTTCCATAG CCGCCAATAC AAGCATCCAG  600
   ATGGATCTGA GGGAAAACGC ATCCTGGTGA TTGGAATGGG AAACTCGGGC TCAGATATTG  660
   CTGTTGAGCT GAGTAAGAAT GCTGCTCAGG TTTTTATCAG CACCAGGCAT GGCACCTGGG  720
   TCATGAGCCG TATCTCTGAA GATGGCTATC CTTGGGACTC AGTGTTCCAC ACCCGGTTTC  780
   GTTCTATGCT CCGCAATGTA CTGCCACGAA CAGCTGTAAA ATGGATGATA GAACAACAGA  840
   TGAATCGGTG GTTCAACCAT GAAAATTATG GCCTTGAGCC TCAAAACAAA TACATTATGA  900
   AGGAACCTGT ACTAAATGAT GATGTCCCAA GTCGTCTACT CTGTGGAGCC ATCAAGGTGA  960
   AATCTACAGT GAAAGAGCTC ACAGAAACTT CTGCCATCTT TGAGGATGGA ACAGTGGAGG 1020
   AGAACATTGA TGTCATCATT TTTGCAACAG GATATAGTTT CTCTTTTCCC TTCCTTGAAG 1080
   ATTCACTCGT TAAAGTAGAG AATAATATGG TCTCACTGTA TAAATACATA TTCCCCGCTC 1140
   ACCTGGACAA GTCAACCCTC GCGTGCATTG GTCTCATCCA GCCCCTAGGT TCCATTTTCC 1200
   CAACTGCTGA ACTTCAAGCT CGTTGGGTGA CAAGAGTTTT CAAAGGCTTG TGTAGCCTGC 1260
   CCTCAGAGAG AACTATGATG ATGGACATTA TCAAAAGGAA TGAAAAAAGA ATTGACCTGT 1320
   TTGGAGAAAG CCAGAGCCAG ACGTTGCAGA CCAATTATGT TGACTACTTG GACGAGCTCG 1380
   CCTTAGAGAT AGGTGCGAAG CCAGATTTCT GCTCTCTCTT GTTCAAAGAT CCTAAACTGG 1440
   CTGTGAGACT CTATTTCGGA CCCTGCAACT CCTATNAGTA TCGCCTGGTT GGGCCTGGGC 1500
   AATGGGAAGG AGCCAGAAAT GCCATCTTCA CCCAGAAGCA AAGAATACTG AAGCCACTCA 1560
   AGACTCGGGC CCTGAAGGAT TCATCTAATT TCTCAGTTTC TTTTCTGTTG AAAATCCTGG 1620
   GCCTTCTTGC TGTTGTTGTG GCCTTTTTTT GCCAACTTCA ATGGTCCTAG TCAGCATAAT 1680
   GCTTTGGGCT TTATTATCTT GTCAGTCACT ACCTCCTAAA GAAAAAAAAA A 1731
(2) INFORMATIONS POUR LA SEQ ID NO: 3:
   (i) CARACTERISTIQUES DE LA SEQUENCE:
      (A) LONGUEUR: 535 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 3:
   Met Ala Lys Lys Val Ala Val Ile Gly Ala Gly Val Ser Gly Leu Ile
   1               5                   10                  15
   Ser Leu Lys Cys Cys Val Asp Glu Gly Leu Glu Pro Thr Cys Phe Glu
               20                  25                  30
   Arg Thr Glu Asp Ile Gly Gly Val Trp Arg Phe Lys Glu Asn Val Glu
           35                  40                  45
   Asp Gly Arg Ala Ser Ile Tyr Gln Ser Val Val Thr Asn Thr Ser Lys
       50                  55                  60
   Glu Met Ser Cys Phe Ser Asp Phe Pro Met Pro Glu Asp Phe Pro Asn
   65                  70                  75                  80
   Phe Leu His Asn Ser Lys Leu Leu Glu Tyr Phe Arg Ile Phe Ala Lys
                   85                  90                  95
   Lys Phe Asp Leu Leu Lys Tyr Ile Gln Phe Gln Thr Thr Val Leu Ser
               100                 105                 110
   Val Arg Lys Cys Pro Asp Phe Ser Ser Ser Gly Gln Trp Lys Val Val
           115                 120                 125
   Thr Gln Ser Asn Gly Lys Glu Gln Ser Ala Val Phe Asp Ala Val Met
       130                 135                 140
   Val Cys Ser Gly His His Ile Leu Pro His Ile Pro Leu Lys Ser Phe
   145                 150                 155                 160
   Pro Gly Met Glu Arg Phe Lys Gly Gln Tyr Phe His Ser Arg Gln Tyr
                   165                 170                 175
   Lys His Pro Asp Gly Ser Glu Gly Lys Arg Ile Leu Val Ile Gly Met
               180                 185                 190
   Gly Asn Ser Gly Ser Asp Ile Ala Val Glu Leu Ser Lys Asn Ala Ala
           195                 200                 205
   Gln Val Phe Ile Ser Thr Arg His Gly Thr Trp Val Met Ser Arg Ile
       210                 215                 220
   Ser Glu Asp Gly Tyr Pro Trp Asp Ser Val Phe His Thr Arg Phe Arg
   225                 230                 235                 240
   Ser Met Leu Arg Asn Val Leu Pro Arg Thr Ala Val Lys Trp Met Ile
                   245                 250                 255
   Glu Gln Gln Met Asn Arg Trp Phe Asn His Glu Asn Tyr Gly Leu Glu
               260                 265                 270
   Pro Gln Asn Lys Tyr Ile Met Lys Glu Pro Val Leu Asn Asp Asp Val
           275                 280                 285
   Pro Ser Arg Leu Leu Cys Gly Ala Ile Lys Val Lys Ser Thr Val Lys
       290                 295                 300
   Glu Leu Thr Glu Thr Ser Ala Ile Phe Glu Asp Gly Thr Val Glu Glu
   305                 310                 315                 320
   Asn Ile Asp Val Ile Ile Phe Ala Thr Gly Tyr Ser Phe Ser Phe Pro
                   325                 330                 335
   Phe Leu Glu Asp Ser Leu Val Lys Val Glu Asn Asn Met Val Ser Leu
               340                 345                 350
   Tyr Lys Tyr Ile Phe Pro Ala His Leu Asp Lys Ser Thr Leu Ala Cys
           355                 360                 365
   Ile Gly Leu Ile Gln Pro Leu Gly Ser Ile Phe Pro Thr Ala Glu Leu
       370                 375                 380
   Gln Ala Arg Trp Val Thr Arg Val Phe Lys Gly Leu Cys Ser Leu Pro
   385                 390                 395                 400
   Ser Glu Arg Thr Met Met Met Asp Ile Ile Lys Arg Asn Glu Lys Arg
   405 410 415
   Ile Asp Leu Phe Gly Glu Ser Gln Ser Gln Thr Leu Gln Thr Asn Tyr
   420 425 430
   Val Asp Tyr Leu Asp Glu Leu Ala Leu Glu Ile Gly Ala Lys Pro Asp
   435 440 445
   Phe Cys Ser Leu Leu Phe Lys Asp Pro Lys Leu Ala Val Arg Leu Tyr
   450 455 460
   Phe Gly Pro Cys Asn Ser Tyr Xaa Tyr Arg Leu Val Gly Pro Gly Gln
   465 470 475 480
   Trp Glu Gly Phe Arg Asn Ala Ile Phe Thr Gln Lys Gln Arg Ile Leu
   485 490 495
   Lys Pro Leu Lys Thr Arg Ala Leu Lys Asp Ser Ser Asn Phe Ser Val
   500 505 510
   Ser Phe Leu Leu Lys Ile Leu Gly Leu Leu Ala Val Val Val Ala Phe
   515 520 525
   Phe Cys Gln Leu Gln Trp Ser
   530 535
(2) INFORMATIONS POUR LA SEQ ID NO: 4:
   (i) CARACTERISTIQUES DE LA SEQUENCE:
      (A) LONGUEUR: 25464 paires de bases
      (B) TYPE: nucléotide
      (C) NOMBRE DE BRINS: double
      (D) CONFIGURATION: linéaire
   (ii) TYPE DE MOLECULE: ADN (génomique)
   (xi) DESCRIPTION DE LA SEQUENCE: SEQ ID NO: 4:
   TCCAGTCCTG CAGCAACCTC CTAGTTCCTG CTCTTTCAGC TCTTTGACCT TTTGCAAGCA 60
   CCTAATTCCC TGTAGTATAT ACCTTTCTTC ATGATATATA GTGTTTTTTA TCTCCTGCAC 120
   TAAATCATGA GCATATGCAT ATAAATCATA ATATGAAATC TTAAAAACAG AAGTACTTTT 180
   GCTGAGGCAT TAAGCATATA ATCAGTCAGC AGGTCCCCAA ACATCTAATT CCTGAATATC 240
   TCATATATCC TGTCTCCATT ATCCATTCCT CTAATGCTAC TCTAATTTAA GTCCTCAGTC 300
   TCTCTGGCCT AGATTGTTGA AATAACATCC TGGGTTTTTG GTCTCCTTGA TTCTAGTCAC 360
   CATCCTCTCT AGCCTCCAGG TGAATCTGAT CTTGTCTGAT GTTGTCACTT CCTTGTTCAA 420
   AATTCTCGAA TGGACAACCG TAATCCAGAA GGTAGTATCC AAACCTGTGA TTGTGGCACT 480
   TCAGTATCCT TCATAACCTA TGTCCTGCAT GTTTAACCCA TATTTTGCTA TTCCCATCAC 540
   TTATGGTCCA GCAAAACTGA ACTAATTGTA GTTCCCCCAT CACGTGTTCT TACTTTTCTA 600
   TGCATTTTCA CATATTTTTC TCTCTGCCTT TTTTCTATTT CTTGTCCCTT ATCTGTCTGG 660
   AAAACATCTA TTCTTCCTTC AAGACTCAGC TGTCTTCTCA CACTCCTTGA AGCCTCTCTT 720
   TCCTCCTCCA AGTGGACCTA GATTTTTCTT CCTACATGCT AGCACTACAC TGAACCATAC 780
   TTCCACTGTG ACATTTATCA TCTCCCTCAA CACTAGACTT CATGGTTCCA GATGGAAAGC 840
   ACTGTGTCTT CTCACCTTTG AATCCCCCAA AAGACTATTA TAATGCATGA CATATAGTAG 900
   GCTGTCAGTA CAGTGAAAGG AATGGCCAGA GGAAGGAAAG GAGGGAAACA GAAGCAGAAA 960
   GGACAGGTAT AGAAGCCGGA GGGAGCCAGA GACAAGGTTC AGAGACCACA ATTCTGTCTT 1020
   TTGAGTTCAC TAGTTTTACA AGCTCATCTA TAAGCGTTAG TTCAGCAACT CAGATCAGGC 1080
   CCTAAGTTTC CAGAAATTTG AGCTACTTTT CACTGTTGGC ACAACAAAAC GTTTCATTAT 1140
   AGTCCAGGTG CATAGCCTTT GTTTATATAT TCTATATTTC CAAAGCAAAC ATAAATGAAA 1200
   GAATCATTGT TCCCCTAATC TCCCAGGAGT TTCACCTTAC AGCTCCAGTG GCCATGGCAG 1260
   TCACTGTTTT ATATTTTTTG TAACAAGAAC CAAAGACTTC ATTCTTCCTT TTTCCTACCC 1320
   CTTTCTTTTT ACTTCACCCA TGCCTCCCCT GTTCTTCTCT TATCCCTACC ACACTCGTCC 1380
   TTCTCTTTCA GATTTTACTA TGGCTCTATA CCATTAAAAA TACAAGAAAA AAAAGGAATT 1440
   TTACTTTAAG AATAACTCCT CCCCCTTCCC CAGTTTTCAC ATCAAAAGAC ATTGTTAAAT 1500
   GCCATTCTCT TCCACATTTC GAGAACTGCT GATTCTCTGG GGAGAGAAAG GTGATTGCTT 1560
   AAGAGGTGAA GTCCCTTAGA GCATTCAAAA TGAGGAGTGA TTCTGTACAG AGGATATCAT 1620
   GCAGCAGGCT GGATGTCTAG TTCCAATTCC TTTATTTGTT ACCTCTGAGA CCTTGAAGAA 1680
   GTAGTTTCTA GTCTCAGCAT ACCAAAGCGT CATCTGCAAT TGAGAGCATT GGATTGATGA 1740
   TCTTCAAGGT CCTTCCTGCT CTAGCATTCA CTGAATCTGC TATTTTTGAC ATATTGAATA 1800
   ATCAGAAGCA GCCAGTTTTA GAATCTTATT ATAGCAAAAG TGGTAAAAAT AATGAGCATA 1860
   TACTATCAAT GTGCATCTAT GTCTTCTTAT GTTTGAGTGA GGATCCTGAT ACATAAACCT 1920
   TGGCTGATAA TTTCTACTGA AAAAAATCGT AAGTATTAAA GACACTCTTC TGAAGATGTT 1980
   CTCTCCAGAC TCTGCTACAG GCAATCATGA GCAAGAGGGT TGGCATCATC GGAGCTGGAG 2040
   TCAGTGGCTT GGCTGCCATA TGGTGCTGTC TGGAGGAGGG GCTGGAGGCC ACTTGCTTTG 2100
   AAAGGAGCGA TGATGTTGGA GGCCTGTGGA AATTCTCAGT GAGTGGCACA TCATTAGAAC 2160
   ACCAGTGGAA GGAGATGGAT TCCAATGCAA ATCAAATCTG ATCAGTTCTA ATTCAGATTT 2220
   AGAAGGCAGA TCACAAAAGC TCCAAATCTG GAAAGTAAAA TCTTACCTCT CCAATCATAC 2280
   TAATGCCCAA AAAAACTATT TCATACCAGC AAAATTTGTC CTGAAAAGGA CATTTTCAGC 2340
   TCATTAAACA TCATCACCTG CATGGTGAAA TCCAGATCTC CAAGCTGTAA AGGGCACTAA 2400
   TGTTGGTAAT TAGTCAAAAA TATACCATGG GCTTCCCAGG TAAGTGAAAC AATTCTATTC 2460
   TTTATTGCTC TTAAATGCCA GGAACACGAC TAGAAAAGAG ACAAACAAAC CTGGACTGAG 2520
   ATCCTGAGGT CAGAAGTCCT GAGTTCTAAT TTCAACTTGT AGGTTTTCTA GGCAGATAAG 2580
   ATTTCAGTCC AGTTGCTTTT GTTTCCCTGG ACCTCAAATG CTCATTTGTC AAATGCAGAG 2640
   GATATGATTC TATAATTAAC TTATGTCTAT TGGGCAGATA GAAATTATTA TAGATGATGA 2700
   TTGTGTGTGC GGCTGTTGAA TAGCCTATCA GCTCCAAATC CAGAGGGAAA AATTATGGTC 2760
   TTTGCCATTT GGGCTCATTG TAGAAATAAT ATAATTAGGA AATAGTCCTT GTAAACACAT 2820
   TTTTTTTTAA ATTTCAAAGC CAAGTTTGGA GAAACTTCTA GTTCTTCTGT CCTGGATTTC 2880
   CCAGCCATTG TAATCAGTTG TCGATGATAC ATATTTGGCT TGAAAACATA TTCACATCAT 2940
   TCATATTGTA ACTACTTCCT GTCCTGGTCT CAGTTACTGC TCTGCCTGCG CCAATAGCCT 3000
   CCTCCAATAG AGTATATCAG TGCTAACTTA GAACACATTT TTATTCTTCT CCAAGCTTTT 3060
   TTTAAAAAAA ATTGTGGTTT TGTAACCCTG AAAGCACTCC ATGAGATATA AGGTCATTAA 3120
   TTTTTATTTC CCAGTAGGGG GTAATCAAGA GTTAATATTT TTCAAGAATT TAATTTTGCC 3180
   TATTTACATT TGCTCAGGGA AATGTGGACA GCTTAGAGTA AATCATAAAA TGGCTTTCTA 3240
   CCATCTCCCT AGTAACAATT AAATGATGCT TGAGCATCTA TTCTGGTAGT TTGTGCTAAG 3300
   TACTGGGATG ACAAATATGG AATATAATCA CTCCTTGTAA ATGGTTCCAT TTCATTTGAT 3360
   TAAGCAAGCC ATAATATAAT TCCGTAATCC TTTGATAGCA AATGGGCAAA AACTCATTTG 3420
   ATAGCCGAAC CTCTTCTGAA ATCGTAAGGT TAAATACCGT GAATTGGATC AACATGAAGC 3480
   TAAGTCTCAC CTTCTGTTGC ACGGCAGAAA TTTTATTGCA TTTGACAGAT TGCTGCGCCA 3540
   GATCTCACTA GGAGTATTAT GGAGCAAAAT CCAAAAATGT ACACATTCCA AAATATATCT 3600
   GGCCCTAAGA CTTTTAAAAT AAGAGATTAT ATAACTACAA CAACAAGATA GACCTTGTCA 3660
   CCATCAATTC AATGGACGAG TGCCTCGAGC GTTTAGAAGA GGGTGGACTA CGGAAATCTT 3720
   AGTAGGTCAA AGAAAACCTC CCACAGGGAT GACACTTAGC CTTGAAGGAT AACCCCAGAC 3780
   AAGCAAAATA GAGGACCACC TGTGACACAA CTCCTAGAGA GTGCATTTCC CAATAAAGTC 3840
   TGCGAATGGC ACTCCATAGG CCTATGCAGT CATTGGCAGT GTGCCAGCAC CAGGTTAAGA 3900
   GAGACCAACA ATCCATGAAA GGCACAGAAA AGGCAATGAA CATGGTGTGT GCAGAGAGGG 3960
   ATCCATGAGT TATCCAATAT AGCCAGATCA GAAAGTTTAC TTAAGGAAGC AATAATATGA 4020
   TACAAAGATC AGTAAGATTC AAAGTTGGAT TCTGAGTTAT CCACAAGAGG AAATTCTTCT 4080
   TTTCCATAAG GTCATGTCTA TAAGCAAAAT TCTACTCAAA GTCCTGGTGA GGATATGGAC 4140
   CCATACAAAT ACTCAAAACT TTAGCCTCCT CCACATACCC CAGCCCTTCC TTCTTTTCTT 4200
   AGAAAAGTTG CTTGGCACAA TATATAATCA GAGAGGGATT TTTTTTATGT GTTACATAAG 4260
   ACTTTATCTT GTAAGCCTTT TTTAGAAGGT GTTCTAGCAG ACAGAAACGT GGTAATTCTG 4320
   AACTTTTCAC TATTTGCTTT TTCTGAGAAA TGAAAACCAA ATGGGATTTA AATACTAGCA 4380
   GGCTGAATGT GTGTTTTAAG TTTCATCCAC TCCTAAATAG GGCCTCGTGT CCTCAAAAGA 4440
   TTTCATTACT GCTGTAATAA GAAGTTGCTC AACAGCCAGG TGCGGTGGCT CATGCCTATA 4500
   ATCCCAGCAC TTTGGGAGGC CAAAGCGGGT GGATCACGAG AGGTCAGGAG TTCAAGATCA 4560
   GCCTGGCCAA CACAGAAAAA CCCCATCTCT ACTAAAAACA CAAAAATTAG CCAGGTGTGG 4620
   TGGTGGGTGC CTGTAATCCC AGCTACTCAG GAGGCTGAGG CAGGAGAATC TCTTGAACCC 4680
   AGGAGGCAGA GGTTGCAGTG ACCTCAGATC ATGCCACTGC ACTCCAGCCT GGGCAATAGA 4740
   GAAAGACTCC ATTAAAAAAA AAAAAAATGC TTACCAATAG GTTAGTAGCA TTTTGATTGC 4800
   AAAAGCTGAA GCCAGGACTA TTTGAACTTT TTCCCACTCA TTTATTCCTT TGTTCATTCA 4860
   ATGAATACAT ACTGTGTACT TTATGTGTAG GGTACTATAT TAAGCATAAG CTGCAGATAA 4920
   GAGGCCAGCC AGCACTTTAA AAGCCGTGAG AAAACAAGTA TCAGAATAAC TATAAGTGAC 4980
   TATATAATTA GGGCAATAAG GATAATGGGA CCTTAGTAAA ACTAAAGATG ATTTGGCAGT 5040
   AGCTGAGAGG GAAGGTAAAG AAAGCCATGA CAAAGTTGAA GGCAACTTTT GAGCATATTT 5100
   CAAGGGCATA TTTAGACAAG GAGATATGGG ACTCATAAGC AGAGCTGGAA TAGGAAAGAA 5160
   GATCAAGGTA AACTGCTTAG ATGCATGTAC AACATTCTGA AATTAACCTC TGACTTTGCC 5220
   CTCAAGTTAC TTATGTTCTC GTGGGAAAGA TGAGAGATGA ACACGGTTAT CATCCAAGAC 5280
   AGATGGTGCC CACAGCTGCT TAGATCTCTG GTTCCAGGGT AAAGCTCCCT CAGCTAGAGG 5340
   CAGAGTCAAA GTTGAATTTC CTCCTTACTG GCTCAAACCA CACCTCATAT TGAAATAATA 5400
   AAAATGCATG CTCCCTGGAG CAACTGACTT GTTATCTAAT ACATTTGCTT TTTTGTGTTC 5460
   ACTTGGAGAA CAGTCTTTTC GGAAAAATTC CAAGGAGCTG TAGTGTACAT ACTCTTCTCT 5520
   CCTGGTGTTA TAATTGGCTG AGGTCAAGGG GCAAAAAAGC AGAGATTCAT TCAAGATGGA 5580
   AATATTCCAA GGCCTTAGCA TCTGTTTCCC AGAACAGAGT CTTACATTCT TTAACCAGGC 5640
   TCCATCCCAC AGTTCAGCCC TGCCTCCTTT CAACAGGCAG CTGAAAAAAC CTCCTTCCCA 5700
   CCTCTCCTTC TCACAACCAT CAGTAGAAGG CGCTAGCTGT GGGTGAAAGG GAAGCACTCA 5760
   GCCTGCCAAA CTGCTGGACA TGAGCCTTCA CCCTTTTTCT GACCTCCACA AAAATTTTAA 5820
   AAAGTTTAAA TTCCTGTGCT TCCACGCTTA TGAGAAATAC AGCAACCATG AATAGAGGAA 5880
   GATTATGTTT TCAACTTGAG AAAAAATACT GAGGCTTTGG GCAGCCCCCC ACTTCCCCAC 5940
   GGGGACACAA TCCTCTCAAC CCTTTCCAGC ACTTTTTGTT TCCCTCTTGC AGAGGTCATC 6000
   TGGTGTGAGA GGGAGATACA CATCTTGAAT CCAGCAGCAA CGTGACATTC CATCTCTTTC 6060
   CCCCCATTGC ACAAGAGTCC CTTGCGGACC TCGGGAAGCA GAAGCTGCCA GCTCTGAAAT 6120
   GTATTTTCAA GGCAGCACAT TGTGTGCACT TTTACCCTAC CCTCACAACT GAGAGGAAAT 6180
   GTTTATTTTC AATTTAGCTT TTGACTGCTT CTAAAAAATA AGCCACTTTT CAATTACACA 6240
   GAGGCTTTAA AATGAAGTGC CAAGATTTAA CACATGTTCT AAGGGCTCTG GTTTCCTGTG 6300
   TTTCTTTGGT GAGGAGTGAA GTCCAGCAAC TGGTGAGCCA AAGAATAGGA TTCATTTACA 6360
   ACAGAGCAGT GGTTCTCAAA GTGTGGTTCC TAAACCAGCC ACATCAGCAT CACCAGGAAC 6420
   TTGATAGAAA TGCAAACCAC CCCAGACTCC ACCCCAGACA GATTGAATCC GAAATTCTAA 6480
   GAATAGGGCC CAAGAATCTA CGGTCTAGGG AGCTTCCAGG CGATTCTCAT TACGCCAAAG 6540
   CTGGGAAACC ACTGCAATAT TGGGTTGTTG CCAGTGAAGA GTTTGCTAAA CTCCAAAAGC 6600
   AAATAAATAG GCTAGAAGTC AGAGCCTCTT CTAGACAGTT TTGTTTTTTG TTTTTTTTTT 6660
   AACCTGAGTA TAAGATCAGA ACCAGTGGTG GCACAGGAGA AAGCAAAAAC CACTAAGTGG 6720
   CTATAAAGAC AGAGCTAACA CTGAGGGTAA TTACAGTAAG AGGATTCACA TGGAAAGAGC 6780
   TCCAGTTCTG TGCCAGGTTA CGCGAAGGGC TTTCCATTCC TTATCTTACT GAGAGCTTTT 6840
   AATTTTTGTT TACGCTTTTA AACATGAAAA GGGTTTTAGT CAACCAAGAA TTGAACCACT 6900
   GTGTTCACTG AAGGGAACAC AATTCTTGGC TTTCTCTTTA AGCTTTCTTA TTCTCCCTAG 6960
   GACCACACAG AAGAAGGCAG AGCCAGCATT TACCAGTCTG TATTCACAAA CTCTTCCAAA 7020
   GAAATGATGT GCTTTCCAGA CTTCCCTTAT CCGGATGATT ACCCAAACTA TATACACCAC 7080
   AGCAAGCTCC AGGAATATAT AAAGACATAT GCTCAAAAGA AGGATCTTTT AAGATACATA 7140
   TAGTTTGAGG TAGGGGTCTC ATAACTTGTA CTGTTGAAAT TAAGATATGT GTGGGTTAGA 7200
   GAAAAAGGAG GCAGCAAACT ATTATAAAAA TTAGAGCCAA ATGTTTGGGC ACCTCAGTAA 7260
   TCAAATGTTG GGTCTGATTA TAAAGCATTC ATGCATTGAT TTTTTCTCTC CTAGACTTAC 7320
   TAGTTCACTA GTCTCTGAGA GCTTTCAGAC TACCTTAGAA AATGGAGGCA GCTAGCCCAT 7380
   CATTGTCCAC TTTCCACCCT CATGCTCTGA TGTTTTGGAA ATAATCCAAA ATGCTTTAGT 7440
   ATATATTAGG AATTTTGTCA GTTCAATGCC AATGAGTTGT GGTTCAAAAA ACCAGAGCAT 7500
   TTGGTAGGGT TTCTCCCATT ACATTATGAA AAGGTTAACA ACTTAAATGG GAAATATAGT 7560
   CATTGCCCCC ATCTTTACCC ACTCAGTTCA TTAGTTTTTT TATTAAAAAG GTGAGATTTC 7620
   AGCATTGTTT CTGCGAGAAT AATGTTTTAC ATTTATTTGG GACTCTTTAT TGAGCATTTC 7680
   TGTCTGTATG TTTGGAACTC TTAACCTCAA TTAACTGCTG CTAAATGCAG AACACTTGCA 7740
   TATAGTGGGA AAAACAATCA GCAAAATTAT GAACCATGGT GATATTTACA TCATTATTTT 7800
   ACCTGGAGTA GCCCCAAATG TATAGTTAAA ATAAAATTTT CCAATAGTCA TTTTATTCCA 7860
   TTCATTCATT ACATTCATTT GCTTCCATTA TGGTGTTAAT ATCAACAAAC ATTAATGAAG 7920
   TTCCTATTGT GTGCTTGCAT TGTGCTATGT GTTATATGTA AAAGAAAAAG AGGTCTAAGA 7980
   CTTAGCTCTC AAGAAGTTAT TTCAAAATAA ATATGTAAAG AGTAAGTAAA AAGATTCCAG 8040
   TAACAATTTC AATCAAAGAG AAAATTTTTT AAAGCTCTTT ATGATTTGTT TATAAATAAA 8100
   ACAATGCTAT GGAGATCATG AAGCAAGAGG CAACACTTTG GGGGAAGGTA TTTTCTAGAG 8160
   GAGGTAAAAT TTAGTTGTAT TTAGTAGGTG TTTTAGATAA ATGAGTGGCA TGAGTAAAAT 8220
   TAGAGAGGTG GGAAAATGCC CTGCTCATTT GGAGAACAGT GGGCAAACCA AGTTGGTTAG 8280
   GAGGGAGATA TATATGCTAG GATGAGATAT GGCCACATAT ATCAGTAAAC TAGTGTGTAC 8340
   TGTGACTTTG AAAAATAGAG GATTATTTTG CAACCATGTA AAAGAAGTCC AAAGAAGGGA 8400
   CATCCAGAGC TTATGTGATG GCACCAAAGT TATCAAAGAT TCAGCTTCAC GCATCTTAGC 8460
   ACGTGGCCTA CATCATGACG TTTGCCTTGT GGTGCAAAAC AGTTGCTGAA GCTTGAGCCG 8520
   TCACATCTGC CTTCTAGCAA AAAAAAAAAA AAAGTAAAGA ATGAAGGGCA AAGGGATGTT 8580
   CTCTCAGCTG AATCAGCTCC CCTTTTACAA ATTCTCCTGA AAAAACTGTC CAACATTGCT 8640
   TATATCTCAC AGGCCACCCT AGTTGCACAG GAACCTGGAA AATGCATCCC TTTTCTGTGT 8700
   ATGTTGTCGC TCCAAACAAA ATCAGGGTTC TGTTAGTAAG AATGAAGGGA GAATGGACAT 8760
   TAGGGAAGCA ATTTGCAGAA TATGTTCCAG AAAAGTCTGT GGGAATAACA GAAAATAAAA 8820
   CTAAAAGAGT AAATTGGAAC AAAATTGTAT GGACTTAATA GTAATCGCAT TCAAAATGTA 8880
   GAATAAGTTT TAGAGGCTGT GAAGTAACAG AAATTGAGCA GTGAATTGAG CAGAGAAATT 8940
   GAGAAATGAA TATAGTCCTT CAGGAAGATT AATCTGACAA GCAGGACAAA GGATGGCTTG 9000
   TAGGAAATGG GAGGCTGAAG ACAGGCTAGG TATAGGTTCT TGCCGTAGTC CATGCAAGGG 9060
   AGTGATAAGG ACTTGAATGA AGGCAGTGTT AGCAATCATG GAAAGAAAGC GTGAGATTGG 9120
   GAGATAAATA CTGTTTAAAC ATGAGGCAAG GATGGAGAAA TAACAAGGAA AACAAGTCAT 9180
   GGATTTGAAG CATAAGTGGC TGGGAGTTTC ATGTCATCAT TCAAAGAAAT AAGAAAGTCA 9240
   GAAGCCAGTT TCAAAGGAAA TTTAAGTAGG TCAATCAAAA CCTGCTACAT ATGAGGAAGT 9300
   ATTAGGTGGC CCTCCAGATG GAAAGGTCAA GCTAAACTGG ATAGAAGAGA GACCAAGGAT 9360
   AGATGTATTT GTATATTCAT ACCACAAAAC TTGCTAATTT TTTTTTTTTT TTTTTGAGAC 9420
   GGAGTCTCGC TCTGTCGCCC AGGCTGGAGT GCAGTGGCGC AATCTCGGCT CACTGCAACC 9480
   TCCGCCTCCC GGGTTCACAC CATTCTCCTG CCTCAGCCTC CTGAGTAGCT GGGACTACAG 9540
   GCGCCCGCCA CCACGCCCGG CTAATTTTTT GTATTTTTAG TAGAGACGGG TTTCACCATG 9600
   CTAGCCAGGA TGGTCTTGAT CTCCTGACCT CGTCATCCAC CCGCCTCGGC CTCCCAAAGT 9660
   GCTGGGATTA CAGGCATAAG CCACCGCGCC TGGCCGTAAA GTTGCTATAT TTCTAAGATA 9720
   AGAGTATTTA TGCAGAGCAA AAGAGATGCC AACGATCAAA CCTTGAGATA TTCCCATACT 9780
   TATTGAGTAG ATGGAAGATG AGGTCAGAAA AGGAGGAAGC CATGTCAGTA GAGGGTAGCC 9840
   ATAAGAAAAT AACACAGATT TGTTATATGA CATCATTCAC AAAAATATTC AGTGTGATTT 9900
   ACCCCTAAAT CAACTAACTT GATGTCAAAA AGTAAATGTA CTCCAGTGAG TAATTTTTCT 9960
   TGTGAGATTC AAAGACTCAC TGAAGATTCA CTGTGACTCC AATTTTACTA TCTTTCTATA 10020
   CATTTCTGAA TGACCAAGAG AGCTCGTAAC AATTATTTCC TCCACAGAAA CAAGGCAAGA 10080
   AGGAAAAAAA CTTTCACATG TAGAATTATA AATGGAAAAA TAAATTTTCT AGTTTTCTTA 10140
   AAGACCCTGG TTTCCGGTAT AAAGAAATGT CCCAGCTTCT TAGTCACGGG CCAATGGGTT 10200
   GTTGTTACTG AAAAGGATGG GAAACAGGAA TCTACTATTT TTGATGCTGT AATGATTTGT 10260
   TCAGGACATC ACGTATACCC CAATCTGCCA ACGGATTCCT TTCCTGGTAA GTTTGGAAAA 10320
   TATATAATAA TCTAGGGACT TATATGCAAA CATCAAGAGT TAGAAACATA TCTTTCTATA 10380
   GGTATTACAT AATGATTATT CTTAGATTTC AAAAGAAAAA AATTAAGTTT AATGATAGGA 10440
   TATAGTAATA AATAGCCTCA TAAGTCCTTA TGTTAAAATA ATCAAGGACT GCAAGCCAGA 10500
   GATCAGACAA ACACAAGTTC CTGTGTTACA GACAGTAACT CAAATATAAG TTCTAACAGC 10560
   ACACGGGGTC TCCGAGCACA GTTACATTAA AAAAAAGTAG AGTCCAACTG CCAAATGGTT 10620
   TAAAGAAAGA CACGTTTACT TATGTTATTT ATAGGAGACT CCTAGGTTTC TAATTTCATC 10680
   TTCATCCACA ATTTGCAAAT AAACTTTAGA AATCTCAGTG ATTTGTGTGT GGGTACACAC 10740
   ATGGGTGTGT GTATAGCAGC ATACTTCATT ACCATCCGAA AGTGGCAAAC CTCAAATAAA 10800
   TACAATATAC ATGGAGGCTT CCTTCCATTT TTCCTTCCTT CCTTGCCACA GGAACACAAT 10860
   CTACTCAAAG ATATTAGAGT TTCCATGTCT AGGTATGATG TCCATAGGCC GAGGAAAATT 10920
   AAAGAGTGAA GGTTCAGGAG GAATATAAGA TTAAAACTCT TAATGTTAAC GGGCAGCATA 10980
   TTTAATGTTT ATGAGCATGG GATCAGAACA CCTGGCCTCA ACTTACTATT CCACTAGTTC 11040
   CTTACCACTT AACTTCTTTG TCTCAATTTC CTCTTCTTTT AAAATAGGGA CAATAGCCCA 11100
   CCATGCAGGG ATGTTATCAA GATTAAATAG TTAAAACGTG TAAAGCATTT ATCAGAGGAT 11160
   CTAGCCCACA GAGTTAACTT AATAAATATT AACCATTATT ATTATCGAAA CATACATTCT 11220
   CATGCCTTAA GATTTTTTAA GGAACTAAAA GTAAGTTTTA GGGGGCTTAA TGTCAAAAAA 11280
   TGCTAAATGG ATAAATGCAC TTCAACTAGG GAATTTTTTA ATTACAACTG ATAATAGGTT 11340
   TAAAAAGACA CAAAGAAAAC ATCTTCATAA TTTCTGAAAA TCAGTTCAAA CAACTTGCCA 11400
   TGTTCCACTT AGGCCTGGAC CAGTTTCGAG GCAACTACCT CCATAGCCGG GATTATAAGA 11460
   ATCCAGAAGC CTTCAAGGGG AAGAGGGTCC TCGTGATTGG TCTGGGGAAT TCGGGATCTG 11520
   ACATTGCTGT TGAGCTCAGC CGTCTGGCTA CACAGGTACA TGACGTAAAG GTTTTGGGAA 11580
   ATAAACCTAA GGTAGGGCTG TGCTACTAAA TCAGTAGCCA AGGCACAGAG GATGGTACTT 11640
   CTATGTCACA CCACAAGAGA TCCACCTCTT CTATGTGGCC CTTCAAATCA AGGAGGACTT 11700
   GAGACATCCT CCATGTGAAG CCAGGTAATG TGGCCCGTGC TAGTAAGGAA GTACATTCCA 11760
   CTGAATCCAG AAGTAAGTGC ATGAGTGCGT GTATGTACAG ATGAGTGTGT ATGTGTGTAT 11820
   TTCTTGTTTT CATTTTATAT TCTGATCACC TCCAAATAGA CTAGTTCCTG GTCAGGCTTA 11880
   ATCTTTATTT ATTTAACAGT ATTTATTATA ACGTATCATG CAAAAAGCAC TGTGTTTACC 11940
   ACTCTGAAGT TCTGAAAGAT ATGCATGACT TGGTATTTAC TAACATTAAT TCAATCAACA 12000
   GCAGATGCTC AACAAATATT GGGCACTTAC TATGCTTACT ATGTGTCAGA ACTATGATAA 12060
   ACTAAAAATA AATGCATAAA TAAGTTAGAC TAGTTCCTGA CTTCAAGAAA GAGTCAATGG 12120
   ATGGAGATGG AGTTGACAGG TACACACAGA CTATCACCAG AGGAGATGGT GAGTCTTCCA 12180
   GTAGAATTAG GTGTGGCAAT AGCAACACAG GGAAAAGAGA ATCTAACTTA GCCTGGATGA 12240
   GGTCAAGGAA GACTTCCCAG AGGACTCCAA GCTAAATCAT GTATCATCGA TAGACCCTAA 12300
   AGAAACAACA TATTTTTAAG AAAACAGGTT CTCAATAAAT AAATTCTTAA ATGGATGTAA 12360
   ATAAAACCTT AATTTTTTAA ACTAAAAATT CCCTTCAGTT ATCACAAAGT TAAAGTCTAT 12420
   TTTGCAAAGA CGGTAAAATA GATAAGCAGC CAGACTCATC TCAGGGCTGA GGCGGTTGCC 12480
   ATGGTTTGGG TTGCTCAGGA GAAGTCCTTG GGGTATGTGT ATAGGGAGAA CTGGAAAAGG 12540
   CAACCAGAGA CAGAGAACAG AATTAAATCC TTGACATCTC GTCAGCCTAA TTTCAGCTAG 12600
   AGATTTAGCT ACACTTTTCC CACACCTAGT CCACTATCAC CAGCCACAAC CACTGGGGCT 12660
   CACTGGATCA TCTGGTCCCT ACCAGACTTG CCATCTTAGT CTATGAGTAT GTGAAGATTA 12720
   AACCATCACA GTTGAACACA GAGCCCTGTT GTTCCTAGAG TGATGATTCT AATCCTTTCA 12780
   ACAACTACAC ACCAGCCCTC AGGGGCAGTG AAAGAATCCT GTCTCTACTA GTTTAAATTT 12840
   TAGACTTTAA AAAAAATTTT TTTTATTTTA AGTTCTGGGA TACATGTACA GAACATGCAT 12900
   AGGTCTGCAC ATGCCATGGT GGTTTGCTGC ACCTATCAAC CCTTCATCTA GATTTTAAGC 12960
   CCCACATGCA TTAGGTATTT GTCTTAATGC TCTCCCTCCC CTAGCCCTCC ATCCCCCCGA 13020
   CAGGCCTTGG TGTGTGTTGT TCCCCTTCCT GTGTCCATGT GTTCTCATGA TTCAACTCCT 13080
   GCTTATGAGT GAGAACATGC AGTGTTCGGT TTTCTGTTCC TGTGTTAGTT TGCTGAGGAT 13140
   GATGGTTTCC AGCTTCATCC ATGTCCCTGC AAAGGACATG AACTCATTCT TTTTTATGGC 13200
   TGCTAGACAA CTTATTTAGA CTCGCCTTTT AAAAGTGTTC CTACTTGGAT ATTGAGGAAA 13260
   ATGCACGGAA GTGCCCAAAG AAGTGTGTTG TGTTTGCTTA TTTCTTACAG AGTAATGCTG 13320
   AAATCTGTGT TGCTTTTCCC CACCAGGTCA TTATCAGTAC CAGAAGTGCT TCCTGGGTCA 13380
   TGAGTCGGGT CTGGGATGAT GGCTATCCTT GGGATATGAT GTATGTTACC CGCTTTGCAT 13440
   CCTTTCTCCG GAATGTCCTT CCTTCATTCA TCTCTGACTG GTTATATGTC CAGAAGATGA 13500
   ACACGTGGTT TAAGCATGAG AACTATGGCC TGATGCCTTT AAATGGGTAC TTAAAAATGG 13560
   AAATTTTTTT TATTCAAAAA AGGGGGGCAC TCATTTAATG AATTTATTCT CTCTAGAACT 13620
   TACTTTTGTT GTCTCATTGA GCCTAGAAAC ATTAAACTCA AGGTTTCACA GGTGACGGAA 13680
   TATGCCCAGA GACCACGTAT GGCTTGGAAA ACTTATTGAA ATTAGTGCAG TACAGAAAGG 13740
   GTATGGAAAA ATCTGAAATG GAGATGACGC AGGCAGATAA ATCACCCTGA CATGCATGAT 13800
   GCATTTGTGG TGGCTACAAG CTATAGCATA GAACTTTGAG GACTGAACAA ACTCAAATTG 13860
   GTTTTTGGAA GAATATCTTG TCCGTGCTTA TGGGTGTATG AAGACATCAA TAATAATACT 13920
   TGCTTCTCAA GATGGTTGTG GTATTCAATA ATATAAAAAT ATAAAAATTG CTTTCTAAAT 13980
   GATAAAGCTT TAAAAAAATT GGTTCTTCTT AGTCTCAATT TTTCTAATGT GCTTCAAAGG 14040
   AGCAAATAAC AAAATAGTGT TAATCAACAT GTCTCAGCAA GTAGGAAGTC TCAAAACAAA 14100
   AGTGCACACT TCCTCCACCC CTGAAATGTT GACATTTTTG CAGAACCATC AGGAGGCATG 14160
   GAACACATAA AGTAATGGAG AGTCACAACT AACGTGGCCT GTAAGATTAG TCAGATTCAT 14220
   TTATTTACTT CTTTATAGAG ACAGGGCCCA ACATTTACTA ATTAGGAAGT CATTCCAGGT 14280
   AGAAGAATCA GCATATCAAT AGAAAAAAAG AATATTTAAG TTGGTAAGAA AAGAAAGAAT 14340
   TGAGAAATTT TATCTCCTGG CCCATGCTAG CCAAAAAGTT TCATTGTGTT TAGAGAAAGA 14400
   TGGTAAGAAA AAGGAGGAAC TGTAAATCAA AAGAGCAAAT GCCAGATTTA GGAGCTAAAC 14460
   TGTCAGTCCA AAGCACTTAT ACTACCAAGT CTTGCAGGCT GCTATAACCC TTTAAAATAT 14520
   GTTGATTTTA TGCATTTAAA ATTATGTTTA ACACTGTGGC TTGCTTGACA GTAGAGGGTG 14580
   GGAGGAGGAA GAGAATCAGA AAAAAATACT TATCAGGTAC TATGCTTATT ACCCAGGCGA 14640
   CAAAATTATC TATACACCAA ACCCCTGTGA CACACAATTT ACTTATATAA CAAACCATGG 14700
   ACCCCCAAAC CTAAAATAAA AGTTTTTAAA AATTATGTTT AATATAGTAA GTCCCATAGC 14760
   TTGAGCTGGT TAAGATTTTT TATCTTGTAA GAGTAACTAT AAATTATATT TTGGCCTTGC 14820
   CATTTAGACA ATTAAAACAT AGTTTTAGAA ATTCATTCAT TCTGAAAACT AAGCTTCCTT 14880
   TTGGAAAGGG TTCCAATTAC CCTAAGTTTC TGGAGGGAGA AAGGGGGAGG AAAAACAGGT 14940
   TTCATTGTGG TCTATGTTTT GCTACCTTGT AAGGTAAAAG AAGAGGTTGC AGGATTAGAT 15000
   AAACAGAAAA TGATGTGGAA GTATAGAGAC AAATTTCAGG ATTTACAAGG TTTCTTTGTG 15060
   TCTGAGATAC TTGCAGGAAA TTCCGGAATC TCAAAGGAAA CTTAAATCAA AATGAAATAT 15120
   ATTGTCCTGA AAAATATTAT TCCTAGAATT TTGGCAACTA AAATGCAATA TCAAAGTTGT 15180
   TACACTTTTT TGTGGACACA GCTGATGAAA GAAAACCAAA CATGGCAATA AAACTTCCCA 15240
   CCACTGCAAG TCTGATTTCT CCATGTAAGA CAAGACGTTA AAGTTATGAT AATAGTGCAC 15300
   TTATAACAAC AGTGCTTGCA TGTGCCAGGA ACTGTTTTAA GTGCTTTAAG GATAATTGAT 15360
   CATTTAATTT TCACAACAAC CTATGAGGAA GATTCCATCA TCATCCCCAT TTTACACATA 15420
   AAGAAACAAA TACAGAAAAG TAACAACTAG TAAGAGATGG AGCTAGGTTA TGAACCTGGG 15480
   CCATCTGCTT CCAGAGTTGG CGTTCTTAAC CACTTTAGTA TGTCTATAAA TTAGTTTTAG 15540
   TCTCATTTAG GAAAGGAATT GCCATGAGAG AAGAGAGTCA GTGGCACTCA TGCTGATGTT 15600
   TAAGTGCTTG ATGTTATTTC AATGTTATGG GCTGTTGCAG GTATTTCTTG GAAATGAGCT 15660
   ATTTACAGCA AGGGTGTTTG CCTCTCATTG CTGTAGTTCC CTGAGAAAAG AGCCTGTGTT 15720
   CAATGATGAG CTCCCATCCC GCATCCTGTG TGGCACTCTG TCCATCAAGC CCAGTGTGAA 15780
   GGAGTTCACG GAAACCTCAG CTGTGTTTGA GGATGGGACC ATGTTTGAGG CTATCGACTC 15840
   TGTCATCTTT GCAACAGGCT ATGATTATTC CTACCCCTTC CTTGATGAGA CCATCATGAA 15900
   AAGCAGAAAC AATGAGGTTA CCTTGTTTAA AGGCATCTTC CCCCCACTAA TGGAGAAGCC 15960
   AACCTTGGCT GTGATTGGCT TGGTTCAGTC CCTTGGAGCT GCCATCCCCA CAGCAGACCT 16020
   GCAAGCCTGG TGGGCTGCTA AAGTATTTGC AAGTAGGTGG GCCATTCTGT CTTTCATTCA 16080
   TTTTATCAAT GAACATTTAC TGAACACCTG CTATATGCAA AGCACTGTGC TAGGGATACA 16140
   ATGAGAACAA GACAAACATG TTCCTTGACC TCTCAAGGCT TAAAATGGGG TGTGGGGGAT 16200
   GCCATAATAG GGGAAATTTG GGGGGGTTCT AGTGAGGGGA GTTGGACTGT TGCACAGAGC 16260
   AAACAGTATA CAGGAAGTCA TAAAGGTGAG GGAAAGCATG AAATGTGTAA GGACCCAGAA 16320
   ACATTTTGGT GGAAGGGAAT ATAAAGCAGA GGCAGGGAGT GGCAAGAAAT ATAGGTTTAT 16380
   AAGCCACGTT AAAGAGCTTA AACTTCTCAT AGGGATTAAG GACTTCGCAA GATTTTAAGC 16440
   AAGAAAAAAA TAGCAGAGGA TAACTGCAAT GTCAGGCTAC ATTATAAAGA TTGGAAGGGC 16500
   CCTGGTGAGG GTTGGAGGTG TGCCAGAAAC CTCACTGGTG TCAACTTCTG TCAGAATAAC 16560
   AAAGTCAGGC CACTCTGATT CTCATGACAA TCTTCTTCTT CTCTCCCTCT ACTCTAGACC 16620
   TCATGGTCTC CAGGGGCTAC AAGTATGCTT ATGTGAGGAA ATCAAGAATA TGAGGATTAC 16680
   ATGGAGAAAG GCAATGTCTC AAATATATTA ATTTACTCCA GTCATACTGA ATATTATCAT 16740
   TATTATTGAA AAGTGTTCTT TTATTCAGGT ATTCTCCAAA ATATTGACCA ATATAGGTAT 16800
   AACTTACCTA ACATAACTAA TCCATAAAAA CTTACACTAT TGGTAATTAA CAAACCATTA 16860
   CAATCATGGA ATATATGTAT ATATATTGTC TAAAACTTTG TAGATAAATA AATTTCTATT 16920
   TCAAATACAC CATGAAAGAT CATCATTTAA ATAAACCCCA TCATGAAATC TTTTGTAAAG 16980
   GTGCTCCCTG CAAAATACTT CTATTGCCTT TTTCCTTCGA AAGGCACAAC AATGCCAAGA 17040
   GCCTGGGGTA TTATGAGAAG ACTGGATATA GTTCATAAAC CTAAGAAATT TACATGAAGC 17100
   AAATGGTATC ATTTATTTAT TCAGCAAATA CTTACTGAAC ACCTACTATG TGTCAGGCTC 17160
   TAACCTGGCA CTTAGGACAC AACAACAAAC GAAGCAGAAC AAAATTCTGG CCTCTTACTT 17220
   TCTAGCAGGG TGTCCAGCCA ATATCAATCA TAGGGTACTA CCAGGTTGAC ATAAGACACT 17280
   AACGATGACT GGGAAATATT CATGCACTGC AAATTTTAGA GTAACTTTCT TCCACTGTTA 17340
   CAAAGGCAAA TAAGCTACCA TCACCAGTTA AAAGAAGTTG CATTGATGTA GTGAAATTCA 17400
   CAAAAAGCTA AAACTTGTCT GCTGCCCCTT AAAACACCTT GCATAGTTGC AGAAGATGTT 17460
   TAAAATCCTA TGCTTCCTTC CATTACCTCA TTTAAAATGG CAGAAACCTT AAAGGGAACT 17520
   GTTTTACCAG ATTCTTTCTT CAGAGAAGTT TTAGGAAAAG GATACAGAAA AAAAAGGAAG 17580
   AAATTATTAA GCTATTATAT GCATGAAGTG TACTGAGCAC ATATGTTGAG GATTAGGTCC 17640
   TCTATAATGT TACCGAAATA AGAGACTGAG TGATTTGAAG CTACAAATGT CTCTGCTGTC 17700
   ACTATCTCAC TACAGGCCAG CTTTTCCAAT TCCCAAAGGT TCATTAACTT TTCAGATCTT 17760
   TGTTTCTATG AACTGGTATT TTGCTAAAGA TATCAAAGAC ATCTCCAGCT CCTCTTAATA 17820
   CAAAAGTTTT CAGGAATACA GTTTATAAAA ACCAAATGAT TTCCATCATA TGTCATTATA 17880
   TATTTCTGAT TTGTGTTTTT CAATATTTTT CTCTTCATTT CTTTTCTAGA CTCATGTACC 17940
   CTGCCAACCA CGAATGAAAT GATGGATGAC ACTGATGAGA AAATGGGGAA AAAACTCAAG 18000
   TGGTAAGCAG CTAACTGTAC TTGCTAATAG AGCAAGTTCC TAAAATGTGC CTTTATGTGT 18060
   AGAAAAACAT TAATATGCTT TAATATTGTC ATTAGTCAGA GTTTACATTT TCTGAACACT 18120
   TGCAATAATC AAAAAATGTT TAGATAGTAA ACAGTCATCA CACTTCTCTT GTGTAACTCA 18180
   AGAATAGAGG TTTTCTATCA GGGATAATTT TGCCCTCCAG GTGACATATG GCAAAATCTG 18240
   GAGACACTTT TGGTCATTGT GAGTGGAGAG GGCATGCTAT CAGCATCTTA TGAGTAGAGA 18300
   ACAGGGATTC TGCTAACCAT CCAACAATGC AGAGCACAGT TCACCAAAAC AATTATCTGG 18360
   CTCAAAATGT CAATAGTGCT GAGGTTAAGA AACAACTCTA TAAATGACTA CAGTTGACCT 18420
   TTGAACAACA CAGGTTTGAA TTATATGGGT CCACTTATAC ATGGATTTTT TCAATTAACA 18480
   TAATGCAGAT TGGGCATGGT GGCTCACGCC TGTAATCCCA GCACTTTGTG AGGCTGAGGC 18540
   GGGCGGATTA CCTGAGGTCA GGAGTTCGAG ACCAGTCTGG CCAACATGGT AAAACCCTGT 18600
   CTCTACTAAA AATACAAAAA AAATTAGTCG AGTGTGGTGG TGTGCACCTG TAATCCCAGC 18660
   TACTCGGGAG GCTGAGGCAG GGGAATTGCT TGAATCAGGG AGGTGGAGGT TGCAGTGAGC 18720
   CAAGATCGCG CCACTGCACT CTAGCCTAGG TGACAGAGTG AGACTCCATC TCACAAAAAA 18780
   AAAAAAAAAA AATGCAATTT TTTGGAGATT TGCAGCAATT TAAAAACTCA AGGCCAGGCG 18840
   CGGTGGCTCA CGCCTGTAAT CCCAGCACTT CGAGAGGCCG AGGCGGGTGG ATCATGAGTT 18900
   CAGGAGATCA AGACCATCCT GGCTAACATG GTGAAACCCC GTCTCTACTA AAAATACACC 18960
   AAAATTAGCC GGGCGTGGTG GCGGGTGCCT GTAGTCCCAG CTACTCGGGA CGCTGAGGCA 19020
   GGAGAATGGC GTGAACCCAG GAGGCGGACT TGCAGTGAGC CCAGATTGTG CCACTGCACT 19080
   CCAGTCTGGG CAACAGAGTG AGACTCCGTC TCAAAAAAAA AAAACCTCAA AGATGAATTG 19140
   TGTAGCCTAG AAATATTTTA AAAAATTAAG AAAAAGATGC CATGTATAAA ATATTTGTAG 19200
   ATACTAGTCT ATTTTATCAT TTACTACCAT AAAATATACA CAAATCTATT ATTAAAAATT 19260
   AAAATTTATC AAAACTAAAT GCATACAAAC TCTTAGACTA TACATGGCAC CATTCATAGT 19320
   CAACAGAAAT GTAAACAAAC ATAAAGATGC AATATTGTCA TAACTGCATA AAATATAGCA 19380
   CATAATGTGC TAGTATAATA ATTTTGCAGT CACCTCTTGT TGGTATTGCA GTGAGCTCAA 19440
   GTGTTTTGAG TATCTACTTA AAATGCTGTG TGACATTAGT CATTTTCACC TGAGCAGTTC 19500
   ATATCTCCAG TAAATTCTGC CTCACAGTAA AAAGTGATCT CTCAAGGTTC TCACATATTT 19560
   TTATCATGTT TAGTGCAATA CCTTAAGCCT TTAATAACAC CATGGGCTCC ATATGAAGTG 19620
   TCATTAATGA TGTTGGAAGT GCTCCCAAGA AGCAGAGAAA AGTTATGACA TTATAATAAA 19680
   AAAATTGAGT TGCTTAATGT ATACTATACA TTGAGGTCTG CAGCTATAGT TGCCCACCAT 19740
   TTCAAGATAA ATGAATCCAG TGCAACTATG CCAGCAGGCA TGAAATCTTG CACTTTTTGT 19800
   AAAATATCTT TTTATTTTGG ATTGAAAATG CAGCTTTTTA TGTGGGTGCA GGATTGCTAT 19860
   AAGGAAGTAT ACATATAGAC TCTAATATAA TTTGAGAAAA AGTGAAGTTA TTATATGACA 19920
   AAGCAAAAGG AAGGTGAAGG ATCTAGAGCT GGAAAAGTTA ATGCCAGCAA AGGATGATTT 19980
   GATTACATCA GAAAGAGTTT GGCTTCAAAA ATGTCAAGAT AACAGGAGAC ACGCATGCTG 20040
   CCAACCAAGA AGAAGGAGAT GAATTCCCAG ATGTCATTAT GAAAATCATT GAGGAGAAAG 20100
   GATTTCTGCC TGAACAGATT TTTAACACAG ACAAAAGTGC CCTATTCTGG AAAAAAAAAA 20160
   AAAAAAAAAG CCACAAAGGC CATTTATTAA TAAGGAGCAG AAGTGAGCAC CAAGATTAGG 20220
   CAGGAAAGAA TAAGCTAACT ACTGTTTTGT GCAAATGCAG TCAGATTTAT GATCAGGATG 20280
   GCCCCTACCT ATGAAGCTAC CCCCTCAAAC CTTGAAGGGA AAAGATGAAT ATCAGCTTCC 20340
   TATCTTTTGG TTATACAAGA CCCTTTTTCT GGATTAGCTC TGTCAATGCT TTGTCCCTGA 20400
   AGTCAGAAAG TCCTTGCCAA TAAGAGACTG CCTTTTAAAG TTTTTTTGAT ATAGACAATG 20460
   CCCCTGACCA CCCAGAACCC CATGAGTTCA ACATGGAAGG CATCGAAGTA GTCTAATTTC 20520
   CCCCAAACAC AACATTCTAA TTCAGCCTTT ATATCAGGGA GTCATAAGGA CCTTTAAGGC 20580
   TCATCACATA CCATACTCTA TGGAAAAGAT AGTCAATGCT GTGGAAGATA ACCCAACAGA 20640
   GAGAACATCA TGAAAGTCTG GAAGGATTAT ACCATTGAAG ATGCCCTAAT TGTTATAGAA 20700
   AAAGCCATGA AAGCCATCAA TCCTAAAACA ACATATTTCT CCTGGAGAAA ACTATGTCCA 20760
   GATGTTATAT ATGACTTCAG AGGATTTACA ACAGACCAGT CACAGAAATC ATGAAAAAGA 20820
   TTATGGATAT GGCAAAATAA AAAGGTGAGG GTGAAGGGTT TCAAGATATG GATCATGGAG 20880
   AAATTCAACA GCTAATAGAC ACCACTAATA GACACTTTTA ATTCCACACT AGAGGAACTA 20940
   AAAGATGACT TGATGGAGAT GAGTCCTTCC AAAGCAGTGC CAGATGAGAA CGAAGACATA 21000
   GAAAAAGCCA TGCCAGAAAT AAATTGACAT TAGATCATCT GGCAGACAGG TTGCAGTTAT 21060
   TTAAGACTTC TTTTGACTTC TTTTATATAA CATGGACCCT TCTATGATAC AGGCACTGAA 21120
   ACTAAAGCAA ATGATAGAGG AAGGATTACT ACTATATAGA AAATTTTTAG AGAAATAAAA 21180
   AAGCAAAGTC AGACAGAAAT TATAATATAT TTCCATAATT ACACCAATGG GGCTGGCTCT 21240
   CCTGCCCCCA ATTCTACCTC CTCCATCTCT TCCGCTTCTG CCAGGCCTGA AACAGCAAGA 21300
   CCAACCCCTT CTGTTTCTCC TCCTACTCCT CAGCCTACTC AACATAAAGA TGATAAGGAT 21360
   GAAGACATTT ATGATAAACC ACTTCCACTT AATGAATAGT AAACATATTT TTTCTTCCTC 21420
   ATAATTTTCT TAATAACATT TTCTTTTCTC CATATTACTT TATTGTAAGA ATAGTATTTA 21480
   ATACGTATGA CATATAAAAT ATGTGTTAAT CAACTGTTTG CATTATTGGT AAGGCTTCCA 21540
   GTCAACAGGT TATTAAGAGT TAAGTATTTG GGGAGTCAAA AGTTATACAT GGATTTTTGA 21600
   CTGCAAGAGG GCTCAATGCC CCTAACCCCT CAGTTCTTCC AAAGTCAACT GATATAGGAA 21660
   GTTTCTTTAC TTTTTCAAGC ATTTAACATT GCATTGATAT GTCAACCTAA AGGAAAACAC 21720
   TGAGGCAAAT TTAATATAAA AAGAGAATTG ATTTGGGCCA AGTTTGAGGA CTGCAACCCA 21780
   GGAGCACAGA GTCAAATTGC CCTGAATATG CACTCCGTTG GCAGCAGTTA CAAGTAGGTT 21840
   TTTTAAAGGA AATACAAAAG AGTCAACTTC TAAGTTGTTT ACCAAGAACT TACATTAAAA 21900
   TTATATAAGC TATTGATTGG CTATATACTG TTCTTCGTAT CACAAATTCT ACGAACATGA 21960
   AGATGATGAG TGAGACAGCT AGTCAGGAAT AAAAATGCCT TTTAACAATT GCCGCCAGGC 22020
   TTGGTAGAGG GCAGCATGAC AAGTCCCATA CACGTGGCTC TCTCAGCTTG ATAAATTTTG 22080
   CATACCTCAC ATAGTGCAAA CTACTCTGAG CTATTTTTCT TCTCTCACAT TGAATGCCAC 22140
   AATGTAGTCA CCCATTCAGG GCCTAGAGAA GAAAAGAAAT GGAACCCTCA GATTCAACAA 22200
   AACCTCTCCT GCACAACTTC AGCCAGTTGA CGAACAACTT GCAGAGTTGG GCACTTTTAT 22260
   GTGCTAACAA TTCATGCAGC TTGATACCCT TTCCTTTAGA GCCCAGTAGA AATAAAAATG 22320
   AGGAAATAGA GAGGTTAAAA TGTTCATCTT ATTGCTTAAA TGATAAGCTG CTCTTCAGAG 22380
   TTTCAAAAAG CAAATTACAC CATATTCCAA CTAAAAGAAC TATAGAGGCG GAAAGGAGGT 22440
   GATCTCTTTT CTCTCTGTCA TAAAAGGTAA TGGCCAACAC CCCTATAACA AAAGACAGGT 22500
   TAACAAGAGA AAACGTGACA GATTTATTAC GTGCACATGT GTGCATGAGA GCCTTACAAA 22560
   ACATGAACTC AAAGGAGGGC CAGATCATTC ATGTTTAAAT ATTCTCTTCA CTGGGGTTAG 22620
   GGGAGATGGA AGTGTAAAAG TAAATGATTT TTCAGAGGAA ATTAATAAGT CCAAAGAACA 22680
   CAGATTAGAC CAAGTTTCTC TGGGCTTTGG GGGAGGTGTA ATCACCCAAC AGATTCATCT 22740
   TGCTCACTGC CCAGAAAAGC TGATGCCCTG AGAACAGCAG GTTTTTCCAA TAGAGAGAGT 22800
   TTAATAAACA CACAGCTGTC AGAGGCATTT GAACCAGAGT GACTCCATCT TGAATAGGGG 22860
   CTGGGTAAAA TGAGGCTGAG ACCCACTAGG CTGCATTCCC AGGAGGTTAG GCATTCTTAG 22920
   TCACAGGATG AGAAAAGAGG CCAGCACAAG ATTCAGGTCA CAAAGACCTG GCTGATAAAA 22980
   CAGGATTCAG TAACGAAGCT GGCCAAAACC CACCAAAACC AAGATGATGA AAAAAGTGAC 23040
   CTCTGGTCGT CCTCACTACT CATTATATGC TGATTATAAT GAATTAGCAT GCTAGAAGAC 23100
   ACTCCCACCA GCACTGTGAT AGTTTACAAA TGCCATGGCA ATATCAGGAA GTTACCCTAT 23160
   ATAGTCTAAA AAGGGGAGGA ACCCTCAGTT CTGGGAACTG CCCATTTCTT TCCTGTAAAA 23220
   CTTATGAATA ATCCACCCCT TGTTTAACAT GTAATCAAGA AGTAACTATA AGTATACTCA 23280
   GTTGAGCAGC CCATGCCACT GCTCTGCCTA TGAAGTAGCC ATTCTTTTAT TCCTTTACTT 23340
   TCTTAATAAA CTCGCTTTCA CTTTATGGAC TGGCCCTGAA TTCTTTCTTG TGAGAGGTCC 23400
   AAGAACCCTC TTTTGGGGTC TGGCCAGCTA AACGGAAGGA CAGGAGTTTA TTACTACTCA 23460
   AATCAGCCTC CATGAAAATT CAGAGGCTAG ATTTTTTTAA GGATAGTTTG GTAGTCAGGG 23520
   GCTAGGGAAT GGGGAATGCT GATTGGTTGG GTCAGGGATG AAACCATAGG GAGTCAAAGC 23580
   TTGTCTTCTG GTCTTCCTGG GAGGAGACCA CATGACAAGA TGAACCAGTT TACCAGTCTG 23640
   GGTAGTGCCA GCCGGCCCAT CAGAATGCAG GGTCTGAAAA ATATCTTGAG CACCAATGGT 23700
   AGGTTTTATA ATGGTGATGT TATCCATAGG AGCAATTGGG GACTTCTGAC TGCATGACTC 23760
   CTGAGCCCTA ATTTCTTATC TTGTGGCTAA TTTGTTAGTT CTACAAAAGC AGTCTGATCT 23820
   CCAAGCAAGG AGGGGGTTTG TTTTGGGAAA GGGCTGTTAC CATCTTTGTT TCAAAGTTAA 23880
   ACTGTAAACT AAATGTCTCC CATAGTTAGC TTGGCCTATG CTCAGGAATG AATAATGGCA 23940
   GCTTGGAGAT TAGAAGAAAG ATGGAGTAAT TACATTTTTT TTTCACATTT TTTTCACTGT 24000
   CACAATTTTT TTAAAGGTGA TTTCAGAGGT AACATCACAG GACATGGGAG ACTAAAGGGA 24060
   GGAAAGTATG TCAAACAAAG GCTGTCCTGT TCTGCAGACG AAACCTCACA GAAAGCAACT 24120
   CTCAGAGTCA GTAGCCTATG ATGAAAGTTT CTCTGTCAGA CATTCAGCAG TGCCTGACTC 24180
   TCAGTCTCTC TCTCCTGCAA GTTAATCTTT CCTAGAGTGG GCAAGGGAGG CCTCCGAGAA 24240
   AGCCTAGTTT CCATCTTCTG TTTACTTCCT TTTATTTTCT CCACAGATAA AAATCTCCTT 24300
   CACAAAAGGC AGCTTTTCAG GGCTGTTTCT GTCTGCAGGC CCTCTGAATA GCCATCTCAA 24360
   AATCTGTCAA CGAAGTGTAT ATTTTGCAGT AAAATATTTT TTGTTTTCTT TAGTATGAAA 24420
   CAATTTATAT TATTAGATTA CAGGAGTATT AAAACCATCC ATGATCTCAC TTTTAAACAA 24480
   ACCAATCTGA AAGTCTAACA TTGGGGCAGA TTCTAAGCAA TGTCTTATAA AGAATAATTA 24540
   TGTGTTAATG AGTAAACTAA GTTAATTAGT CTCCTTAAAC CAGAGGGTCA GTTTACTCCA 24600
   GGCCACATGG TCAAAGGCAA AAGTCCAACA TTACATCAAA CTCAAATAGA GATTAGGAAG 24660
   GAGGAGAAAA GCAGCTCACT TAGCTAAAGA AAAAACAATA AATTCAATTT TGTGGAAAAG 24720
   GAGGGCATAA ATGGAGGTGC TATCTAAAAT GTTATTTTTC TGAAAGAAAA AATAAGAAAT 24780
   TAATGCTCCT ATTTGCAACT GTAACACTTA TTCCAGTATG TTCTCTTCTT TCTTCATGTT 24840
   TGGCCAGAGC CAGACTTTGC AGACAGATTA CATCACATAT GTGGATGAGC TGGGCTCTTT 24900
   CATAGGGGCC AAGCCTAACA TACCATGGCT CTTCCTGACA GATCCCCGCC TGGCCCTGGA 24960
   GGTGTACTTT GGCCCTTGCA GCCCATACCA GTTTCGACTG ATGGGACCAG GGAAGTGGGA 25020
   TGGGGCCAGA AATGCCATCC TGACCCAGTG GAACCGGACA GTGAAGCCAA CCAGGACAAG 25080
   AGTTGTCAGT GAAGTTCAGC GACCCCATCC CTTTTACAAT TTGCTTAAAA TGCTTTCATT 25140
   CCCATTACTC CTTCTGGCTG TTACACTTAC ATTTTATTAA TGAGAAAGTC TTTGAGGTCT 25200
   CAAAATTCAG CATAGAAGTG TAATCACACA ATACAACACA CACCACACAT ACACACACAC 25260
   AATCACAACA TAGTTCCTCT CTCCTTTCCT GAAGATATGA AAATCAGTCT TGGCCCATTT 25320
   GAATTAAAGT ATAAGTAAAA TGGAAAATAC TCAGCCTCTC TCTCTCTGTT GGGAATCTGT 25380
   TCTCTAAAAG GCTTTTCACA TGCTGAATTG GCAAATTTGG GGATGCTTAA GATAAGACAG 25440
   GAAGTTGAAT AAGCATGAGC ACAG 25464
(2) INFORMATIONS POUR LA SEQ ID NO: 5:
   (i) CARACTERISTIQUES DE LA SEQUENCE:
      (A) LONGUEUR: 1605 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 5:
   GCAATCATGA GCAAGAGGGT TGGCATCATC GGAGCTGGAG TCAGTGGCTT GGCTGCCATA 60
   TGGTGCTGTC TGGAGGAGGG GCTGGAGCCC ACTTGCTTTG AAAGGAGCGA TGATGTTGGA 120
   GGCCTGTGGA AATTCTCAGA CCACACAGAA GAAGGCAGAG CCAGCATTTA CCAGTCTGTA 180
   TTCACAAACT CTTCCAAAGA AATGATGTGC TTTCCAGACT TCCCTTATCC GGATGATTAC 240
   CCAAACTATA TACACCACAG CAAGCTCCAG GAATATATAA AGACATATGC TCAAAAGAAG 300
   GAACTTTTAA GATACATANA GTTTGAGACC CTGGTTTCCG GTATAAAGAA ATGTCCCAGC 360
   TTCTTAGTCA CGGGCCAATG GGTTGTTGTT ACTGAAAAGG ATGGGAAACA GGAATCTACT 420
   ATTTTTGATG CTGTAATGAT TTGTTCAGGA CATCACGTAT ACCCCAATCT GCCAACGGAT 480
   TCCTTTCCTG GCCTGGACCA GTTTCGAGGC AACTACCTCC ATAGCCGGGA TTATAAGAAT    540
   CCAGAAGCCT TCAAGGGGAA GAGGGTCCTC GTGATTGGTC TGGGGAATTC GGGATCTGAC    600
   ATTGCTGTTG AGCTCAGCCG TCTGGCTACA CAGGTCATTA TCAGTACCAG AAGTGCTTCC    660
   TGGGTCATGA GTCGGGTCTG GGATGATGGC TATCCTTGGG ATATGATGTA TGTTACCCGC    720
   TTTGCATCCT TTCTCCGGAA TGTCCTTCCT TCATTCATCT CTGACTGGTT ATATGTCCAG    780
   AAGATGAACA CGTGGTTTAA GCATGAGAAC TATGGCCTGA TGCCTTTAAA TGGTTCCCTG    840
   AGAAAAGAGC CTGTGTTCAA TGATGAGCTC CCATCCCGCA TGCTGTGTGG CACTCTGTCC    900
   ATCAAGCCCA GTGTGAAGGA GTTCACGGAA ACCTCAGCTG TGTTTGAGGA TGGGACCATG    960
   TTTGAGGCTA TCGACTCTGT CATCTTTGCA ACAGGCTATG ATTATTCCTA CGCCTTCCTT   1020
   GATGAGACCA TCATGAAAAG CAGAAACAAT GAGGTTACCT TGTTTAAAGG CATCTTCCCC   1080
   CCACTAATGG AGAAGCCAAC CTTGGCTGTG ATTGGCTTGG TTCAGTCCCT TGGAGCTGCC   1140
   ATCCCCACAG CAGACCTGCA AGCCTGGTGG GCTGCTAAAG TATTTGCAAA CTCATGTACC   1200
   CTGCCAACCA CGAATGAAAT GATGGATGAC ACTGATGAGA AAATGGGGAA AAAACTCAAG   1260
   TGGTTTGGCC AGAGCCAGAC TTTGCAGACA GATTACATCA CATATGTGGA TGAGCTGGGC   1320
   TCTTTCATAG GGGCCAAGCC TAACATACCA TGGCTCTTCC TGACAGATCC CCGCCTGGCC   1380
   CTGGAGGTGT ACTTTGGCCC TTGCAGCCCA TACCAGTTTC GACTGATGGG ACCAGGGAAG   1440
   TGGGATGGGG CCAGAAATGC CATCCTGACC CAGTGGAACC GGACAGTGAA GCCAACCAGG   1500
   ACAAGAGTTG TCAGTGAAGT TCAGCGACCC CATCCCTTTT ACAATTTGCT TAAAATGCTT   1560
   TCATTCCCAT TACTCCTTCT GGCTGTTACA CTTACATTTT ATTAA                   1605
(2) INFORMATIONS POUR LA SEQ ID NO: 6:
   (i) CARACTERISTIQUES DE LA SEQUENCE:
      (A) LONGUEUR: 532 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 6:
   Met Ser Lys Arg Val Gly Ile Ile Gly Ala Gly Val Ser Gly Leu Ala
   1               5                   10                  15
   Ala Ile Trp Cys Cys Leu Glu Glu Gly Leu Glu Pro Thr Cys Phe Glu
               20                  25                  30
   Arg Ser Asp Asp Val Gly Gly Leu Trp Lys Phe Ser Asp His Thr Glu
           35                  40                  45
   Glu Gly Arg Ala Ser Ile Tyr Gln Ser Val Phe Thr Asn Ser Ser Lys
       50                  55                  60
   Glu Met Met Cys Phe Pro Asp Phe Pro Tyr Pro Asp Asp Tyr Pro Asn
   65                  70                  75                  80
   Tyr Ile His His Ser Lys Leu Gln Glu Tyr Ile Lys Thr Tyr Ala Gln
                   85                  90                  95
   Lys Lys Glu Leu Leu Arg Tyr Ile Gln Phe Glu Thr Leu Val Ser Gly
               100                 105                 110
   Ile Lys Lys Cys Pro Ser Phe Leu Val Thr Gly Gln Trp Val Val Val
           115                 120                 125
   Thr Glu Lys Asp Gly Lys Gln Glu Ser Thr Ile Phe Asp Ala Val Met
       130                 135                 140
   Ile Cys Ser Gly His His Val Tyr Pro Asn Leu Pro Thr Asp Ser Phe
   145                 150                 155                 160
   Pro Gly Leu Asp Gln Phe Arg Gly Asn Tyr Leu His Ser Arg Asp Tyr
                   165                 170                 175
   Lys Asn Pro Glu Ala Phe Lys Gly Lys Arg Val Leu Val Ile Gly Leu
               180                 185                 190
   Gly Asn Ser Gly Ser Asp Ile Ala Val Glu Leu Ser Arg Leu Ala Thr
           195                 200                 205
   Gln Val Ile Ile Ser Thr Arg Ser Ala Ser Trp Val Met Ser Arg Val
       210                 215                 220
   Trp Asp Asp Gly Tyr Pro Trp Asp Met Met Tyr Val Thr Arg Phe Ala
   225                 230                 235                 240
   Ser Phe Leu Arg Asn Val Leu Pro Ser Phe Ile Ser Asp Trp Leu Tyr
                   245                 250                 255
   Val Gln Lys Met Asn Thr Trp Phe Lys His Glu Asn Tyr Gly Leu Met
               260                 265                 270
   Pro Leu Asn Gly Ser Leu Arg Lys Glu Pro Val Phe Asn Asp Glu Leu
           275                 280                 285
   Pro Ser Arg Ile Leu Cys Gly Thr Leu Ser Ile Lys Pro Ser Val Lys
       290                 295                 300
   Glu Phe Thr Glu Thr Ser Ala Val Phe Glu Asp Gly Thr Met Phe Glu
   305                 310                 315                 320
   Ala Ile Asp Ser Val Ile Phe Ala Thr Gly Tyr Asp Tyr Ser Tyr Pro
                   325                 330                 335
   Phe Leu Asp Glu Thr Ile Met Lys Ser Arg Asn Asn Glu Val Thr Leu
               340                 345                 350
   Phe Lys Gly Ile Phe Pro Pro Leu Met Glu Lys Pro Thr Leu Ala Val
           355                 360                 365
   Ile Gly Leu Val Gln Ser Leu Gly Ala Ala Ile Pro Thr Ala Asp Leu
       370                 375                 380
   Gln Ala Trp Trp Ala Ala Lys Val Phe Ala Asn Ser Cys Thr Leu Pro
   385                 390                 395                 400
   Thr Thr Asn Glu Met Met Asp Asp Thr Asp Glu Lys Met Gly Lys Lys
                   405                 410                 415
   Leu Lys Trp Phe Gly Gln Ser Gln Thr Leu Gln Thr Asp Tyr Ile Thr
               420                 425                 430
   Tyr Val Asp Glu Leu Gly Ser Phe Ile Gly Ala Lys Pro Asn Ile Pro
           435                 440                 445
   Trp Leu Phe Leu Thr Asp Pro Arg Leu Ala Leu Glu Val Tyr Phe Gly
       450                 455                 460
   Pro Cys Ser Pro Tyr Gln Phe Arg Leu Met Gly Pro Gly Lys Trp Asp
   465                 470                 475                 480
   Gly Ala Arg Asn Ala Ile Leu Thr Gln Trp Asn Arg Thr Val Lys Pro
                   485                 490                 495
   Thr Arg Thr Arg Val Val Ser Glu Val Gln Arg Pro His Pro Phe Tyr
               500                 505                 510
   Asn Leu Leu Lys Met Leu Ser Phe Pro Leu Leu Leu Leu Ala Val Thr
           515                 520                 525
   Leu Thr Phe Tyr
       530
(2) INFORMATIONS POUR LA SEQ ID NO: 7:
   (i) CARACTERISTIQUES DE LA SEQUENCE:
      (A) LONGUEUR: 21 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEQ ID NO: 7:
      TCACATAGAG TGCTATGGGG G 21
(2) INFORMATIONS POUR LA SEQ ID NO: 8:
   (i) CARACTERISTIQUES DE LA SEQUENCE:
      (A) LONGUEUR: 25 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEQ ID N0: 8:
      CTTAGGAAGA AGATAAAAAT GCAAC 25
(2) INFORMATIONS POUR LA SEQ ID NO: 9:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEQ ID NO: 9:
      AATGTCCATC ATCATAGTTC TCT 23
(2) INFORMATIONS POUR LA SEQ ID NO: 10:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEQ ID NO: 10:
      TAGGCTTGTG TAGCCTGCCC TCA 23
(2) INFORMATIONS POUR LA SEQ ID NO: 11:
   (i) CARACTERISTIQUES DE LA SEQUENCE:
      (A) LONGUEUR: 16 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEQ ID NO: 11:
      CCTCAGAGAG AACTAT 16
(2) INFORMATIONS POUR LA SEQ ID NO: 12:
   (i) CARACTERISTIQUES DE LA SEQUENCE:
      (A) LONGUEUR: 16 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEQ ID NO: 12:
      GGAGTCTCTC TTGATA 16
(2) INFORMATIONS POUR LA SEQ ID NO: 13:
   (i) CARACTERISTIQUES DE LA SEQUENCE:
      (A) LONGUEUR: 16 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEQ ID NO: 13:
      CCTCAAAGAG AACTAT 16
(2) INFORMATIONS POUR LA SEQ ID NO: 14:
   (i) CARACTERISTIQUES DE LA SEQUENCE:
      (A) LONGUEUR: 16 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEQ ID NO: 14:
      GGAGTTTCTC TTGATA 16